# EUROPEAN PATENT SPECIFICATION

(11) **EP 3 850 100 B1**
(45) Date of publication and mention of the grant of the patent: **06.11.2024**
(21) Application number: 19859763.5
(22) Date of filing: 11.09.2019
(51) Int. Cl.: C12N 15/113, C12N 15/11

(54) **TARGETING EGLN1 IN CANCER**
TARGETING VON EGLN1 BEI KREBS
CIBLAGE DE L'EGLN1 DANS LE CANCER

(30) Priority: 12.09.2018 US 201862730315 P
(43) Date of publication of application: 21.07.2021
(73) Proprietor: The Broad Institute, Inc., Cambridge, MA 02142 (US); Dana-Farber Cancer Institute, Inc., Boston, MA 02215 (US); Instituto Carlos Slim de la Salud, A.C., C.P. 11529 Mexico, D.F., Mexico (MX)
(72) Inventor: HAHN, William, Boston, Massachusetts 02215 (US); VAZQUEZ, Francisca, Cambridge, Massachusetts 02142 (US); PRICE, Colles, Boston, Massachusetts 02215 (US)
(74) Representative: Maschio & Soames IP Ltd
(86) International application number: PCT/US2019/050516
(87) International publication number: WO 2020/055946

(56) References cited:
- US-A1- 2013 085 152
- KLOTZSCHE-VON AMELN ANNE ET AL: "HIF prolyl hydroxylase-2 inhibition diminishes tumor growth through matrix metalloproteinase-induced TGF[beta] activation", CANCER BIOLOGY & THERAPY, vol. 13, no. 4, 15 February 2012 (2012-02-15), US, pages 216 - 223, XP055909911, ISSN: 1538-4047, Retrieved from the Internet <URL:https://www.tandfonline.com/doi/pdf/10.4161/cbt.13.4.18830?needAccess=true> DOI: 10.4161/cbt.13.4.18830
- AMELN ANNE KLOTZSCHE-VON ET AL: "Inhibition of HIF Prolyl Hydroxylase-2 Blocks Tumor Growth in Mice through the Antiproliferative Activity of TGF[beta]", CANCER RESEARCH, vol. 71, no. 9, 24 March 2011 (2011-03-24), US, pages 3306 - 3316, XP055910244, ISSN: 0008-5472, DOI: 10.1158/0008-5472.CAN-10-3838
- CULLEN TANIGUCHI M ET AL: "Abstract 979: EGLN inhibition reduces gastrointestinal radiation toxicity and improves survival in a murine model of locally advanced pancreatic cancer | Cancer Research | American Association for Cancer Research", CANCER RESEARCH, vol. 13, no. S, 1 July 2018 (2018-07-01), US, pages 1 - 4, XP055910195, ISSN: 1538-7445, Retrieved from the Internet <URL:https://aacrjournals.org/cancerres/article/78/13_Supplement/979/630751/Abstract-979-EGLN-inhibition-reduces>
- HAFFEY W D ET AL: "iTRAQ proteomic identification of pVHL-dependent and -independent targets of Egln1 prolyl hydroxylase knockdown in renal carcinoma cells", ADVANCES IN ENZYME REGULATION, PERGAMON PRESS, OXFORD, GB, vol. 49, no. 1, 1 January 2009 (2009-01-01), pages 121 - 132, XP026169273, ISSN: 0065-2571, [retrieved on 20081231], DOI: 10.1016/J.ADVENZREG.2008.12.004
- ERIC METZEN ET AL: "Regulation of the prolyl hydroxylase domain protein 2(phd2/egln-1)gene: identification of a functional hypoxia-responsive element", BIOCHEMICAL JOURNAL, PUBLISHED BY PORTLAND PRESS ON BEHALF OF THE BIOCHEMICAL SOCIETY, GB, vol. 387, no. 3, 1 May 2005 (2005-05-01), pages 711 - 717, XP008157837, ISSN: 0264-6021, [retrieved on 20041124], DOI: 10.1042/BJ20041583
- KIMBERLY J BRIGGS ET AL: "Abstract 2892: EGLN1 is a synthetic lethal target in ARID1A-mutant ovarian cancer", vol. 78, no. 13 Suppl, 1 July 2018 (2018-07-01), pages 2892, XP009527047, ISSN: 0008-5472, Retrieved from the Internet <URL:https://cancerres.aacrjournals.org/content/78/13_Supplement/2892> DOI: 10.1158/1538-7445.AM2018-2892
- KIMBERLY J. BRIGGS, CHENGYIN MIN, HONGXIANG ZHANG AND ALAN HUANG: "Abstract 2892: EGLN1 is a synthetic lethal target in ARID1A-mutant ovarian cancer", CANCER RESEARCH, vol. 78, no. 13, 1 July 2018 (2018-07-01), pages 2892, XP009527047, ISSN: 0008-5472, DOI: 10.1158/1538-7445.AM2018-2892
- CHEN ET AL.: "Diagnosis of the small round blue cell tumors using multiplex polymerase chain reaction", THE JOURNAL OF MOLECULAR DIAGNOSTICS, vol. 9, no. 1, February 2007 (2007-02-01), pages 80 - 88, XP055691037
- FROST ET AL.: "Potent and selective chemical probe of hypoxic signalling downstream of HIF- .alpha. hydroxylation via VHL inhibition", NATURE COMMUNICATIONS, vol. 7, 4 November 2016 (2016-11-04), pages 13312; 1 - 12, XP055548756
- YEH ET AL.: "Molecular and cellular mechanisms of HIF prolyl hydroxylase inhibitors in clinical trials", CHEMICAL SCIENCE, vol. 8, no. 11, 1 November 2017 (2017-11-01), pages 7651 - 7668, XP055691040
- JOKILEHTO ET AL.: "Overexpression and nuclear translocation of hypoxia-inducible factor prolyl hydroxylase PHD2 in head and neck squamous cell carcinoma is associated with tumor aggressiveness", CANCER RESEARCH, vol. 12, no. 4, 15 February 2006 (2006-02-15), pages 1080 - 1087, XP055691042

## Description

### FIELD OF THE INVENTION

The invention relates generally to methods, compositions and kits for the identification and treatment of EGLN1-dependent cancers.

### BACKGROUND OF THE INVENTION

Identifying the genes that are required for tumor maintenance poses an ongoing challenge for the oncology field. A need exists for approaches that can successfully identify genes required for tumor maintenance as preferred targets for gene-targeting and/or gene product-targeting therapies.

### BRIEF SUMMARY OF THE INVENTION

The current disclosure relates, at least in part, to the identification of an EGLN1-dependency for certain forms of cancer, particularly clear cell ovarian cancers and melanoma (though treatment of any form of EGLN1-dependent cancer is contemplated). Compositions and methods for the diagnosis and treatment of EGLN1-dependent cancers are therefore provided. In particular, the instant disclosure has identified that inhibitors of EGLN1 and/or inhibitors of VHL (Von Hippel-Lindau Tumor Suppressor) can be used for treatment cancers that exhibit such EGLN1-dependency. Many EGLN1 inhibitors are known in the art and some are in clinical trials for treatment of anemia in patients with chronic kidney disease, including FG-4592, FG-2216, Molidustat, IOX2 and Daprodustat. VHL inhibitors have also been identified, including VH298, which has been described for targeting hypoxia signaling. In addition, it has been identified herein that EGLN1 dependent cell lines tend to exhibit elevated Hypoxia Inducible Factor 1 Alpha (HIF1A) levels.

In one aspect, the instant disclosure provides <CLAIM 1>.

In one embodiment, the cancer is a melanoma or an ovarian cancer, optionally a clear cell ovarian cancer.

In another embodiment, step (b) includes identifying the presence or absence in the sample of elevated Hypoxia Inducible Factor 1 Alpha (HIF1A), as compared to an appropriate control.

In another embodiment, identifying step (b) includes use of a kit of the instant disclosure.

Optionally, the subject is human.

In one embodiment, the sample is a cancer sample. Optionally, the cancer sample is a melanoma sample and/or an ovarian cancer sample. Optionally, the ovarian cancer is a clear cell ovarian cancer.

In another embodiment, the sample is a tissue sample of a subject having a cancer that is a melanoma or an ovarian cancer. Optionally, the ovarian cancer is a clear cell ovarian cancer.

In a further embodiment, the kit includes reagents for assessing HIF1A levels in the sample.

An additional aspect of the instant disclosure provides <CLAIM 6>.

In one embodiment, step (b) includes performing a single multiplex PCR reaction that is analyzed by capillary electrophoresis.

In another embodiment, step (b) includes identifying the presence or absence in the sample of elevated Hypoxia Inducible Factor 1 Alpha (HIF1A), as compared to an appropriate control.

In an additional embodiment, step (b) includes use of a kit of the instant disclosure.

### Definitions

Unless specifically stated or obvious from context, as used herein, the term "about" is understood as within a range of normal tolerance in the art, for example within 2 standard deviations of the mean. "About" can be understood as within 10%, 9%, 8%, 7%, 6%, 5%, 4%, 3%, 2%, 1%, 0.5%, 0.1%, 0.05%, or 0.01% of the stated value.

In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

Unless otherwise clear from context, all numerical values provided herein are modified by the term "about."

The term "administration" refers to introducing a substance into a subject. In general, any route of administration may be utilized including, for example, parenteral (e.g., intravenous), oral, topical, subcutaneous, peritoneal, intraarterial, inhalation, vaginal, rectal, nasal, introduction into the cerebrospinal fluid, or instillation into body compartments. In some embodiments, administration is oral. Additionally or alternatively, in some embodiments, administration is parenteral. In some embodiments, administration is intravenous.

By "agent" is meant any small compound (e.g., small molecule), antibody, nucleic acid molecule, or polypeptide, or fragments thereof or cellular therapeutics such as allogeneic transplantation and/or CART-cell therapy.

The term "cancer" refers to a malignant neoplasm (Stedman's Medical Dictionary, 25th ed.; Hensyl ed.; Williams & Wilkins: Philadelphia, 1990). Exemplary cancers include, but are not limited to, melanoma and ovarian cancer (e.g., cystadenocarcinoma, ovarian embryonal carcinoma, ovarian adenocarcinoma), with ovarian cancer specifically including clear cell ovarian cancer. Additional exemplary cancers include, but are not limited to, colorectal cancer (e.g., colon cancer, rectal cancer, colorectal adenocarcinoma), endometrial cancer (e.g., uterine cancer, uterine sarcoma), esophageal cancer (e.g., adenocarcinoma of the esophagus, Barrett's adenocarcinoma), and gastric cancer (e.g., stomach adenocarcinoma (STAD)), including, e.g., colon adenocarcinoma (COAD), oesophageal carcinoma (ESCA), rectal adenocarcinoma (READ) and uterine corpus endometrial carcinoma (UCEC). Other exemplary forms of cancer include, but are not limited to, diffuse large B-cell lymphoma (DLBCL), as well as the broader class of lymphoma such as Hodgkin lymphoma (HL) (e.g., B-cell HL, T-cell HL) and non-Hodgkin lymphoma (NHL) (e.g., B-cell NHL such as diffuse large cell lymphoma (DLCL) (e.g., diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (e.g., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (i.e., Waldenström's macroglobulinemia), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (e.g., cutaneous T-cell lymphoma (CTCL) (e.g., mycosis fungoides, Sezary syndrome), angioimmunoblastic T-cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above;, hematopoietic cancers (e.g., myeloid malignancies (e.g., acute myeloid leukemia (AML) (e.g., B-cell AML, T-cell AML), myelodysplastic syndrome, myeloproliferative neoplasm, chronic myelomonocytic leukemia (CMML) and chronic myelogenous leukemia (CML) (e.g., B-cell CML, T-cell CML)) and lymphocytic leukemia such as acute lymphocytic leukemia (ALL) (e.g., B-cell ALL, T-cell ALL) and chronic lymphocytic leukemia (CLL) (e.g., B-cell CLL, T-cell CLL)); brain cancer (e.g., meningioma, glioblastomas, glioma (e.g., astrocytoma, oligodendroglioma), medulloblastoma); lung cancer (e.g., bronchogenic carcinoma, small cell lung cancer (SCLC), non-small cell lung cancer (NSCLC), adenocarcinoma of the lung); acoustic neuroma; adenocarcinoma; adrenal gland cancer; anal cancer; angiosarcoma (e.g., lymphangiosarcoma, lymphangioendotheliosarcoma, hemangiosarcoma); appendix cancer; benign monoclonal gammopathy; biliary cancer (e.g., cholangiocarcinoma); bladder cancer; breast cancer (e.g., adenocarcinoma of the breast, papillary carcinoma of the breast, mammary cancer, medullary carcinoma of the breast); bronchus cancer; carcinoid tumor; cervical cancer (e.g., cervical adenocarcinoma); choriocarcinoma; chordoma; craniopharyngioma; connective tissue cancer; epithelial carcinoma; ependymoma; endotheliosarcoma (e.g., Kaposi's sarcoma, multiple idiopathic hemorrhagic sarcoma); Ewing's sarcoma; ocular cancer (e.g., intraocular melanoma, retinoblastoma); familiar hypereosinophilia; gall bladder cancer; gastrointestinal stromal tumor (GIST); germ cell cancer; head and neck cancer (e.g., head and neck squamous cell carcinoma, oral cancer (e.g., oral squamous cell carcinoma), throat cancer (e.g., laryngeal cancer, pharyngeal cancer, nasopharyngeal cancer, oropharyngeal cancer)); and multiple myeloma (MM)), heavy chain disease (e.g., alpha chain disease, gamma chain disease, mu chain disease); hemangioblastoma; hypopharynx cancer; inflammatory myofibroblastic tumors; immunocytic amyloidosis; kidney cancer (e.g., nephroblastoma a.k.a. Wilms' tumor, renal cell carcinoma); liver cancer (e.g., hepatocellular cancer (HCC), malignant hepatoma); leiomyosarcoma (LMS); mastocytosis (e.g., systemic mastocytosis); muscle cancer; myelodysplastic syndrome (MDS); mesothelioma; myeloproliferative disorder (MPD) (e.g., polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES)); neuroblastoma; neurofibroma (e.g., neurofibromatosis (NF) type 1 or type 2, schwannomatosis); neuroendocrine cancer (e.g., gastroenteropancreatic neuroendocrine tumor (GEP-NET), carcinoid tumor); osteosarcoma (e.g., bone cancer); papillary adenocarcinoma; pancreatic cancer (e.g., pancreatic andenocarcinoma, intraductal papillary mucinous neoplasm (IPMN), Islet cell tumors); penile cancer (e.g., Paget's disease of the penis and scrotum); pinealoma; primitive neuroectodermal tumor (PNT); plasma cell neoplasia; paraneoplastic syndromes; intraepithelial neoplasms; prostate cancer (e.g., prostate adenocarcinoma); rectal cancer; rhabdomyosarcoma; salivary gland cancer; skin cancer (e.g., squamous cell carcinoma (SCC), keratoacanthoma (KA), melanoma, basal cell carcinoma (BCC)); small bowel cancer (e.g., appendix cancer); soft tissue sarcoma (e.g., malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma; synovioma; testicular cancer (e.g., seminoma, testicular embryonal carcinoma); thyroid cancer (e.g., papillary carcinoma of the thyroid, papillary thyroid carcinoma (PTC), medullary thyroid cancer); urethral cancer; vaginal cancer; and vulvar cancer (e.g., Paget's disease of the vulva).

By "control" or "reference" is meant a standard of comparison. In one aspect, as used herein, "changed as compared to a control" sample or subject is understood as having a level that is statistically different than a sample from a normal, untreated, or control sample. Control samples include, for example, cells in culture, one or more laboratory test animals, or one or more human subjects. Methods to select and test control samples are within the ability of those in the art. Determination of statistical significance is within the ability of those skilled in the art, e.g., the number of standard deviations from the mean that constitute a positive result.

The terms "isolated," "purified, " or "biologically pure" refer to material that is free to varying degrees from components which normally accompany it as found in its native state. "Isolate" denotes a degree of separation from original source or surroundings. "Purify" denotes a degree of separation that is higher than isolation.

As used herein, the term "next-generation sequencing" or "NGS" can refer to sequencing technologies that have the capacity to sequence polynucleotides at speeds that were unprecedented using conventional sequencing methods (e.g., standard Sanger or Maxam-Gilbert sequencing methods). These unprecedented speeds are achieved by performing and reading out thousands to millions of sequencing reactions in parallel. NGS sequencing platforms include, but are not limited to, the following: Massively Parallel Signature Sequencing (Lynx Therapeutics); 454 pyro-sequencing (454 Life Sciences/Roche Diagnostics); solid-phase, reversible dye-terminator sequencing (Solexa/Illumina); SOLiD technology (Applied Biosystems); Ion semiconductor sequencing (ion Torrent); and DNA nanoball sequencing (Complete Genomics). Descriptions of certain NGS platforms can be found in the following: Shendure, er al., "Next-generation DNA sequencing," Nature, 2008, vol. 26, No. 10, 135-1 145; Mardis, "The impact of next-generation sequencing technology on genetics," Trends in Genetics, 2007, vol. 24, No. 3, pp. 133-141 ; Su, et al., "Next-generation sequencing and its applications in molecular diagnostics" Expert Rev Mol Diagn, 2011 , 11 (3):333-43; and Zhang et al., "The impact of next-generation sequencing on genomics", J Genet Genomics, 201, 38(3): 95-109.

As used herein, the term "subject" includes humans and mammals (e.g., mice, rats, pigs, cats, dogs, and horses). In many embodiments, subjects are mammals, particularly primates, especially humans. In some embodiments, subjects are livestock such as cattle, sheep, goats, cows, swine, and the like; poultry such as chickens, ducks, geese, turkeys, and the like; and domesticated animals particularly pets such as dogs and cats. In some embodiments (e.g., particularly in research contexts) subject mammals will be, for example, rodents (e.g., mice, rats, hamsters), rabbits, primates, or swine such as inbred pigs and the like.

As used herein, the terms "treatment," "treating," "treat" and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment," as used herein, covers any treatment of a disease or condition in a mammal, particularly in a human, and includes: (a) preventing the disease from occurring in a subject which can be predisposed to the disease but has not yet been diagnosed as having it; (b) inhibiting the disease, i.e., arresting its development; and (c) relieving the disease, i.e., causing regression of the disease.

Unless specifically stated or obvious from context, as used herein, the term "or" is understood to be inclusive. Unless specifically stated or obvious from context, as used herein, the terms "a", "an", and "the" are understood to be singular or plural.

The phrase "pharmaceutically acceptable carrier" is art recognized and includes a pharmaceutically acceptable material, composition or vehicle, suitable for administering compounds of the present disclosure to mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it is understood that the particular value forms another aspect. It is further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. It is also understood that throughout the application, data are provided in a number of different formats and that this data represent endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 as well as all intervening decimal values between the aforementioned integers such as, for example, 1.1, 1.2, 1.3, 1.4, 1.5, 1.6, 1.7, 1.8, and 1.9. With respect to sub-ranges, "nested sub-ranges" that extend from either end point of the range are specifically contemplated. For example, a nested sub-range of an exemplary range of 1 to 50 may comprise 1 to 10, 1 to 20, 1 to 30, and 1 to 40 in one direction, or 50 to 40, 50 to 30, 50 to 20, and 50 to 10 in the other direction.

The term "pharmaceutically acceptable salts, esters, amides, and prodrugs" as used herein refers to those carboxylate salts, amino acid addition salts, esters, amides, and prodrugs of the compounds of the present disclosure which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the disclosure.

The term "salts" refers to the relatively non-toxic, inorganic and organic acid addition salts of compounds of the present disclosure. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate and laurylsulphonate salts, and the like. These may include cations based on the alkali and alkaline earth metals, such as sodium, lithium, potassium, calcium, magnesium, and the like, as well as non-toxic ammonium, tetramethylammonium, tetramethylammonium, methlyamine, dimethlyamine, trimethlyamine, triethlyamine, ethylamine, and the like. (See, for example, S. M. Barge et al., "Pharmaceutical Salts," J. Pharm. Sci., 1977, 66:1-19.).

A "therapeutically effective amount" of an agent described herein is an amount sufficient to provide a therapeutic benefit in the treatment of a condition or to delay or minimize one or more symptoms associated with the condition. A therapeutically effective amount of an agent means an amount of therapeutic agent, alone or in combination with other therapies, which provides a therapeutic benefit in the treatment of the condition. The term "therapeutically effective amount" can encompass an amount that improves overall therapy, reduces or avoids symptoms, signs, or causes of the condition, and/or enhances the therapeutic efficacy of another therapeutic agent.

The transitional term "comprising," which is synonymous with "including," "containing," or "characterized by," is inclusive or open-ended and does not exclude additional, unrecited elements or method steps. By contrast, the transitional phrase "consisting of" excludes any element, step, or ingredient not specified in the claim. The transitional phrase "consisting essentially of" limits the scope of a claim to the specified materials or steps "and those that do not materially affect the basic and novel characteristic(s)" of the claimed invention.

Other features and advantages of the disclosure will be apparent from the following description of the preferred embodiments thereof, and from the claims. Unless otherwise defined, all technical and scientific terms used herein have the same meaning as commonly understood by one of ordinary skill in the art to which this disclosure belongs. Although methods and materials similar or equivalent to those described herein can be used in the practice or testing of the present disclosure, suitable methods and materials are described below. In the case of conflict, the present specification, including definitions, will control. In addition, the materials, methods, and examples are illustrative only and not intended to be limiting.

### BRIEF DESCRIPTION OF THE DRAWINGS

The following detailed description, given by way of example, but not intended to limit the disclosure solely to the specific embodiments described, may best be understood in conjunction with the accompanying drawings, in which:

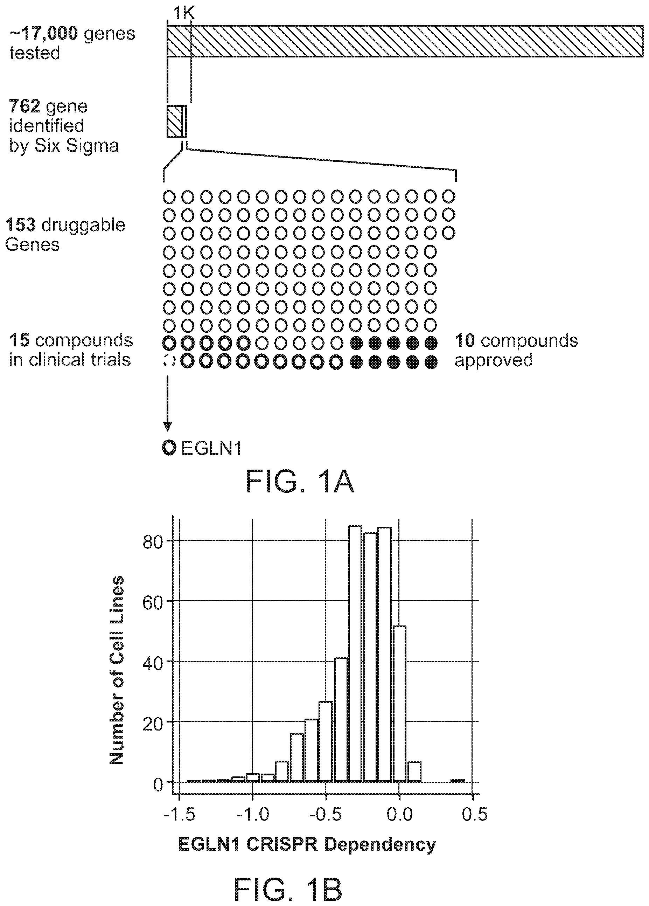

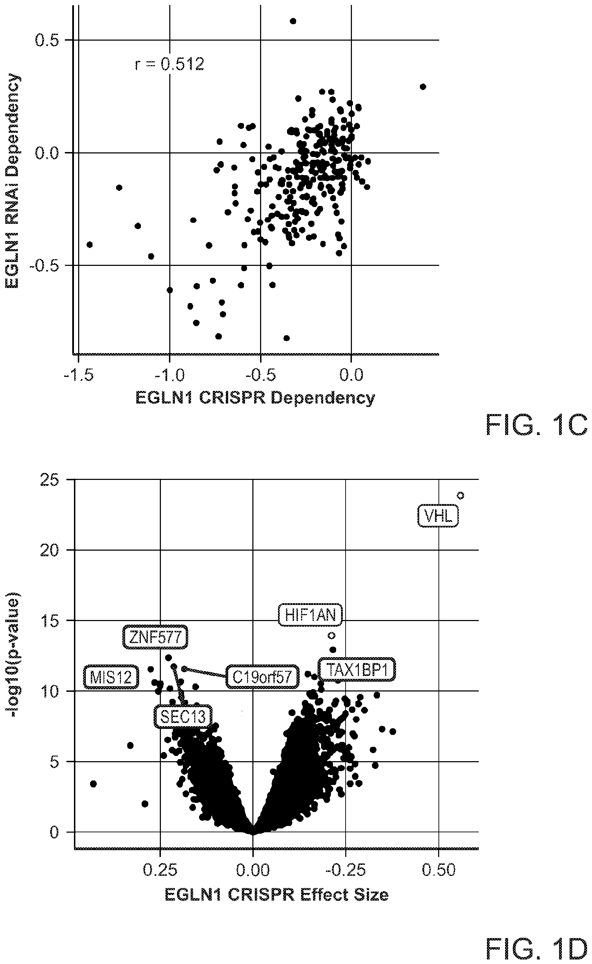

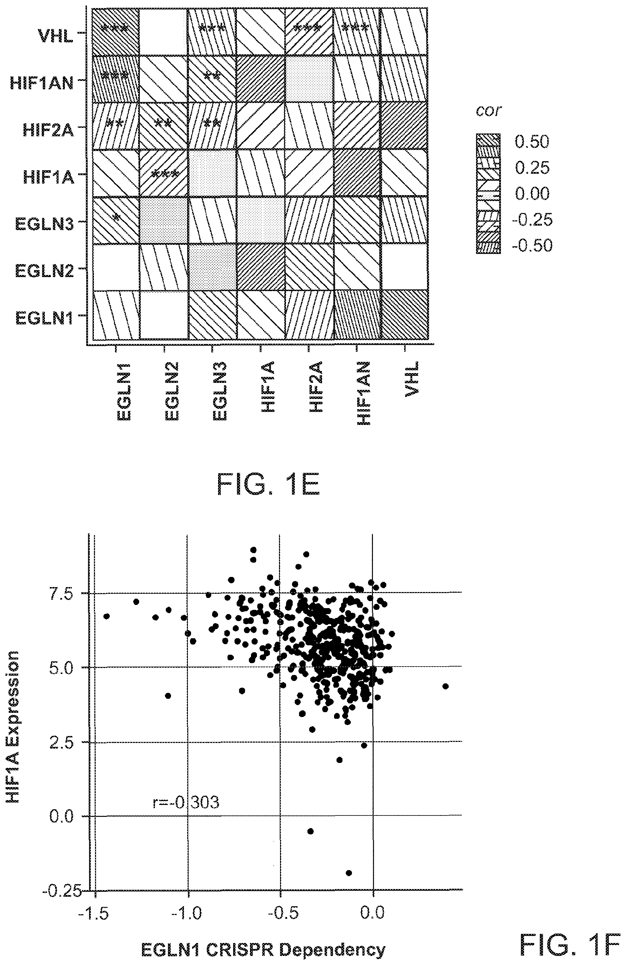

***FIGs. 1A to 1F*** demonstrate identification of EGLN1 as a differential cancer cell dependency. FIG. 1A shows identification of EGLN1 dependency in RNAi data from Project Achilles. From the initial 17,000 genes tested, 762 were found to be strong dependencies (Six Sigma dependencies). From these dependencies, 153 were currently druggable while 15 of them had compounds in clinical trials. EGLN1 was identified as one of these 15 clinically druggable dependencies. FIG. 1B shows identification of cancer cells dependent on EGLN1 using CRISPR-Cas9 data from Project Achilles. Histogram shows the distribution of cancer cell lines (Y-axis) that were dependent on EGLN1 based on their CERES score (X-axis). The line at Ceres score -0.5 denotes the observed cutoff for cell lines dependent on EGLN1. FIG. 1C shows concordance between RNAi and CRISPR-Cas9 datasets. For cell lines in both datasets, DEMETER scores of EGLN1 (RNAi, Y-axis) were graphed against EGLN1 Ceres score (CRISPR, X-axis). Concordance between the datasets was highly significant. Pearson = 0.552, Spearman = 0.523, n=243, p<10⁻²¹. FIG. 1D shows a volcano plot of cancer dependencies associated with EGLN1 dependency, graphed as effect size (X-axis) against p-value (-log₁₀, Y-axis). Colored in red are other members of the EGLN1 pathway. FIG. 1E shows that EGLN1 and VHL were the strongest correlated dependencies within the EGLN1 pathway while EGLN1 and HIF1AN were the second strongest correlated dependencies (P < 0.001). P-values were adjusted using the Benjamini and Hochberg FDR method. FDR < 0.05 (*), 0.01 (**), 0.001 (***). FIG. 1F shows that cell lines that expressed low levels of HIF1A (Y-axis) were not dependent on EGLN1 (X-axis).

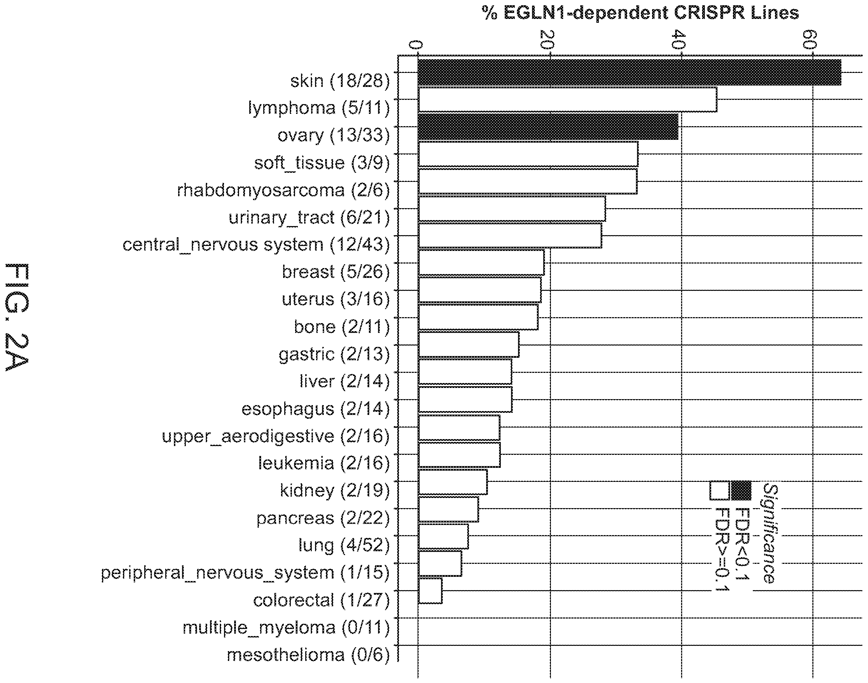

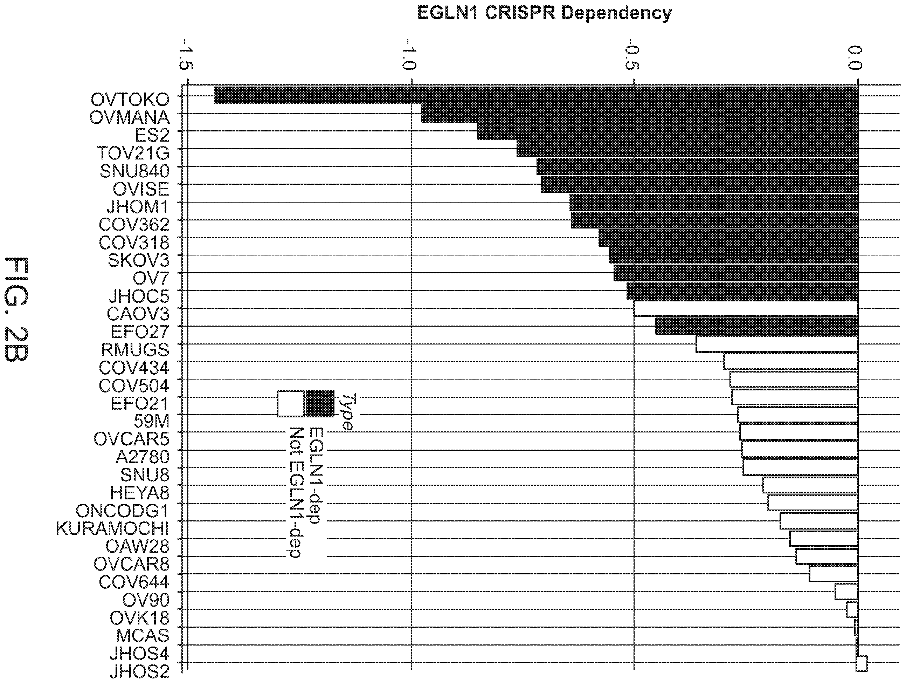

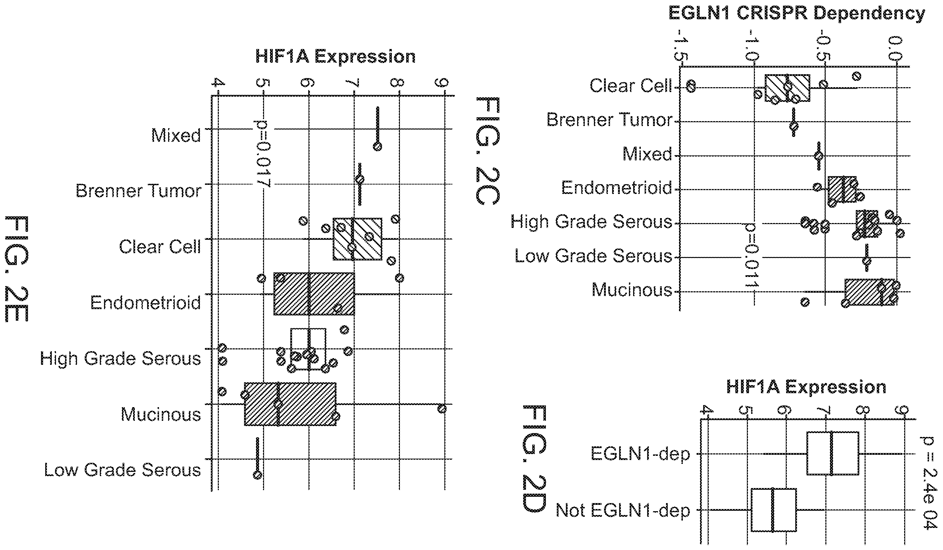

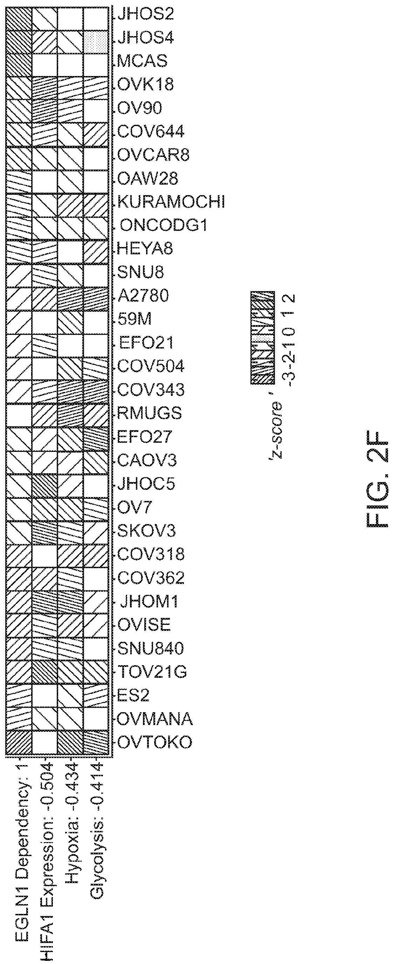

***FIGs. 2A to 2F*** demonstrate that EGLN1 dependency was enriched in clear cell ovarian cancer and melanoma, and was associated with high HIF1A levels. FIG. 2A shows a lineage analysis of EGLN1-dependent cell lines. EGLN1 was significantly enriched in ovarian cancer and melanoma, with several cell lines predicted to be dependent on EGLN1 (red). P<0.05. FIG. 2B shows the distribution of EGLN1 CERES score (Y-axis) in ovarian cancer cell lines. Cell lines (13/33) exhibited a greater than 50% probability of being dependent on EGLN1. *CAOV3 is not identified as an EGLN1-dependency, despite having a lower CERES effect score than EGLN1-dependent line EFO27, because the screen quality and other cell-line-specific differences were reflected in the CERES probabilities of dependency. FIG. 2C shows that EGLN1 dependent cells were enriched in clear cell ovarian cancer (by CRISPR dependency assessment). Subtype analysis of ovarian cancer cell lines revealed that EGLN1 dependency was enriched in clear cell ovarian cancer. FIG. 2D shows that EGLN1 dependency was associated with higher HIF1A expression in ovarian cancer. Ovarian cancer cell lines were grouped as either EGLN1-dependent (n=13) or not EGLN1-dependent (n=20). EGLN1-dependent cell lines expressed significantly more HIF1A (Y-axis) than non-EGLN1-dependent cells in the CRISPR dataset (p < 0.005). FIG. 2E shows that clear cell ovarian cancer cell lines exhibited high HIF1A expression. Subtype analysis of ovarian cancer cell lines revealed that clear cell ovarian cancer lines exhibited high expression of HIF1A. Two-sample t-test p-value p < 0.05. FIG. 2F shows that ssGSEA (single sample Gene Set Enrichment Analysis) revealed that EGLN1 dependency was associated with HIF1A-related pathways.

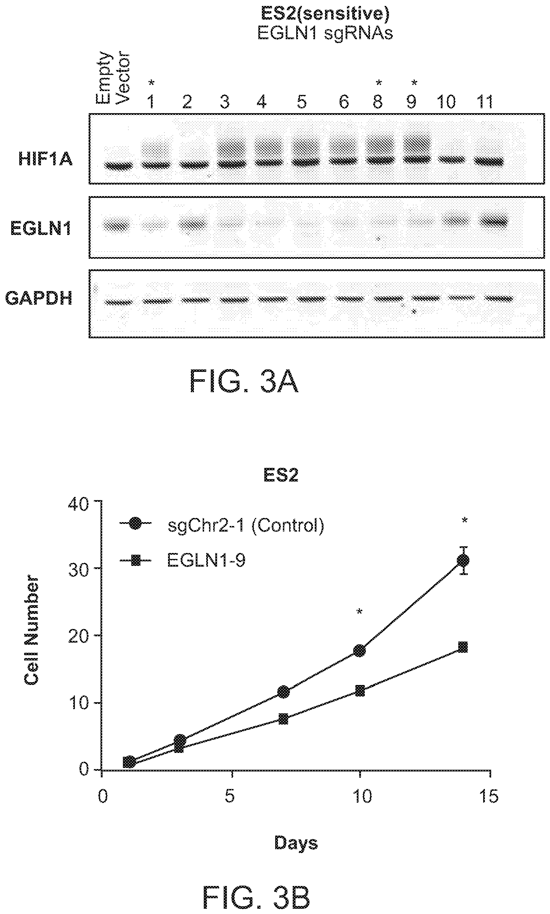

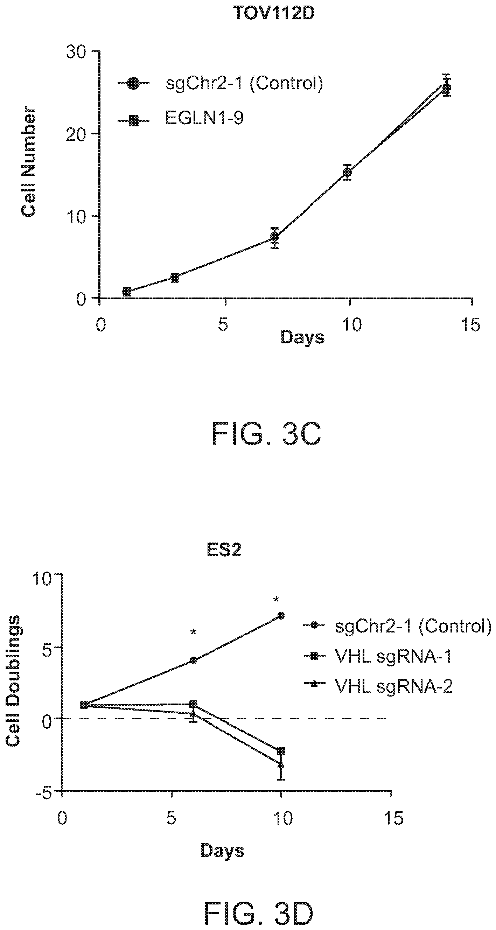

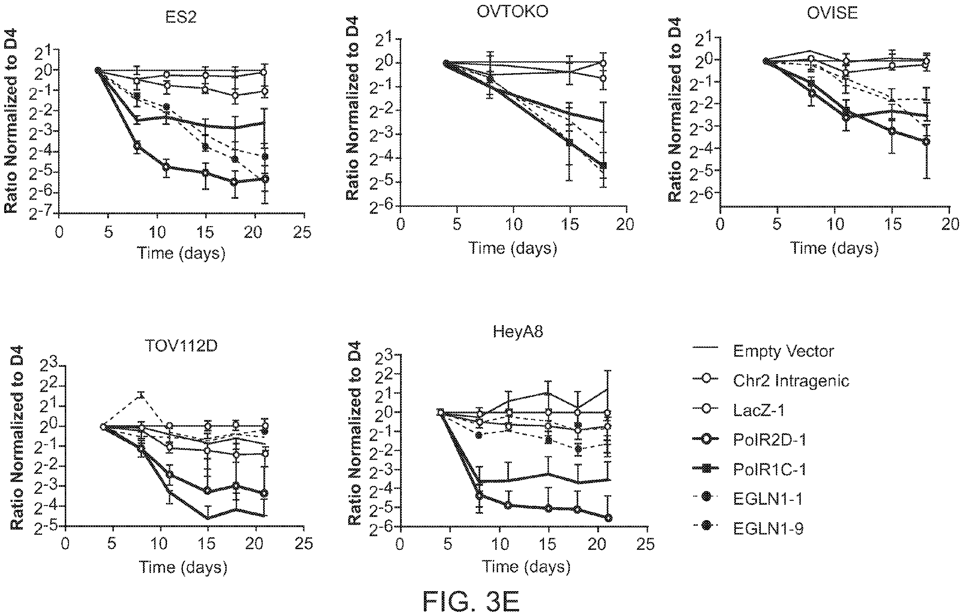

***FIGs. 3A to 3E*** demonstrate that EGLN1 deletion inhibited proliferation in EGLN1-dependent cell lines. FIG. 3A shows the effects of EGLN1 deletion on HIF1A levels. Whole cell lysates of ES2-Cas9 with the indicated sgRNAs. Equal amounts of protein were analyzed by immunoblotting with GAPDH included as a marker of equal loading. Deletion of EGLN1 led to increased HIF1A phosphorylation. Asterisks (*) indicate sgRNAs used for subsequent experiments. FIG. 3B shows that EGLN1 deletion reduced proliferation in EGLN1-dependent cells. Cells were serially passaged every 3-4 days. Results are representative of 3 independent experiments and data are graphed as mean + standard deviation of 3 replicates. *p<0.05. FIG. 3C shows that EGLN1 deletion exerted no effect on proliferation in EGLN1 independent cells. Cells were serially passaged every 3-4 days. Results are representative of 3 independent experiments and data are graphed as mean + standard deviation of 3 replicates. FIG. 3D shows that VΣ-Π, deletion reduced proliferation in EGLN1-dependent cells. Results are representative of 2 independent experiments and data are graphed as mean + standard deviation of 3 replicates. *p<0.05. FIG. 3E shows the results of a luciferase competition assay. Renilla-expressing and Firefly+Cas9-expressing cells were mixed at 1:1 then sgRNA were added. Ratio of Renilla/Firefly luciferase was quantified, and cells were serially passaged every 3-4 days. EGLN1 KO cells (red) were compared to negative controls (blue) and positive controls (black). EGLN1 deleted cells dependent on EGLN1 were outcompeted by EGLN1 WT cells over time in a proliferation assay, whereas EGLN1 deleted cells insensitive to EGLN1 were not. Results are representative of 2 independent experiments and data are graphed as mean + standard deviation of 3 technical replicates. Empty Vector - Empty Vector with no sgRNA. , Chr2 Intragenic - sgRNA targeting intragenic region of chromosome 2, LacZ-1 - sgRNA targeting LacZ gene, PolR2D-1 - sgRNA targeting RNA Polymerase 2 Subunit D, PolR1C-1 - sgRNA targeting RNA Polymerase 1 Subunit C.

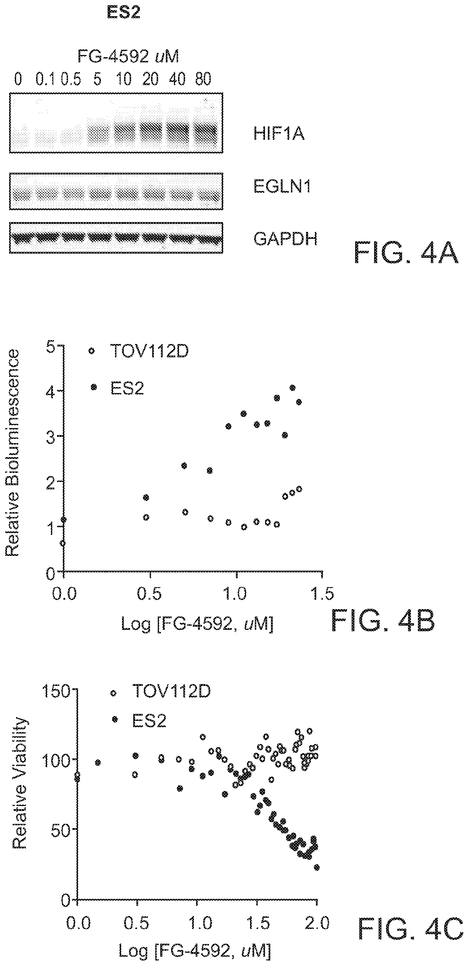

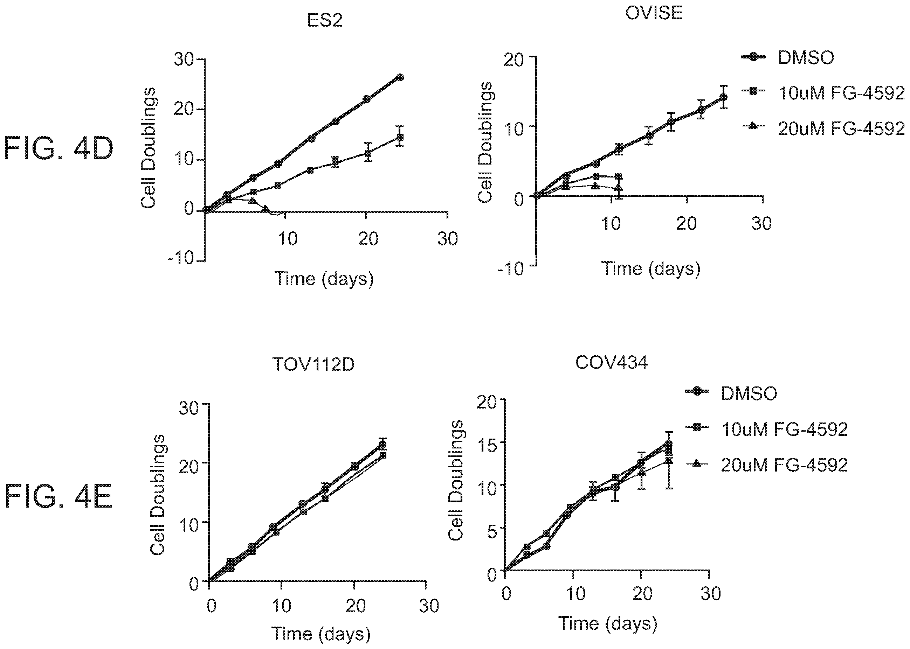

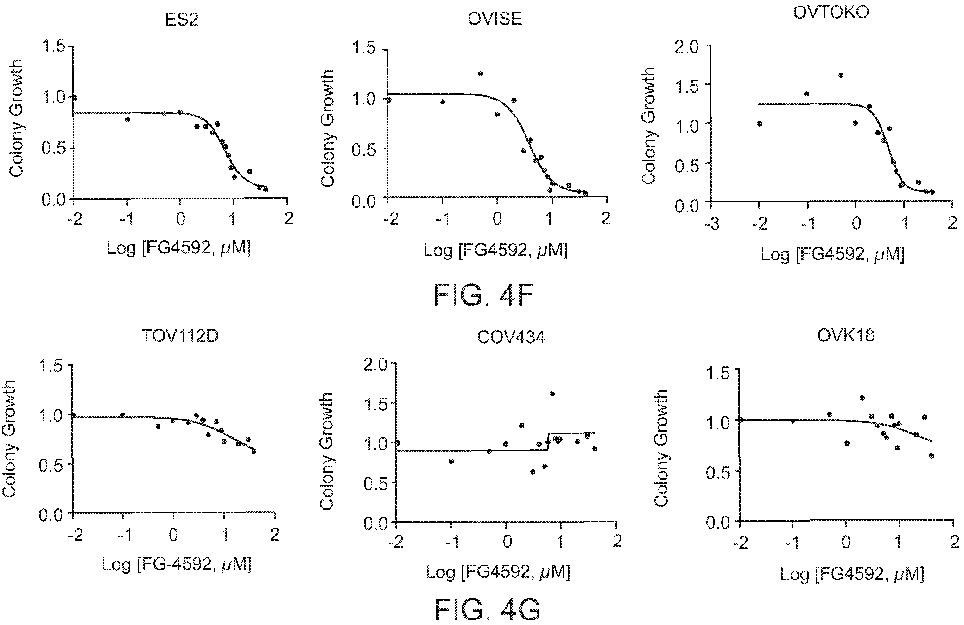

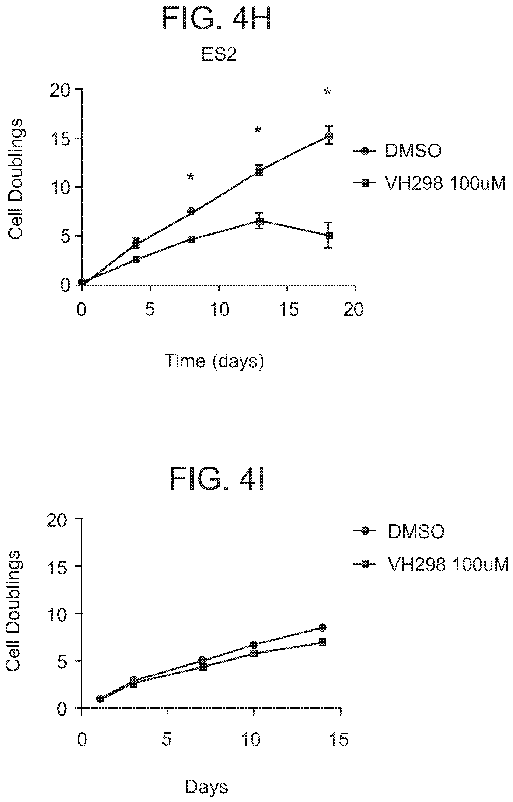

***FIGs. 4A to 4I*** demonstrate that inhibiting EGLN1 increased HIF1A expression and reduced proliferation in EGLN1-dependent cells. FIG. 4A shows an immunoblot demonstrating that pharmacological inhibition (FG-4592) of EGLN1 increased HIF1A levels in a dose-dependent manner. FIG. 4B shows that EGLN1 inhibitor increased HIF1A activity, as measured by using a Hypoxia Response Element (HRE) fused to a luciferase reporter. Luciferase activity was measured 48 hours post-treatment with increasing dose of FG-4592. Data are representative of two independent experiments. FIG. 4C shows that EGLN1 inhibitor reduced viability selectively in EGLN1-dependent cells. Cells were treated for 48 hours with increasing dose of FG-4592 (X-axis). Cell viability (Y-axis) was measured using cell titer glo. The data shown are representative of three independent experiments. FIG. 4D shows that EGLN1 inhibitor reduced long term proliferation in EGLN1-dependent cells. Results are representative of three independent experiments and data are graphed as mean + standard deviation of three replicates. FIG. 4E shows that EGLN1 inhibitor did not affect long term proliferation of EGLN1-insensitive cells. Results are representative of three independent experiments and data are graphed as mean + standard deviation of three replicates. FIG. 4F shows that EGLN1 inhibition reduced colony formation in a dose-dependent manner in EGLN1-dependent cells. Colonies were quantified as absorbance (Y-axis) over drug concentration (X-axis) over 10 days of treatment normalized to DMSO. Results are representative of 3 independent experiments. FIG. 4G shows that EGLN1 inhibition exhibited no significant effect on colony formation in EGLN1-insensitive cells. Colonies were quantified as absorbance (Y-axis) over drug concentration (X-axis) over 10 days of treatment normalized to DMSO. Results are representative of three independent experiments. FIG. 4H shows that VΣ-Π, inhibition reduced proliferation of EGLN1-dependent cells. Results are representative of 3 independent experiments, and data are graphed as mean + standard deviation of 3 replicates. *p<0.05. FIG. 4I shows that VΣ-Π, inhibition did not affect proliferation of EGLN1-insensitive cells. Results are representative of two independent experiments, and data are graphed as mean + standard deviation of biological triplicates.

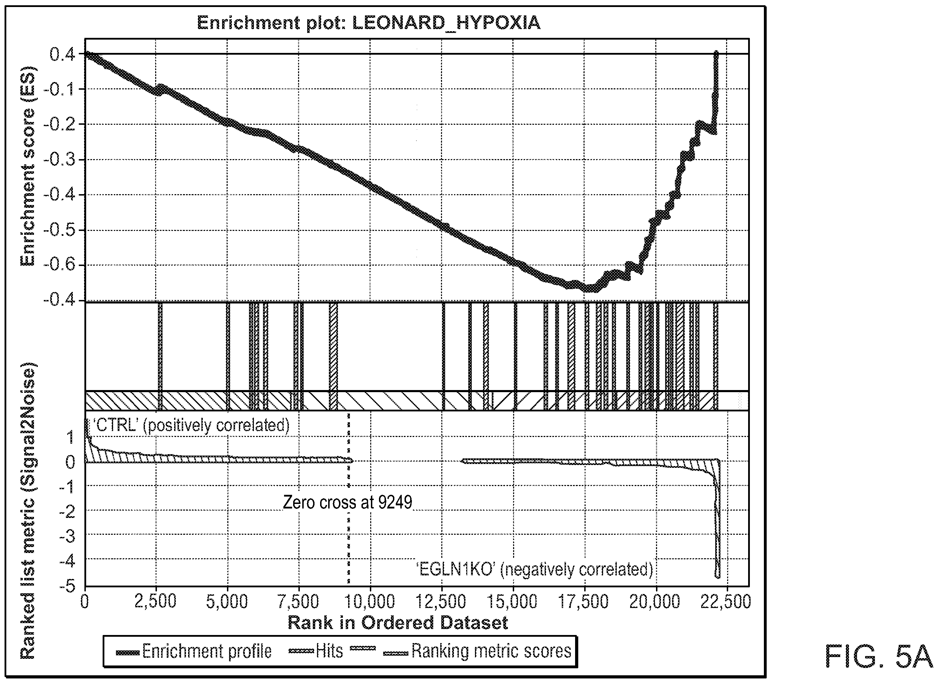

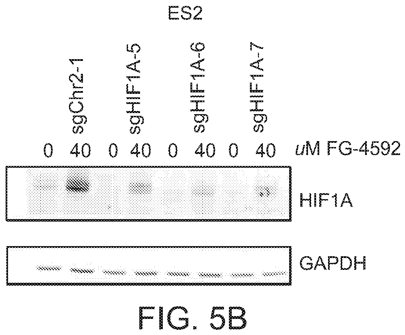

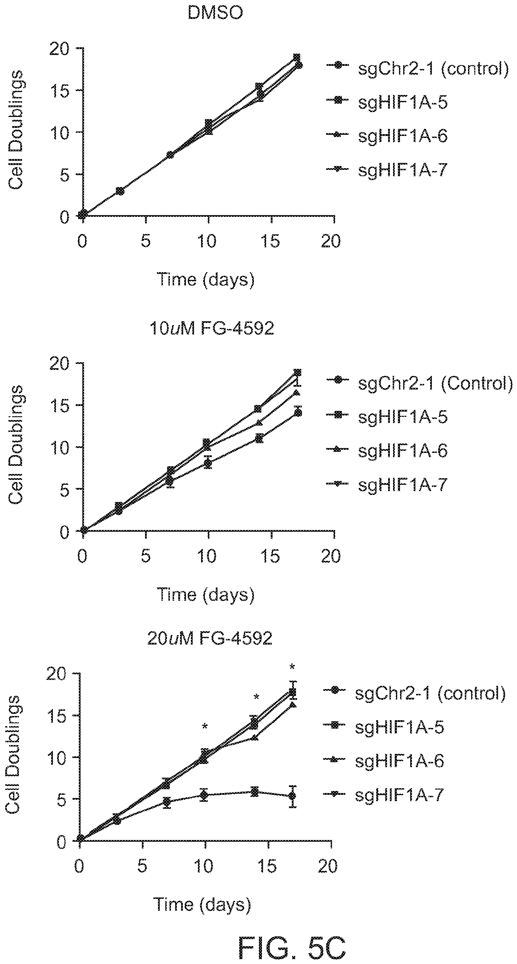

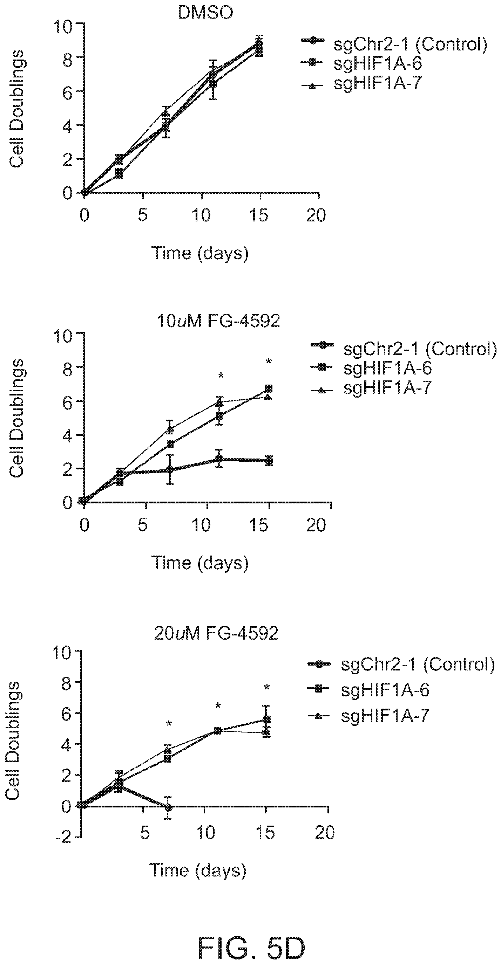

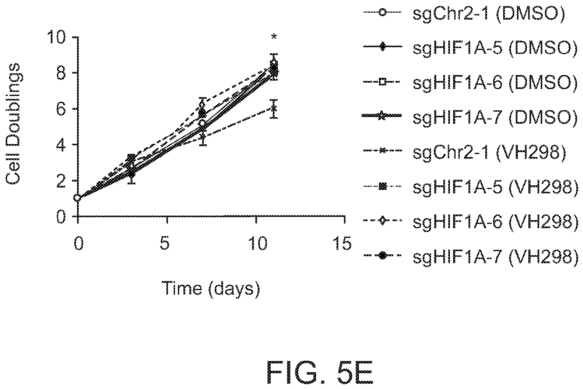

***FIGs. 5A to 5E*** demonstrate that depletion of HIF1A rescued cells from EGLN1-dependency. FIG. 5A shows the results of RNA sequencing performed on four ES2 and OVISE wildtype cells compared to four ES2 and OVISE EGLN1 knockout cells. Gene Set Enrichment Analysis of RNA sequencing data showed that HIF1A-related pathways were affected in EGLN1 KO cells. FIG. 5B shows an immunoblot that demonstrated the efficiency of control sgRNAs (sgChr2-1) or HIF1A deletion with sgRNAs (sgHIF1A-5, sgHIF1A-6, sgHIF1A-7) 48 hours post treatment with control (DMSO) or EGLN1 inhibitor (FG-4592). FIG. 5C shows that EGLN1-dependent ES2 cells were resistant to EGLN1 inhibition after HIF1A knockout. DMSO treatment (top) had no effect on control or HIF1A-knockout cells. Increasing treatment with FG-4592 (10uM middle, 20uM bottom) reduced proliferation of control cells (blue) but not HIF1A-knock. Results are representative of three independent experiments and data are graphed as mean + standard deviation of three replicates *p<0.05. FIG. 5D shows that EGLN1-dependent OVISE cells were resistant to EGLN1 inhibition after HIF1A knockout. Similar to ES2 cells DMSO treatment (top) had no effect on control or HIF1A-knockout cells. Increasing treatment with FG-4592 (10uM middle, 20uM bottom) reduced proliferation of control cells (blue). Results are representative of two independent experiments and data are graphed as mean + standard deviation of biological triplicates. *p<0.05. FIG. 5E shows that EGLN1-dependent ES2 cells were resistant to VHL1 inhibition after HIF1A knockout. Treatment with 100uM of VH298 significantly reduced proliferation of control cells (blue) compared to HIF1A knockout cells. Results are representative of two independent experiments and data are graphed as mean + standard deviation of biological triplicates. *p<0.05.

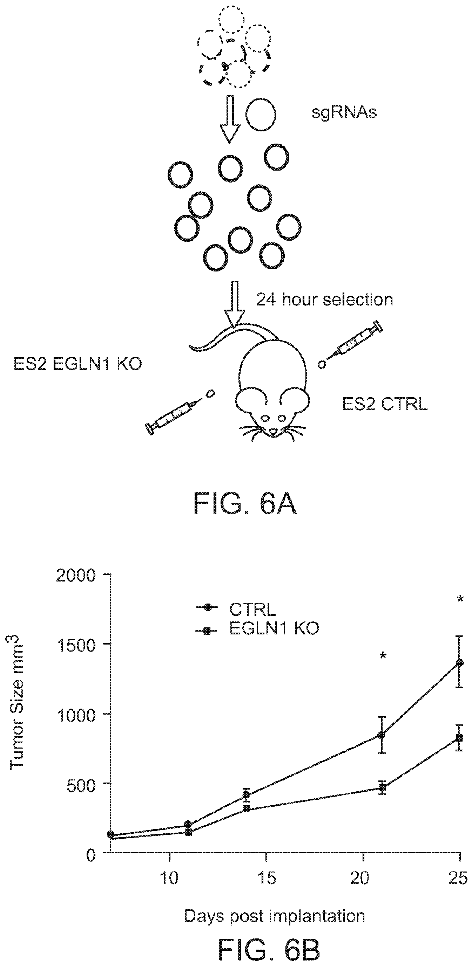

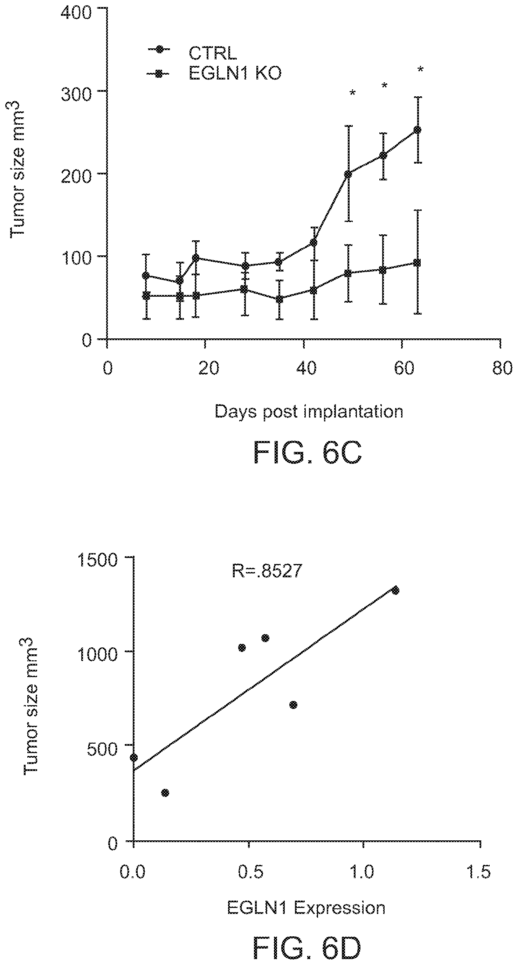

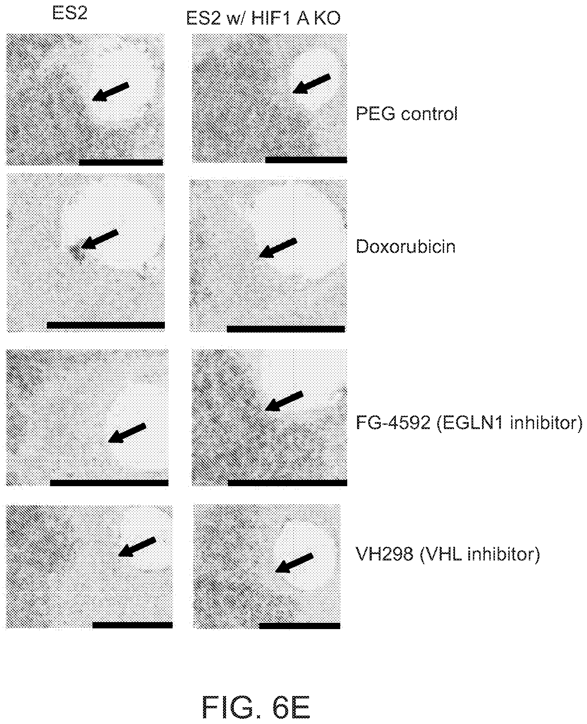

***FIGs. 6A to 6E*** demonstrate that EGLN1 deletion or inhibition reduced tumor formation induced by EGLN1-dependent cells. FIG. 6A shows a schematic of tumor formation experiments. Each recipient mouse received control (sgChr2-1) and EGLN1-KO cells. FIG. 6B shows that EGLN1 knockout impaired tumor formation induced by ES2 cells. Control and EGLN1 KO ES2 cells (100,000) were injected in parallel flanks subcutaneously and tumor size was measured. Data are represented as mean + standard deviation (n=15) and represent two experimental repeats *p<0.01. FIG. 6C shows that EGLN1 knockout impaired tumor formation induced by OVISE cells. Control and EGLN1 KO OVISE cells (250,000) were injected in parallel flanks subcutaneously and tumor size was measured. Data are represented as mean + standard deviation (n=6) *p<0.05. FIG. 6D shows that EGLN1 expression levels in the knockout cells correlated with tumor size. RNA was harvested from tumors and expression was quantified using qRT-PCR. Data are represented as the average of four biological replicates and represent two experimental repeats. FIG. 6E shows results for microdevice delivery of EGLN1 inhibitors and VHL inhibitors to ovarian cancer cells. Microdevices were loaded with PEG-formulated compounds and implanted into 1 cm² tumors formed from ES2 or 1 cm² tumors formed from ES2 with HIF1A KO. 48 Hours post microdevice implantation tumors were harvested and serially sectioned and stained. The control for drug formulation (PEG Control) had no effect on the proliferation of ES2 tumor cells with or without HIF1A (top). Doxorubicin treatment killed ovarian cancer cells and reduced tumor cell proliferation (top middle). Inhibition of EGLN1 with FG-4592 blocked proliferation of tumor cells while knockout of HIF1A rescued EGLN1 inhibition (bottom middle). Inhibition of VHL blocked proliferation of tumor cells while knockout of HIF1A rescued VHL inhibition (bottom). Black arrow indicates area where compound inside the microdevice was released. Figure is representative of three independent experiments. The black line is a scale bar of 1 mm.

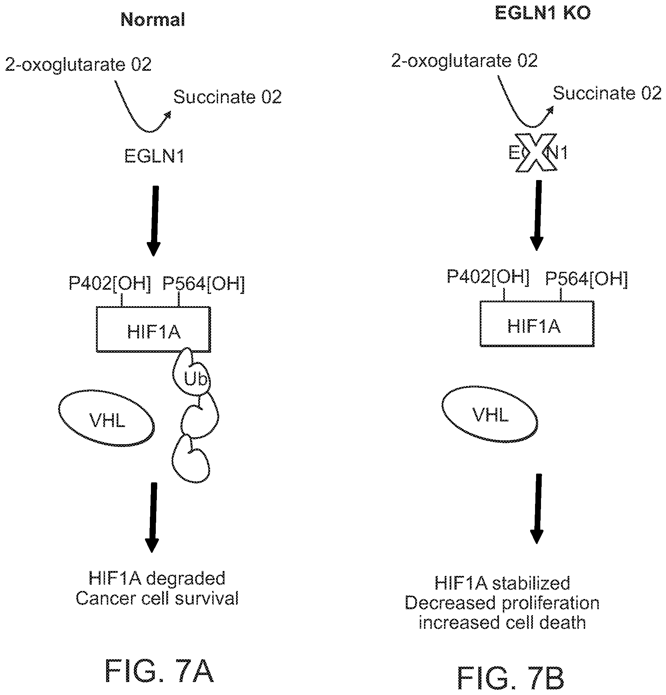

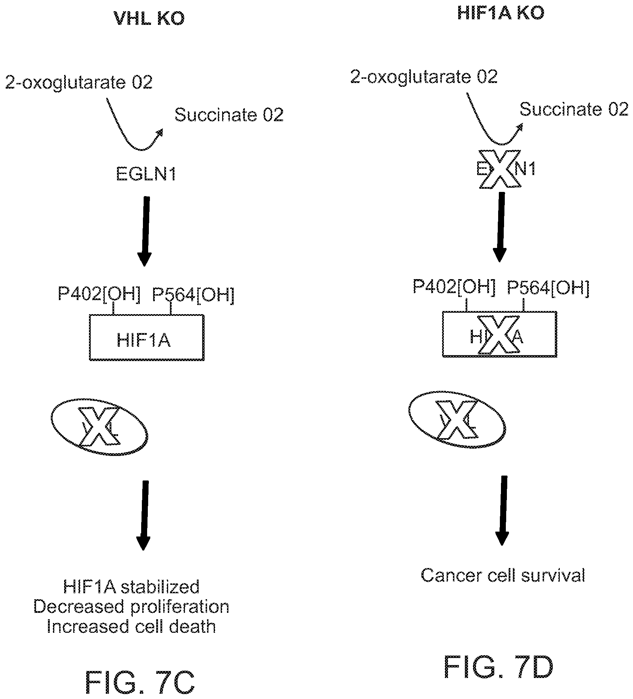

***FIGs. 7A to 7D*** depict a model of EGLN1 and VΣ-Π, dependency. FIG. 7A shows normal conditions, where EGLN1 in the presence of oxygen hydroxylated HIF1A at residues P402 and P564. Hydroxylated HIF1A was then targeted for ubiquitination and subsequent degradation by VHL. FIG. 7B shows an EGLN1 KO, where, after EGLN1 KO, HIF1A was non-hydroxylated and therefore not degraded by VHL. Stabilized HIF1A translocated to the nucleus and activated downstream effectors resulting in a decrease in cell viability and increased cell death. FIG. 7C shows a VHL KO, where, following KO of VHL, HIF1A was hydroxylated by EGLN1 but could not be ubiquitinated and degraded by VHL. Stabilized HIF1A translocated to the nucleus and activated downstream effectors resulting in a decrease in cell viability and increased cell death. FIG. 7D shows a HIF1A KO, where a HIF1A KO rescued cell proliferation and cell death seen in EGLN1 and VHL KO cells.

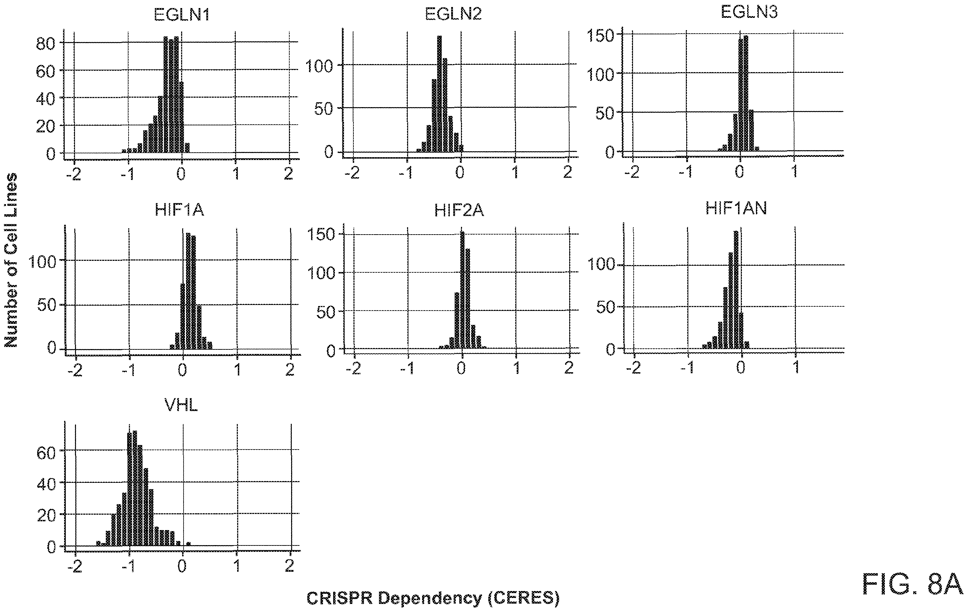

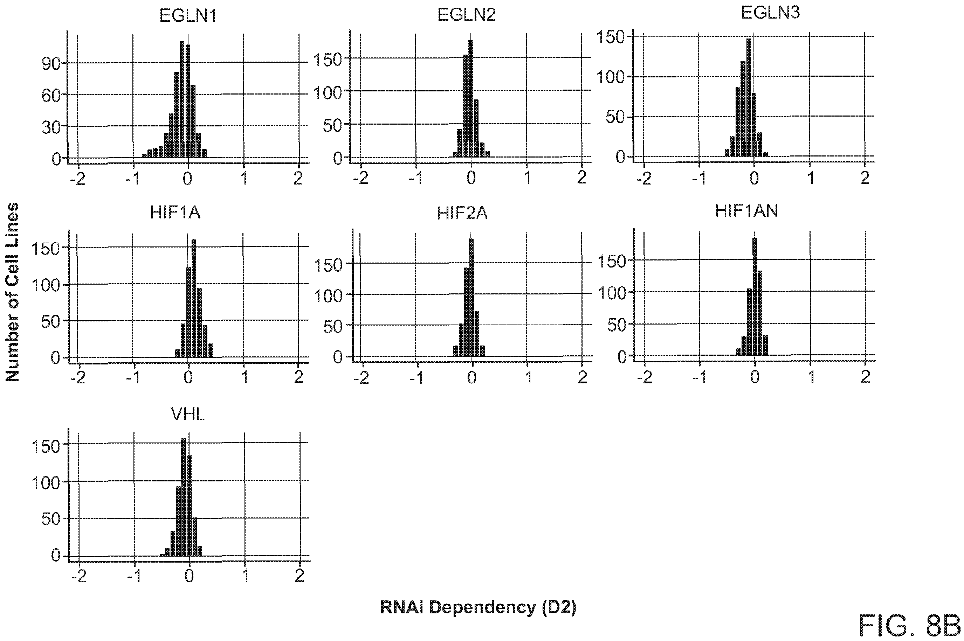

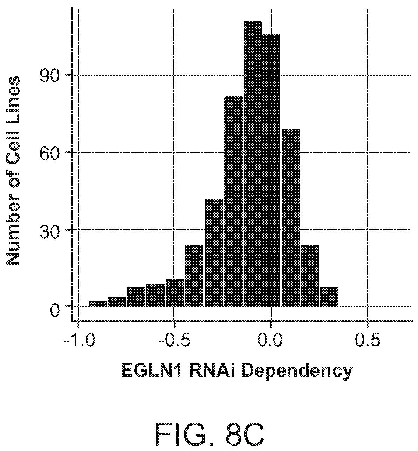

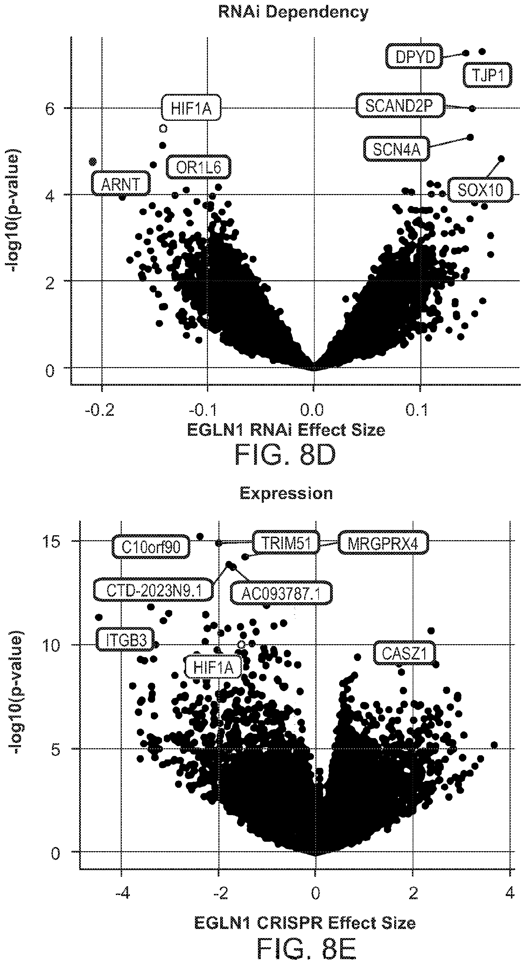

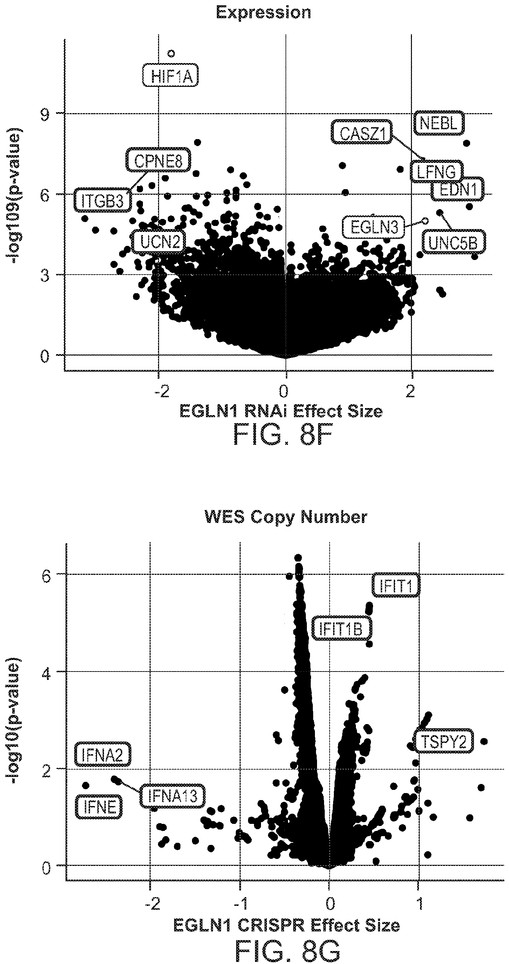

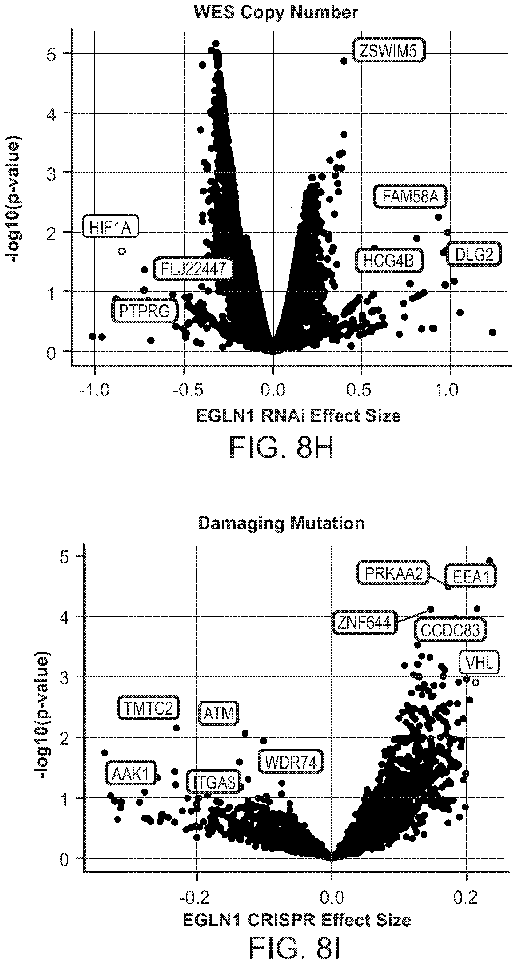

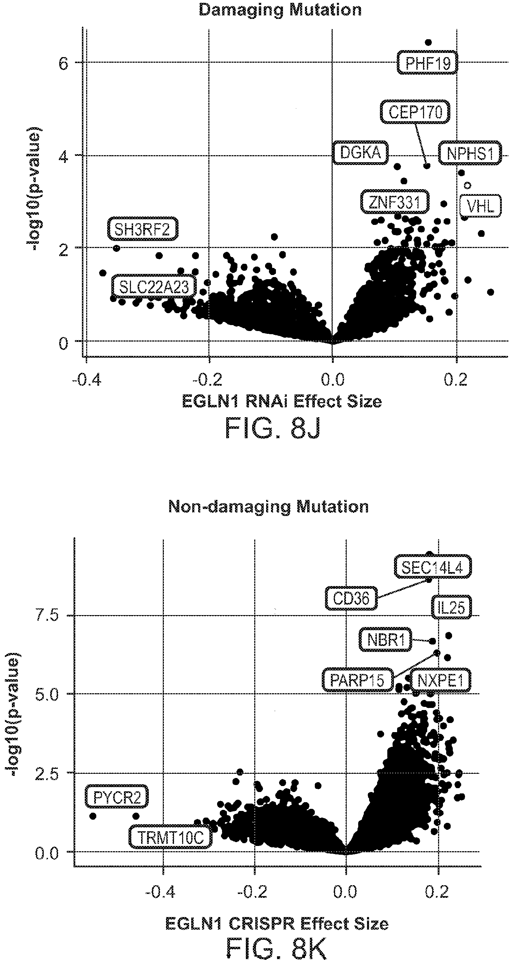

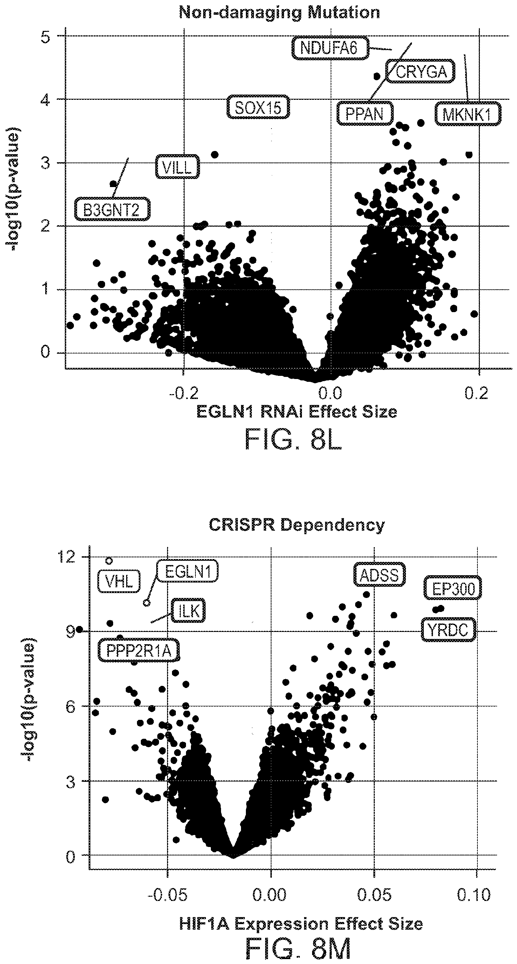

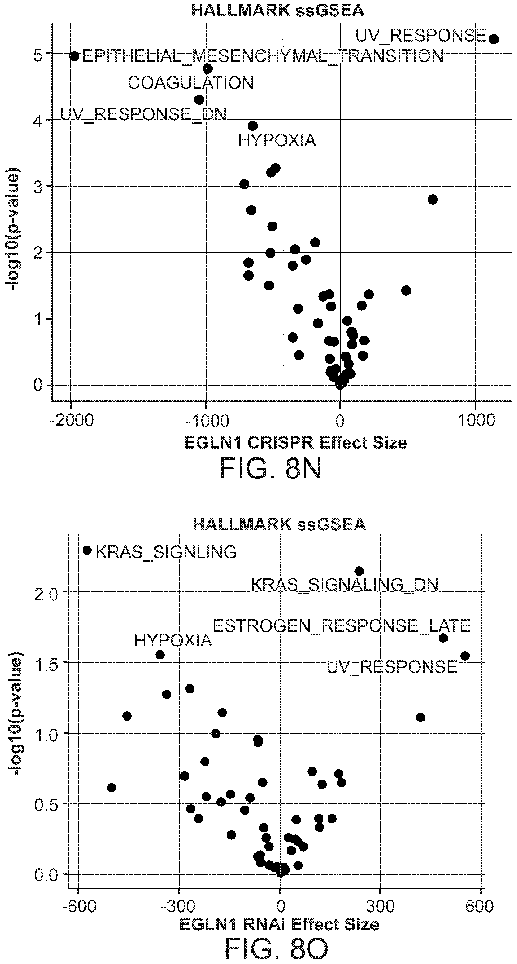

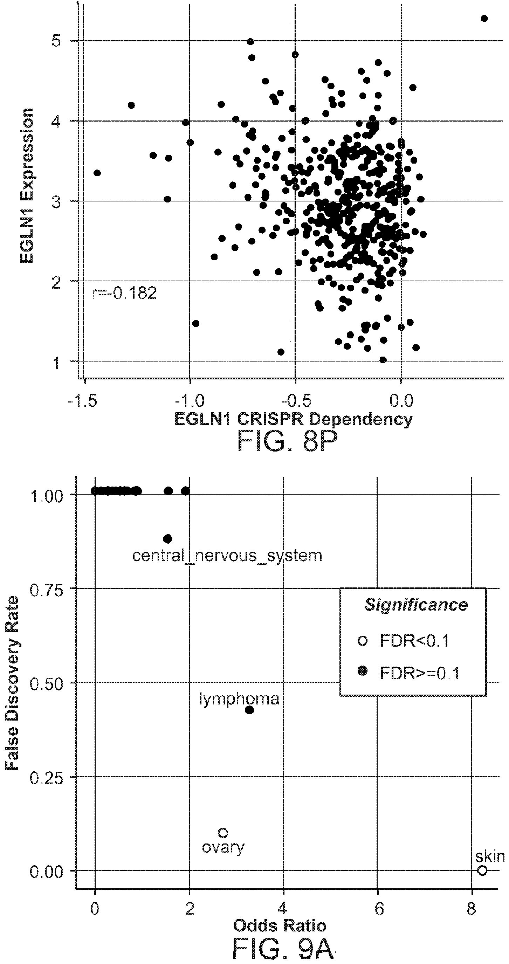

***FIGs. 8A to 8P*** demonstrate the identification of EGLN1 as a preferential cancer cell dependency. FIG. 8A shows identification of cancer cells dependent on EGLN1 using CRISPR-Cas9 data from Project Achilles. Histogram shows the distribution of CERES scores (X-axis) across 436 cancer cell lines screened with CRISPR. Data shows that EGLN1 and VHL exhibited the greatest number of dependent cell lines. FIG. 8B shows the identification of cancer cells dependent on EGLN1 using RNAi data from Project Achilles. Histogram shows the distribution of cancer cell lines (Y-axis) that were dependent on members of the EGLN1 pathway based on their DEMETER2 scores (X-axis). Data show that EGLN1 exhibited the greatest number of dependent cell lines. FIG. 8C shows identification of cancer cells dependent on EGLN1 using RNAi data from Project Achilles. Histogram shows the distribution of cancer cell lines (Y-axis) that were dependent on EGLN1 based on their DEMETER2 scores (X-axis). FIG. 8D shows a volcano plot showing cancer dependencies associated with EGLN1 dependency graphed as effect size (X-axis) against p-value (-log₁₀, Y-axis). Colored in red are other members of the EGLN1 pathway. FIGs. 8E-8L show volcano plots that show the top features associated with EGLN1 dependency by effect size and significance for both CRISPR (FIGs. 8E, 8G, 8I and 8K) and RNAi (FIGs. 8F, 8H, 8J and 8L), grouped by feature type (expression, whole exome copy number, mutation). Colored in red are other members of the EGLN1 pathway. (Data are summarized in Supplementary Tables 3 and 4.) FIG. 8E shows a volcano plot of gene expression associated with EGLN1 dependency graphed as effect size (X-axis) against p-value (-log₁₀, Y-axis) in the CRISPR dataset. Colored in red are other members of the EGLN1 pathway. FIG. 8F shows a volcano plot of gene expression associated with EGLN1 dependency graphed as effect size (X-axis) against p-value (-log10, Y-axis) in the RNAi dataset. Colored in red are other members of the EGLN1 pathway. FIG. 8G shows a volcano plot of genes with copy number alterations associated with EGLN1 dependency graphed as effect size (X-axis) against p-value (-log₁₀, Y-axis) in the CRISPR dataset. Colored in red are other members of the EGLN1 pathway. FIG. 8H shows a volcano plot of genes with copy number alterations associated with EGLN1 dependency graphed as effect size (X-axis) against p-value (-log₁₀, Y-axis) in the RNAi dataset. Colored in red are other members of the EGLN1 pathway. FIG. 8I shows a volcano plot of genes with damaging mutations associated with EGLN1 dependency graphed as effect size (X-axis) against p-value (-log₁₀, Y-axis) in the CRISPR dataset. Colored in red are other members of the EGLN1 pathway. FIG. 8J shows a volcano plot of genes with damaging mutations associated with EGLN1 dependency graphed as effect size (X-axis) against p-value (-log₁₀, Y-axis) in the RNAi dataset. Colored in red are other members of the EGLN1 pathway. FIG. 8K shows a volcano plot of genes with non-damaging mutations associated with EGLN1 dependency graphed as effect size (X-axis) against p-value (-log₁₀, Y-axis) in the CRISPR dataset. Colored in red are other members of the EGLN1 pathway. FIG. 8L shows a volcano plot of genes with non-damaging mutations associated with EGLN1 dependency graphed as effect size (X-axis) against p-value (-log₁₀, Y-axis) in the RNAi dataset. Colored in red are other members of the EGLN1 pathway. FIG. 8M shows a volcano plot of cancer dependencies associated with HIF1A expression graphed as p-value (-log₁₀, Y-axis) against effect size (X-axis). Colored in red are other members of the EGLN1 pathway. FIG. 8N shows that single-sample GSEA (ssGSEA) revealed pathways including Hypoxia as enriched in the CRISPR dataset. FIG. 8O shows that single-sample GSEA (ssGSEA) revealed pathways including Hypoxia as enriched in the RNAi dataset. FIG. 8P shows that there was no strong correlation observed between EGLN1 expression (Y-axis) in cell lines and dependency on EGLN1 (X-axis).

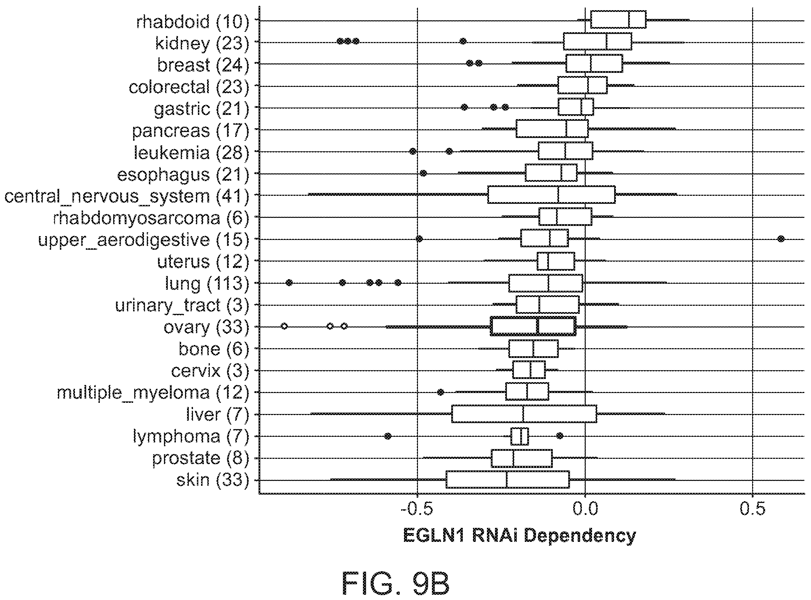

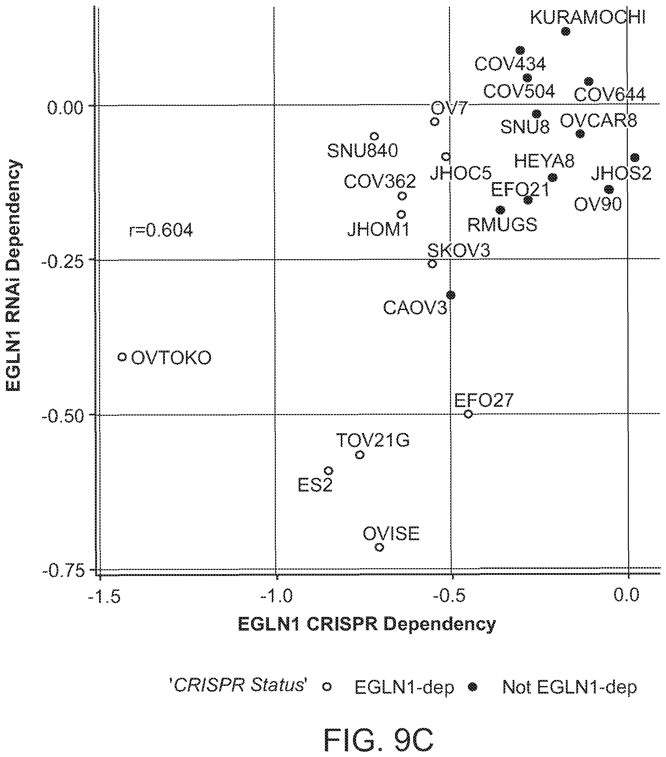

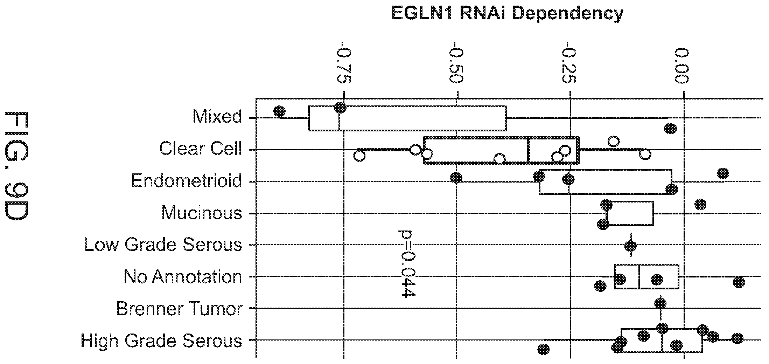

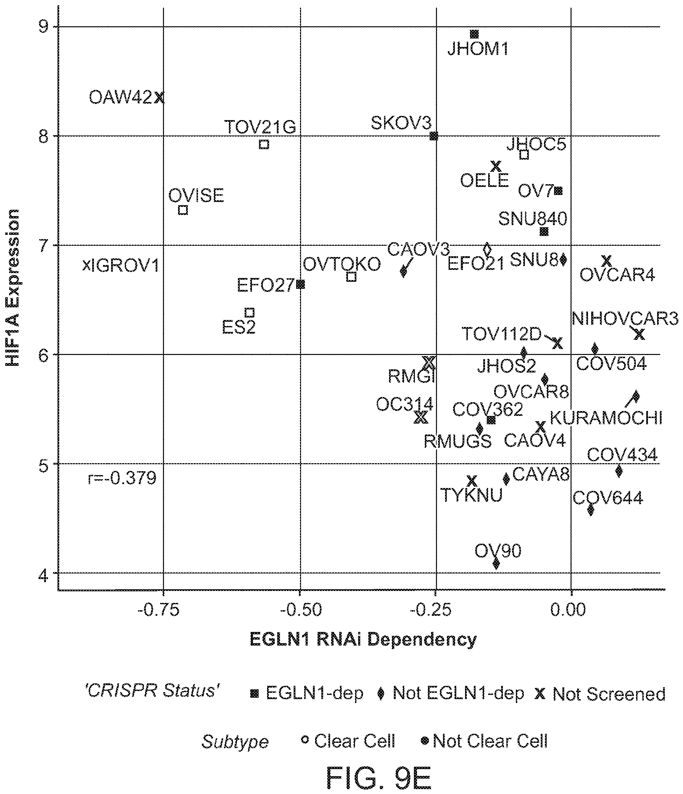

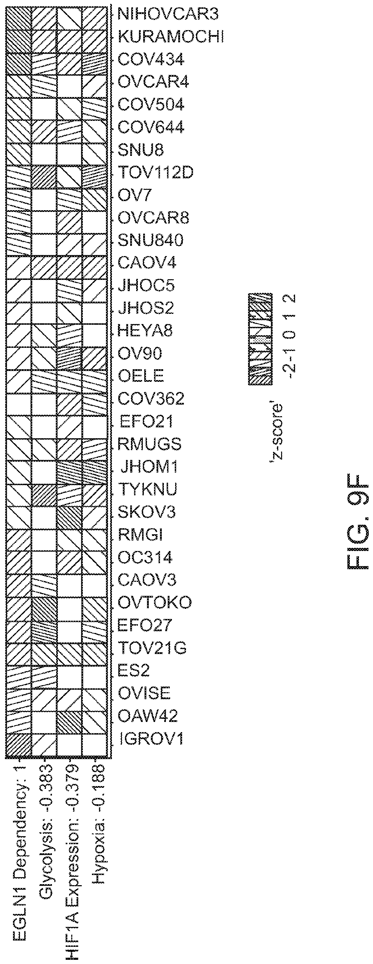

***FIGs. 9A to 9F*** demonstrate that EGLN1 dependency was enriched in clear cell ovarian cancer and melanoma, and was associated with high HIF1A levels. FIG. 9A shows that a lineage enrichment analysis revealed that ovarian and melanoma lines were significantly (FDR < 0.1) and positively enriched for EGLN1-dependent lines in CRISPR. FIG. 9B shows that ovarian cancer cell lines exhibited strong EGLN1 dependencies in RNAi screens. DEMETER2 scores (X-axis) are stratified by lineage (Y-axis). FIG. 9C shows that the strongest EGLN1 dependencies in RNAi were also EGLN1-dependent in CRISPR datasets. Concordance between CRISPR CERES scores and RNAi DEMETER2 scores was strong in ovarian cancer (r = 0.604). FIG. 9D shows that RNAi EGLN1 dependencies were enriched in clear cell ovarian cancer. A two-sample t-test of clear cells vs. all other ovarian subtypes revealed that clear cells were significantly enriched for EGLN1 dependencies (p < 0.05). FIG. 9E shows that strong RNAi EGLN1 dependencies expressed high levels of HIF1A. There was a strong correlation between HIF1A expression and EGLN1 dependency in the RNAi dataset (r = -0.379). FIG. 9F shows that ssGSEA (single sample Gene Set Enrichment Analysis) revealed that EGLN1 dependency in RNAi was associated with HIF1A-related pathways. It is noted herein that some of the strongest DEMETER2 dependencies (IGROV1 and OAW42) were not screened in CRISPR. Pearson correlations of EGLN1 dependency with each profile (Y-axis) were calculated, and z-score normalized profiles are shown.

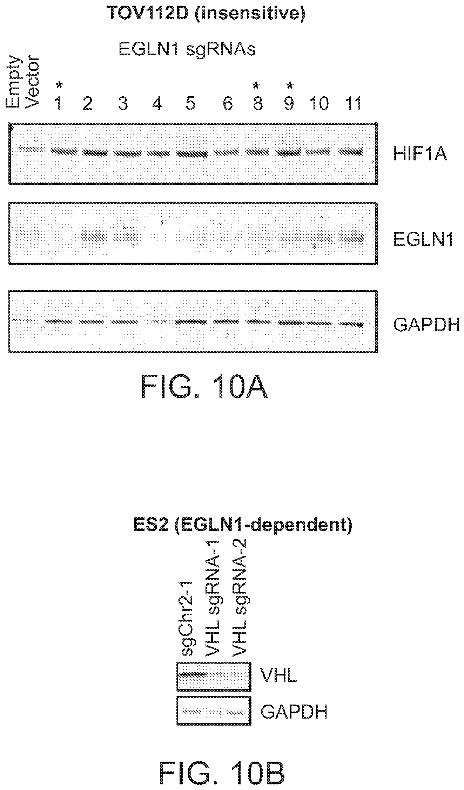

***FIGs. 10A and 10B*** show immunoblots demonstrating the effects of EGLN1 and VΣ-Π, deletion. FIG. 10A shows the effects of EGLN1 deletion on HIF1A levels. Whole cell lysates of TOV112D-Cas9 were contacted with the indicated sgRNAs. Equal amounts of protein were analyzed by immunoblotting with GAPDH included as a marker of equal loading. Deletion of EGLN1 led to increased HIF1A but not HIF1A phosphorylation. Asterisk indicates sgRNAs used for subsequent experiments. FIG. 10B shows a western blot that confirmed the VHL deletion. Whole cell lysates of ES2-Cas9 cells with VHL sgRNA-1 and VHL sgRNA-2 added to cells were prepared for western blotting. Equal amounts of protein were analyzed by immunoblotting with GAPDH used as a marker of equal loading. The immunoblot shows that the addition of the sgRNAs resulted in deletion of VHL.

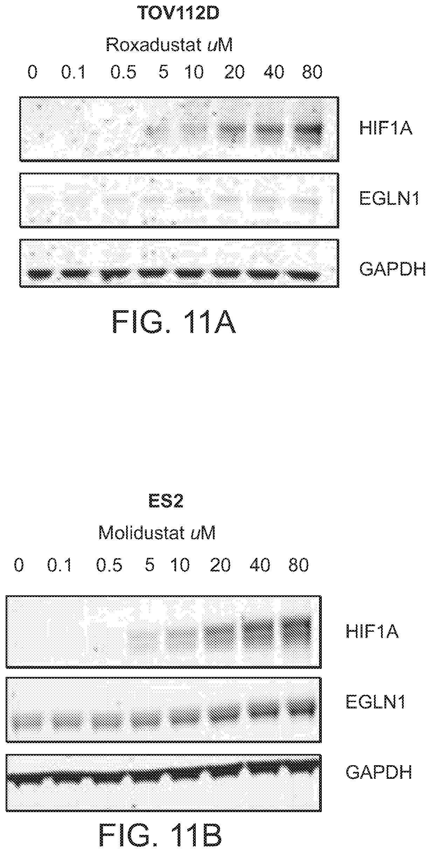

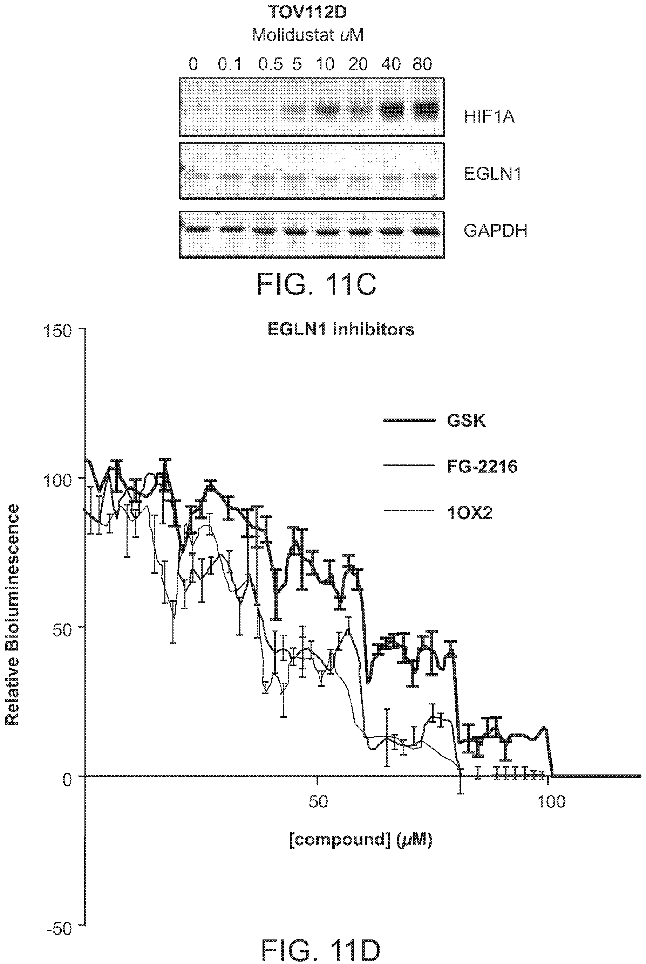

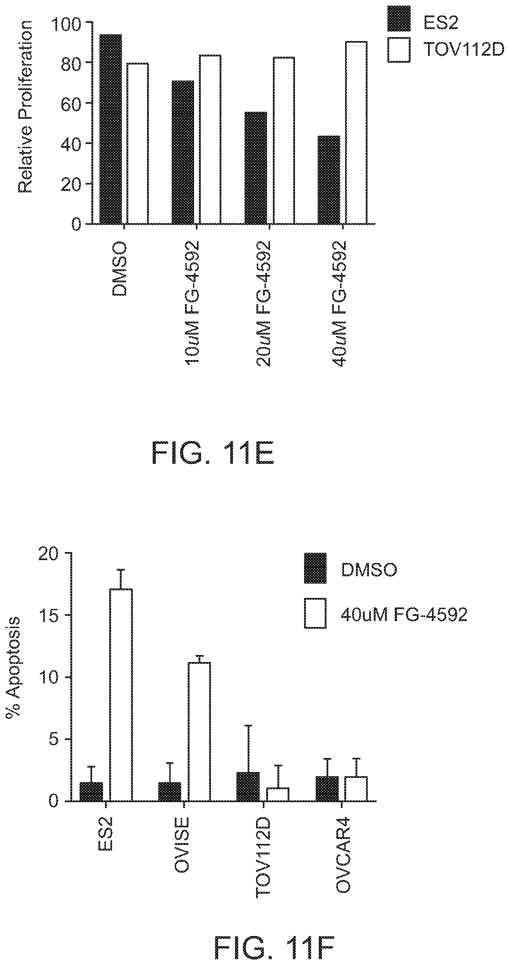

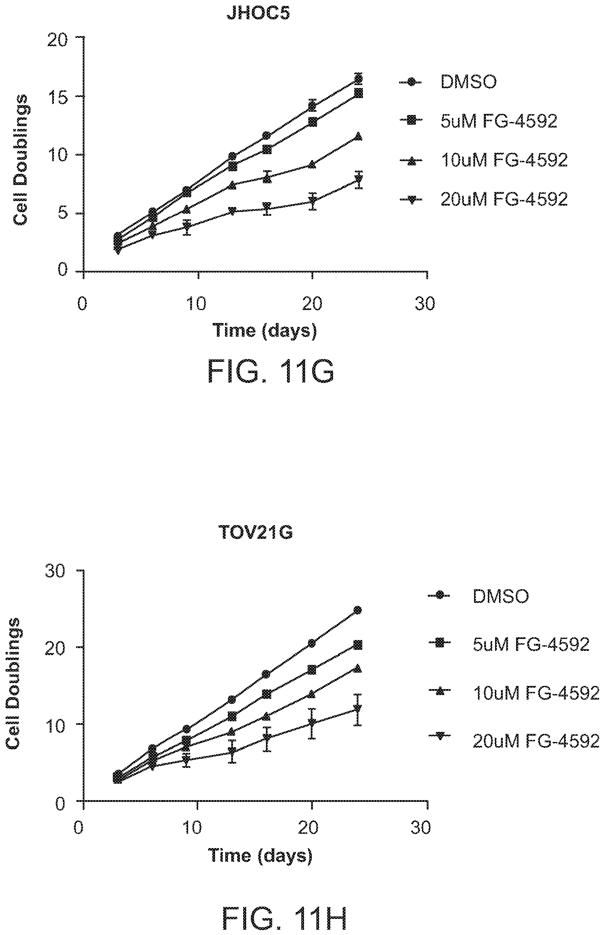

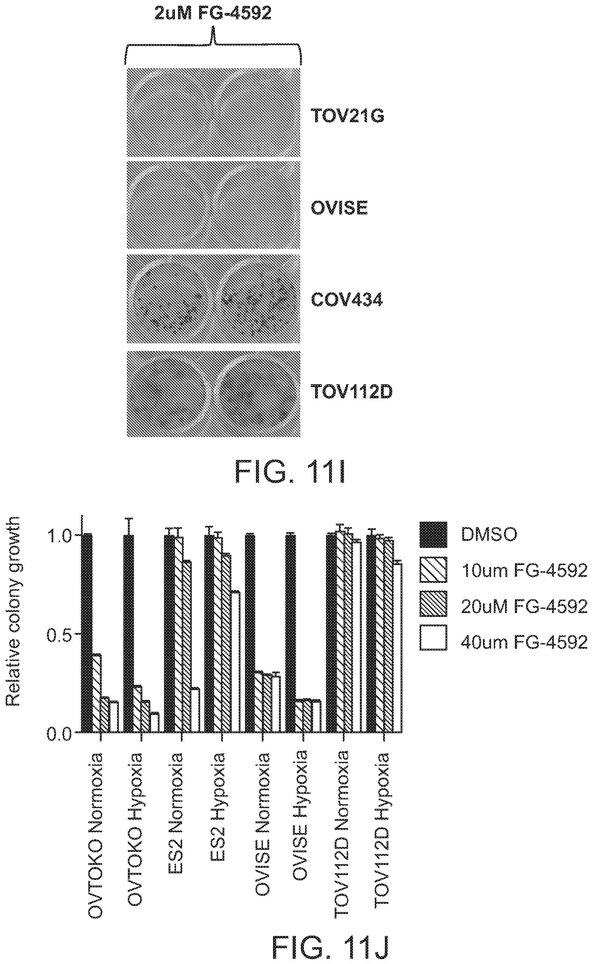

***FIGs. 11A to 11J*** demonstrate that inhibiting EGLN1 increased HIF1A expression and reduced proliferation in EGLN1-dependent cells. FIG. 11A shows an immunoblot demonstrating that pharmacological inhibition (Roxadustat - FG-4592) of EGLN1 increased HIF1A levels in a dose-dependent manner in TOV112D. FIG. 11B shows an immunoblot demonstrating that pharmacological inhibition (Molidustat - Bay 85-3934) of EGLN1 increased HIF1A levels in a dose-dependent manner in ES2. FIG. 11C shows an immunoblot demonstrating that pharmacological inhibition (Molidustat - Bay 85-3934) of EGLN1 increased HIF1A levels in a dose-dependent manner in TOV112D. FIG. 11D shows cellular viability as measured by CellTiter-Glo of ES2 treated with various EGLN1 inhibitors, including IOX2, FG-2216 and Daprodustat (GSK1278863). IC50 was observed around 40-50 µM for most of these compounds, as observed with FG-4592 and Bay 85-3934. FIG. 11E shows cellular viability as assessed through flow cytometry. Increasing concentration of FG-4592 reduced cell proliferation in ES2 while increasing concentration of FG-4592 did not reduce cell proliferation in TOV112D. Proliferation was measured by CFSE stain over 48 hours. FIG. 11F shows apoptosis assessed through flow cytometry. Treatment with 40 µM of FG-4592 increased apoptosis in ES2 and OVISE cell lines while not increasing apoptosis in TOV112D and OVCAR4 cell lines. Apoptosis was measured by Annexin V stain after 48 hours of treatment. FIG. 11G shows that EGLN1 inhibitor FG-4592 reduced long term proliferation in EGLN1-dependent cell line JHOC5 in a dose-dependent manner. Results are representative of 3 independent experiments and the data are graphed as the mean + standard deviation of 3 replicates. FIG. 11H shows that EGLN1 inhibitor FG-4592 reduced the long term proliferation of the EGLN1-dependent cell line TOV21G in a dose-dependent manner. Results are representative of 3 independent experiments and the data are graphed as the mean + standard deviation of 3 replicates. FIG. 11I shows that EGLN1 inhibitor FG-4592 reduced colony formation in EGLN1-dependent cell lines and had no effect on not EGLN1-dependent cell lines. Results are representative of 3 independent experiments. FIG. 11J shows that inhibition of EGLN1 reduced cell viability in hypoxia. Cells were cultured for up to 11 days in 5% hypoxia or normoxia in a colony forming assay. Inhibition of EGLN1 reduced relative colony growth over time in EGLN1-dependent cell lines OVTOKO, OVISE and ES2. Inhibition of EGLN1 in cell line TOV112D, which is not dependent on EGLN1, did not reduce colony growth.

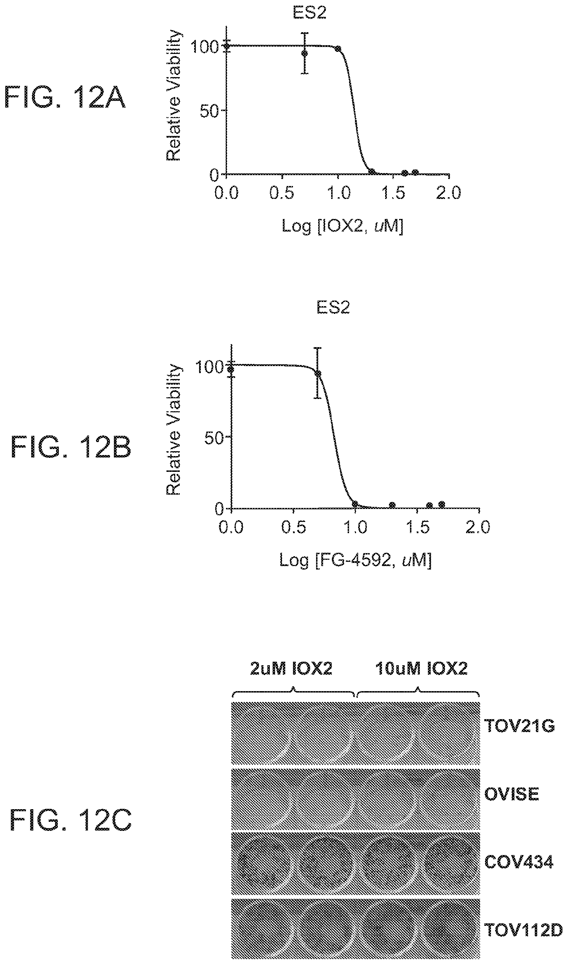

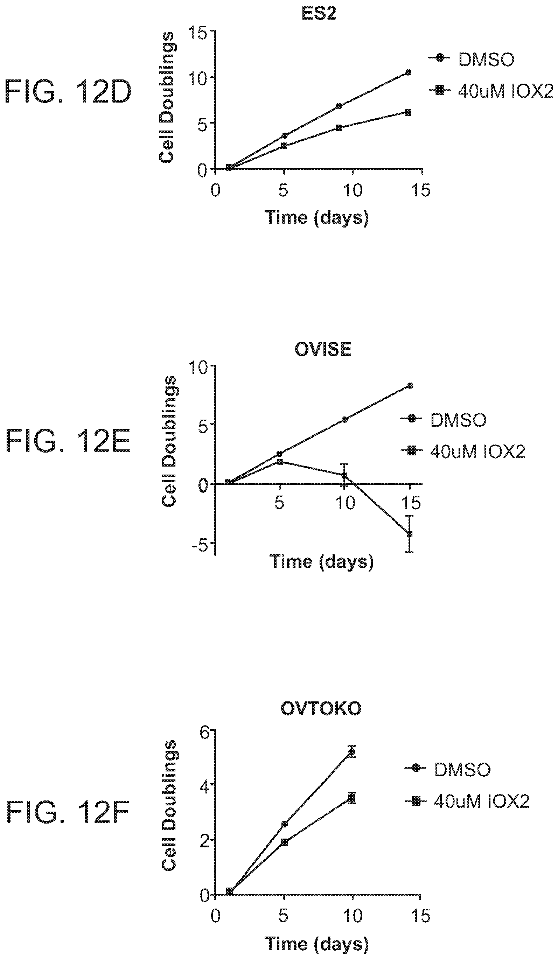

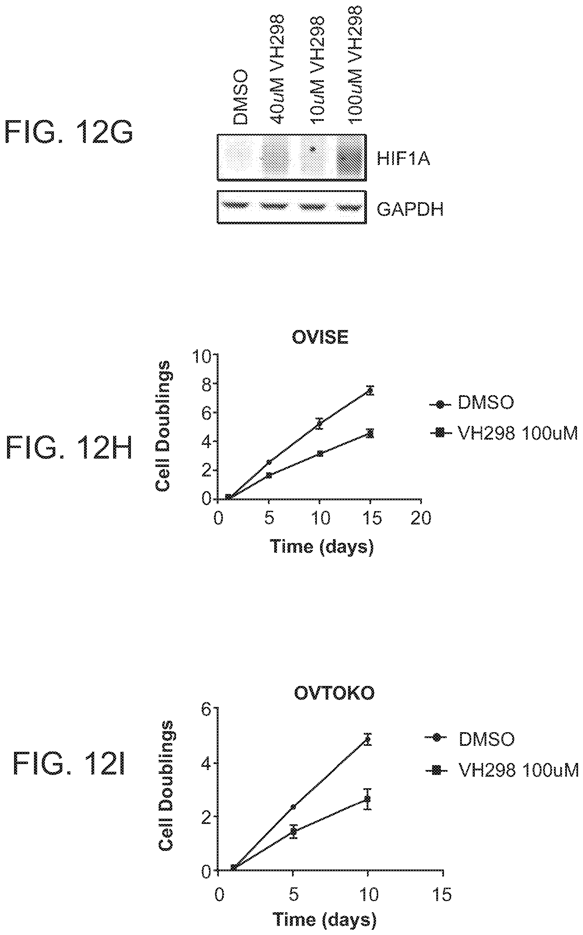

***FIGs. 12A to 12I*** demonstrate that inhibiting EGLN1 or VHDL reduced proliferation in EGLN1-dependent cells. FIG. 12A shows that inhibition of EGLN1 using small molecule inhibitor IOX2 reduced viability in a dose-dependent manner. Cells were treated with IOX2 over 5 days and viability was measured using CTG. Results are representative of 2 independent experiments and data are graphed as mean + standard deviation of 3 replicates. FIG. 12B shows that inhibition of EGLN1 using small molecule inhibitor FG-4592 reduced viability in a dose-dependent manner. Cells were treated with FG-4592 over 5 days and viability was measured using CTG. Results are representative of 3 independent experiments and the data are graphed as the mean + standard deviation of 3 replicates. FIG. 12C shows that EGLN1 inhibitor IOX2 reduced colony formation in EGLN1-dependent cell lines and had no effect on not EGLN1-dependent cell lines. Results are representative of 3 independent experiments. FIG. 12D shows that EGLN1 inhibitor IOX2 reduced long term proliferation in EGLN1-dependent cell line ES2 in a dose-dependent manner. Results are representative of 2 independent experiments and the data are graphed as the mean + standard deviation of 3 replicates. FIG. 12E shows that EGLN1 inhibitor IOX2 reduced long term proliferation in EGLN1-dependent cell line OVISE in a dose-dependent manner. Results are representative of 2 independent experiments and the data are graphed as the mean + standard deviation of 3 replicates. FIG. 12F shows that EGLN1 inhibitor IOX2 reduced long term proliferation in EGLN1-dependent cell line OVTOKO in a dose-dependent manner. Results are representative of 2 independent experiments and the data are graphed as the mean + standard deviation of 3 replicates. FIG. 12G shows an immunoblot demonstrating that pharmacological inhibition (VH298) of VHL increased HIF1A levels in a dose-dependent manner in ES2 cells. FIG. 12H shows that the VHL inhibitor VH298 reduced long term proliferation in the EGLN1-dependent cell line OVISE in a dose-dependent manner. Results are representative of 2 independent experiments and the data are graphed as the mean + standard deviation of 3 replicates. FIG. 12I shows that the VHL inhibitor VH298 reduced long term proliferation in the EGLN1-dependent cell line OVTOKO in a dose-dependent manner. Results are representative of 2 independent experiments and the data are graphed as the mean + standard deviation of 3 replicates.

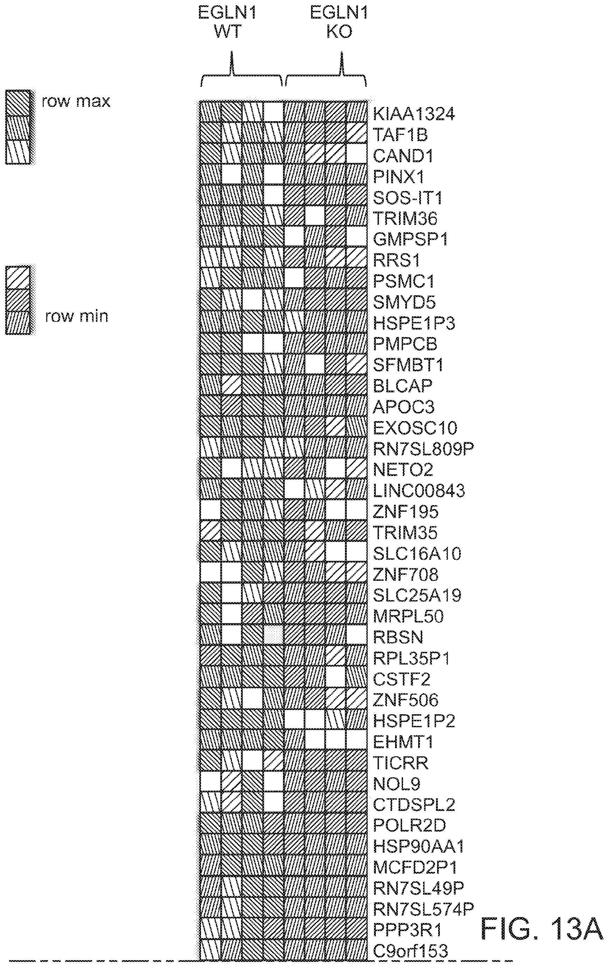

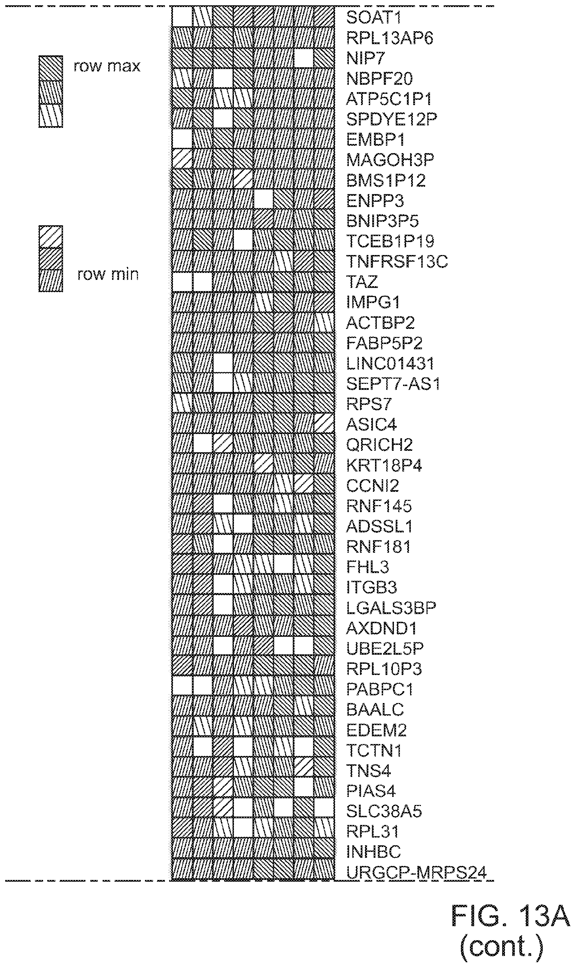

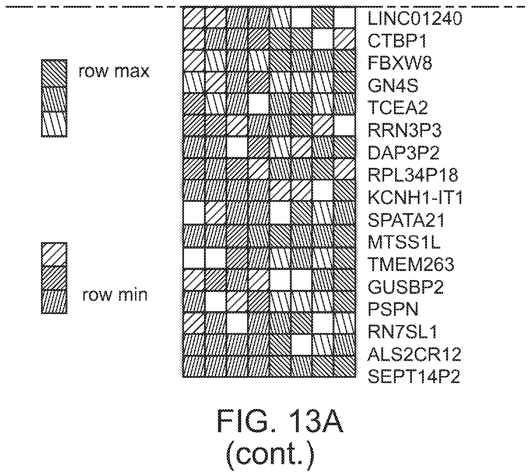

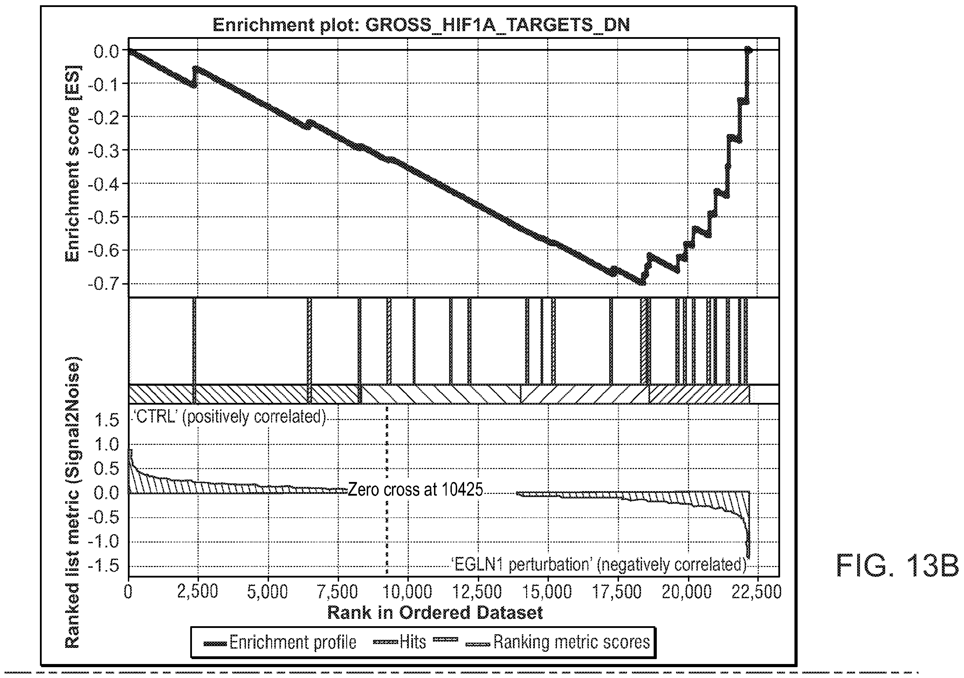

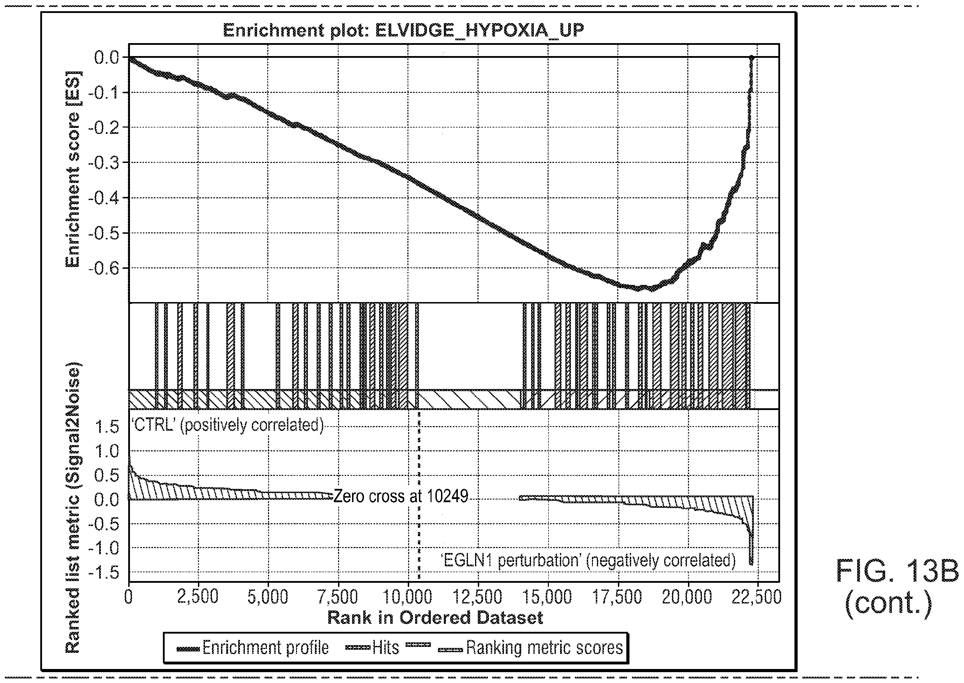

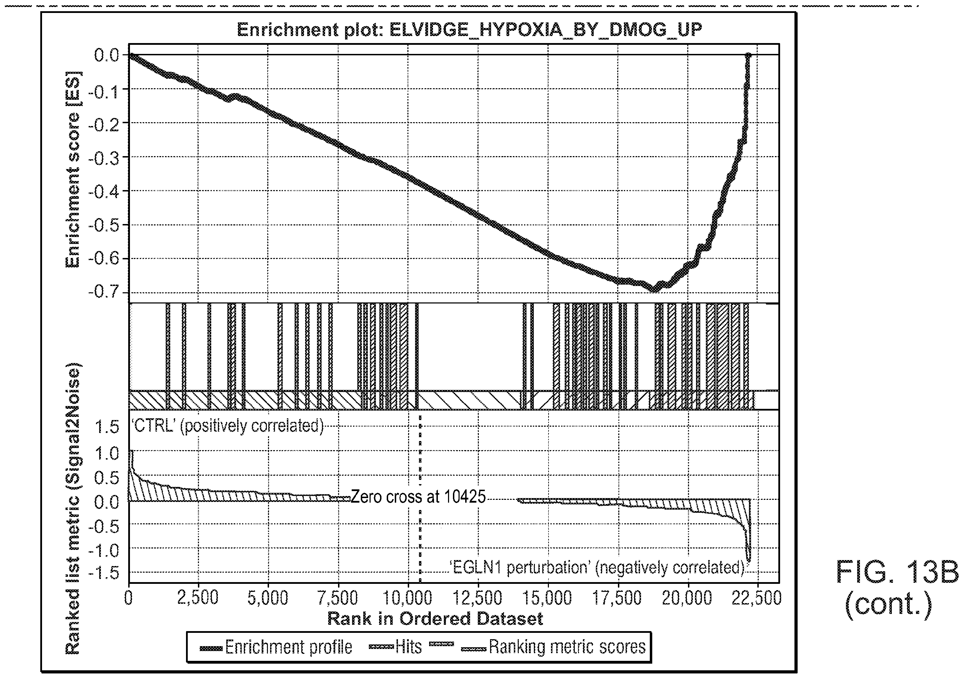

***FIGs. 13A*** ***and*** ***13B*** demonstrate that RNA-sequencing of EGLN1-dependent cells showed altered HIF1A signatures after EGLN1 knockout (KO). FIG. 13A shows RNA-seq of ES2 and OVISE cells with control (sgChr2-1, Lanes 1-4) sgRNAs or EGLN1 (sgEGLN1-9, Lanes 5-8) sgRNAs. Red = high, Blue = low. FIG. 13B shows Gene Set Enrichment Analysis (GSEA) of differentially expressed genes in EGLN1 KO cells compared to EGLN1 WT cells. Several of the top scoring pathways related to HIF1A-related genes or the Hypoxia pathway.

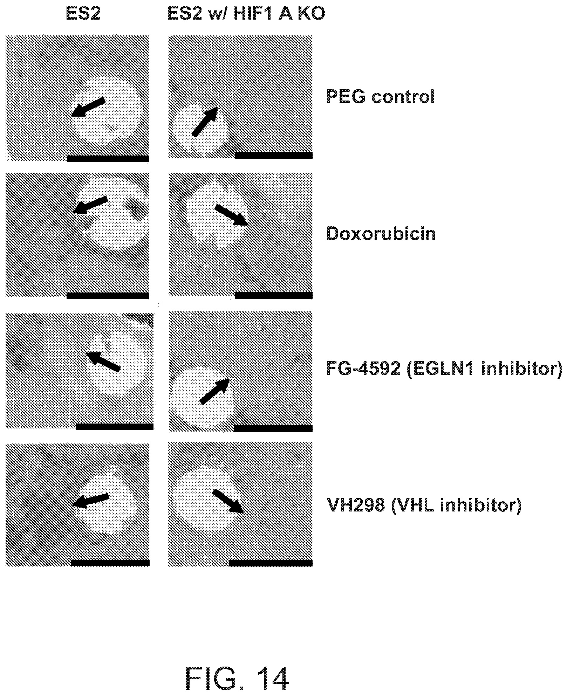

***FIG. 14*** shows that small molecule inhibition of EGLN1 or VHL *in vivo* increased apoptosis. EGLN1 inhibitors and VHL inhibitors were delivered to HIF1A-expressing ovarian tumors *via* microdevice, which resulted in increased apoptosis. Microdevices were loaded with PEG-formulated compounds and implanted into 1 cm² tumors formed from ES2 or from ES2 with HIF1A KO and grown to 1 cm². At 48 hours post microdevice implantation, tumors were harvested and serially sectioned and stained. The control for drug formulation (PEG Control) had no effect on the cell death of ES2 tumor cells with or without HIF1A (top). Doxorubicin treatment increased tumor cell apoptosis (top middle). Inhibition of EGLN1 with FG-4592 increased apoptosis of tumor cells, while knockout of HIF1A rescued EGLN1 inhibition (bottom middle). Inhibition of VHL with VH298 increased apoptosis of tumor cells, while knockout of HIF1A rescued VHL inhibition. This figure is representative of three independent experiments. Arrow shows area where drug was released. Black line is a scale bar indicating 1 mm.

### DETAILED DESCRIPTION OF THE INVENTION

The present disclosure is directed, at least in part, to the discovery that certain types of cancer exhibit EGLN1-dependency. Most notably, melanoma and clear cell ovarian cancers were identified as cancers for which EGLN1-dependency is prevalent. The instant disclosure therefore provides compositions and methods for the diagnosis and treatment of EGLN1-dependent cancers, with such treatment particularly performed *via* administration of EGLN1 inhibitors and/or VΣ-Π, inhibitors.

Identification of a cancer as EGLN1-dependent can be performed via performance of knockdown and/or knockout of EGLN1 in a particular sample, cell, cell line, tissue and/or subject, as exemplified herein.EGLN1 and VHL have therefore herein been identified as a therapeutic target for EGLN1-dependent cancers (especially including melanoma and ovarian cancers, particularly clear cell ovarian cancers), and EGLN1 and VHL inhibitory agents have been specifically discovered herein as an effective therapeutic selection for cancers that exhibit EGLN1 dependency. Compositions and methods for identification of an EGLN1-dependent cancer and selection of a therapeutic that includes an EGLN1 and/or VΣ-Π, inhibitory agent (either as a single therapeutic or as a component of a combination therapy) are described in additional detail below.

In the instant disclosure, to identify potential cancer targets, genome-wide loss-of-function screens were performed in hundreds of cancer cell lines, to identify genes that were necessary for the proliferation/survival of subsets of cancer cells. It was hypothesized herein that candidate dependencies for which there are small molecules that are either FDA approved or in advanced development for a non-oncology indication would likely represent potential therapeutic targets. To test this hypothesis, genome-scale loss-of-function screens were performed herein in hundreds of cancer cell lines. It was herein identified that knockout of *EGLN1,* which encodes PHD2/Prolyl Hydroxylase Domain-Containing Protein 2 (PHD2) reduced the proliferation of a subset of clear cell ovarian cancer cell lines *in vitro.* EGLN1-dependent cells exhibited sensitivity to the pan-EGLN inhibitor FG-4592. The response to FG-4592 was reversed by deletion of HIF 1A (Hypoxia Inducible Factor 1 Alpha), which demonstrated that EGLN1 dependency was related to negative regulation of HIF1A. It was also observed herein that ovarian clear cell tumors that were susceptible to both genetic and pharmacological inhibition of EGLN1 required intact HIF1A. The instant observations therefore identified EGLN1 as a cancer target possessing therapeutic potential.

The development of genome scale methods to interrogate the function of genes now provides a path to systematically identify genes that are essential for cell survival. Several studies using RNAi and more recently CRISPR-Cas9 to suppress or delete genes have identified cell essential genes in human cancer cell lines (1-8). Compared to similar experiments in yeast (9, 10), these studies have identified a larger number of essential genes in human cells (7, 10-12).

Similar approaches have been used to identify genes that are essential for subsets of cancer cell lines (7, 10-13). A number of screens involving a modest number of cell lines have identified several cancer dependencies related to oncogenic mutation (12) or genes that become required in the setting of loss of a paralog (11, 13). In recent work, such efforts have been expanded to hundreds of cancer cell lines and several types of dependencies have thereby been defined that occur in subsets of cancers (2, 5, 7, 12, 13). A comprehensive map of cancer dependencies can ultimately be provided.

A direct downstream sensor of oxygen tension is the family of prolyl hydroxylases called EGLN (Egg-laying defective nine homolog). When oxygen is present, EGLN, also known as hypoxia-inducible factor prolyl hydroxylase (HIF-PH) and prolyl hydroxylase domain-containing protein (PHD), hydroxylates HIF1 which results in a binding site for a ubiquitin ligase complex that includes VHL, leading to HIF1 degradation (14-17). This leads to HIF1 degradation by forming a binding site for a ubiquitin ligase complex that includes VHL (14-17). Among the three EGLN family members, EGLN1 is generally regarded as the primary HIF1 hydroxylase, while EGLN2 and EGLN3 regulate HIF1 under specific conditions (15-17). With decreased concentrations of oxygen, EGLN-mediated hydroxylation of HIF1 is reduced which leads to increased levels of HIF1 (17). When HIF1 is increased under low oxygen conditions, the α subunit heterodimerizes with the β subunit and translocates to the nucleus to activate genes that promote glucose uptake, glycolysis and decreases oxidative phosphorylation (17).

As described herein, an effort was initiated to identify differential dependencies by interrogating the data derived from screening a large number of cell lines with genome scale RNAi and CRISPR-Cas9 libraries. From a list of nominated strong preferential dependencies, EGLN1 was thereby newly identified as a dependency preferentially required for the viability of a subset of cancer cell lines, most notably in clear cell ovarian cancer and a subset of melanoma cell lines.

While EGLN1 has been described previously as an oncogene and a tumor suppressor (36, 42, 49, 51), a specific dependency of a subset of cancers on *EGLN1* has not been previously reported. Jokilehto *et al.* (36) found that *EGLN1* expression was strongly associated with highly proliferative head and neck squamous cell carcinomas (HNSCC). Furthermore, Klotzsche-von Ameln *et al.* found EGLN1 inhibition led to a decrease in tumor growth using mouse lung cancer cell line LLC and osterocarcoma line LM8 (49). Conversely, Chan *et al.* found that low PHD2 expression in colorectal cancers correlated with poorer survival, and experiments in cell line models showed that low PHD2 expression correlated with increased tumor vasculature (50). Bordoli *et al.* observed decreased EGLN1 was associated with increased tumor growth through increased VEGF, Amphiregulin and IL-8 (51). Thus, EGLN1 is another of a growing number of genes that acts both as an oncogene and tumor suppressor gene depending on the context (52).

### Identification of EGLN1 Dependency in a Cell, Tissue and/or Cancer

Identification of a tissue, tumor and/or cancer of a subject as exhibiting EGLN1 dependency can be performed by any method available in the art. Certain methods and compositions described herein relate to identification of a cancer as exhibiting EGLN1 dependency based upon EGLN1 gene-specific knockout and/or knockdown performed upon the cancer (upon the cell, cell line, sample, tissue and/or subject having or at risk of developing a cancer that is EGLN1-dependent).

In certain embodiments, detection of elevated HIF1A levels can readily be performed, e.g., via assessment of mRNA expression levels (e.g., via real-time PCR or other such quantitative method) and/or immunoassay for detection of HIF1A protein levels.

In certain aspects, the instant disclosure provides methods and kits that involve and/or allow for assessment of the presence or absence of EGLN1 dependency in a sample, cell, cell line, tissue and/or subject. In particular embodiments, a subject, tissue, cell and/or sample is assessed for the presence of an EGLN1-dependent phenotype (indicative of an EGLN1-dependent cancer that is likely to respond to an EGLN1- and/or VHL-targeting therapeutic agent).

### EGLN1-Dependent Cancers

As noted herein, certain types of cancer have been identified herein in which EGLN1 dependency is relatively more prevalent than for other cancer types. Such cancers for which EGLN1 dependency is prevalent include melanoma and ovarian cancers (and among ovarian cancers, particularly clear cell ovarian cancers). Consideration of EGLN1 dependency prevalence within such cancer populations is detailed below and elsewhere herein.

### Melanoma

Melanoma, also known as malignant melanoma, is a type of cancer that develops from the pigment-containing cells known as melanocytes ("Melanoma Treatment-for health professionals (PDQ®)". National Cancer Institute. June 26, 2015). Melanomas typically occur in the skin, but may rarely occur in the mouth, intestines, or eye (NCI; World Cancer Report 2014 (PDF). World Health Organization. 2014. pp. Chapter 5.14. ISBN 9283204298). In women, they most commonly occur on the legs, while in men they are most common on the back (WHO). Sometimes they develop from a mole with concerning changes including an increase in size, irregular edges, change in color, itchiness, or skin breakdown (NCI).

Confirmation of melanoma is done with a skin biopsy. This is usually followed up with a wider excision of the scar or tumor. Depending on the stage, a sentinel lymph node biopsy is done, as well, although controversy exists around trial evidence for this procedure ("The Sentinel Node Biopsy Procedure in Melanoma does not offer a survival advantage". Malignant Melanoma. 2008-01-08). Treatment of advanced malignant melanoma is performed from a multidisciplinary approach, including one or more of the following:

### Surgery

Excisional biopsies may remove the tumor, but further surgery is often necessary to reduce the risk of recurrence. Complete surgical excision with adequate surgical margins and assessment for the presence of detectable metastatic disease along with short- and long-term followup is standard. Often this is done by a wide local excision (WLE) with 1-2 cm (0.4-0.8 in) margins. Melanoma-in-situ and lentigo malignas are treated with narrower surgical margins, usually 0.2-0.5 cm (0.1-0.2 in). Many surgeons consider 0.5 cm (0.2 in) the standard of care for standard excision of melanoma-in-situ,[69] but 0.2 cm (0.1 in) margin might be acceptable for margin controlled surgery (Mohs surgery, or the double-bladed technique with margin control). The wide excision aims to reduce the rate of tumor recurrence at the site of the original lesion. This is a common pattern of treatment failure in melanoma. Considerable research has aimed to elucidate appropriate margins for excision with a general trend toward less aggressive treatment during the last decades (Balch et al. Ann Surg. 218 (3): 262-67; discussion 267-9).

Melanomas that spread usually do so to the lymph nodes in the area of the tumor before spreading elsewhere. Attempts to improve survival by removing lymph nodes surgically (lymphadenectomy) were associated with many complications, but no overall survival benefit. Recently, the technique of sentinel lymph node biopsy has been developed to reduce the complications of lymph node surgery while allowing assessment of the involvement of nodes with tumor ("The Screening and Surveillance of Ultrasound in Melanoma trial (SUNMEL)").

If a lymph node is positive, depending on the extent of lymph node spread, a radical lymph node dissection will often be performed. If the disease is completely resected, the patient will be considered for adjuvant therapy.

### Add On Treatment

High-risk melanomas may require adjuvant treatment, although attitudes to this vary in different countries. In the United States, most patients in otherwise good health will begin up to a year of high-dose interferon treatment, which has severe side effects, but may improve the patient's prognosis slightly (Kirkwood et al. J Clin Oncol. 14 (1): 7-17). However, the British Association of Dermatologists guidelines on melanoma state that interferon is not recommended as a standard adjuvant treatment for melanoma (Neil H. Cox; John S.C., eds. British Association of Dermatologists' management guidelines. Wiley-Blackwell. ISBN 978-1-4443-3552-1).

Metastatic melanomas can be detected by X-rays, CT scans, MRIs, PET and PET/CTs, ultrasound, LDH testing and photoacoustic detection (Weight et al. Opt. Lett. 31 (20): 2998-3000).

### Chemotherapy and Immunotherapy

Various chemotherapy agents, including temozolomide, dacarbazine (also termed DTIC), immunotherapy (with interleukin-2 (IL-2) or interferon (IFN)), as well as local perfusion, are used by different centers.

IL-2 (Proleukin) was the first new therapy approved (1990 Europe, 1992 USA) for the treatment of metastatic melanoma in 20 years. Studies have demonstrated that IL-2 offers the possibility of a complete and long-lasting remission in this disease, although only in a small percentage of patients (Buzaid A. Oncology (Williston Park). 18: 1443-50; discussion 1457-59). Intralesional IL-2 for in-transit metastases has a high complete response rate ranging from 40 to 100% (Maverakis et al. Acta Derm Venereol. 95 (5): 516-524).

Therapies for metastatic melanoma include biologic immunotherapy agents ipilimumab, pembrolizumab, and nivolumab (Syn et al. The Lancet Oncology. 18 (12): e731-41); BRAF inhibitors, such as vemurafenib and dabrafenib; and a MEK inhibitor trametinib (Maverakis et al.).

Ongoing research is looking at treatment by adoptive cell transfer (Rosenberg et al. Science. 348 (6230): 62-68). For this purpose, application of prestimulated or modified T cells (Zang et al. Med. Sci. Monit. 20: 953-59) or dendritic cells is possible (Van Lint et al. Cancer immunology, immunotherapy : CII. 63: 959-67).

### Lentigo maligna

Some melanocytic nevi, and melanoma-in-situ (lentigo maligna) have resolved with an experimental treatment, imiquimod (Aldara) topical cream, an immune enhancing agent. Some dermasurgeons are combining the 2 methods: surgically excising the cancer and then treating the area with Aldara cream postoperatively for three months. While some studies have suggested the adjuvant use of topical tazarotene, the current evidence is insufficient to recommend it and suggests that it increases topical inflammation, leading to lower patient compliance (Tzellos et al. Cochrane Database of Systematic Reviews. ISSN 1465-1858).

### Radiation therapy

Radiation therapy is often used after surgical resection for patients with locally or regionally advanced melanoma or for patients with unresectable distant metastases. Kilovoltage x-ray beams are often used for these treatments and have the property of the maximum radiation dose occurring close to the skin surface (Hill et al. Physics in medicine and biology. 59: R183-231). Radioimmunotherapy of metastatic melanoma is currently under investigation. Radiotherapy has a role in the palliation of metastatic melanoma (Boyer (1999). Primary Care Oncology. Ford).

### Ovarian Cancer

Ovarian cancer is a cancer that forms in or on an ovary ("Ovarian Cancer Prevention (PDQ)". NCI. December 6, 2013; Seiden, Michael (2015). "Gynecologic Malignancies, Chapter 117"). It results in abnormal cells that have the ability to invade or spread to other parts of the body ("Defining Cancer". National Cancer Institute). When this process begins, there may be no or only vague symptoms (NCI). Symptoms become more noticeable as the cancer progresses (NCI; Ebell et al. American journal of preventive medicine. 50: 384-94). These symptoms may include bloating, pelvic pain, abdominal swelling, and loss of appetite, among others (NCI). Common areas to which the cancer may spread include the lining of the abdomen, lymph nodes, lungs, and liver (Ruddon. Cancer biology (4th ed.). Oxford: Oxford University Press, p. 223).

Treatment usually involves surgery and chemotherapy, and sometimes radiotherapy, regardless of the subtype of ovarian cancer ("Types of ovarian cancer". cancerresearchuk.org; Marchetti et al. Expert Rev Anticancer ther 10: 47-60). Surgical treatment may be sufficient for well-differentiated malignant tumors and confined to the ovary. Addition of chemotherapy may be required for more aggressive tumors confined to the ovary. For patients with advanced disease, a combination of surgical reduction with a combination chemotherapy regimen is standard. Borderline tumors, even following spread outside of the ovary, are managed well with surgery (cancerresearchuk.org).

### Surgery

Surgery has been the standard of care for decades and may be necessary in obtaining a specimen for diagnosis. The surgery depends upon the extent of nearby invasion of other tissues by the cancer when it is diagnosed. This extent of the cancer is described by assigning it a stage, the presumed type, and the grade of cancer. The gynecological surgeon may remove one (unilateral oophorectomy) or both ovaries (bilateral oophorectomy). The Fallopian tubes (salpingectomy), uterus (hysterectomy), and the omentum (omentectomy) may also be removed. Typically, all of these organs are removed ("Treatment of ovarian cancer". Canadian Cancer Society).

### Clear Cell Ovarian Cancer

Clear cell cancer of the ovary is an uncommon type of epithelial ovarian cancer. It accounts for three to five per cent of patients with ovarian cancer in the western world and 20 per cent in the Far East.

It is likely that the cause of clear cell cancer is different from the common variety. The CA 125 tumour marker is not always elevated in clear cell cancers, so it may be less reliable.

The prognosis for women with clear cell cancer is greatly influenced by the stage of the disease. Clear cell cancer may also arise more commonly in women with a history of endometriosis.

The first treatment for clear cell cancer is the same as for the common form of epithelial ovarian cancer - a total abdominal hysterectomy and bilateral salpingo-oophorectomy.and omentectomy. In some circumstances removal of the lymph glands in the pelvis and back of the abdomen may also be included.

### Chemotherapy

Usually post-operative treatment involves chemotherapy (drug therapy). Chemotherapy has been a general standard of care for ovarian cancer for decades, although with variable protocols. Chemotherapy is used after surgery to treat any residual disease, if appropriate. In some cases, there may be reason to perform chemotherapy first, followed by surgery. This is called "neoadjuvant chemotherapy", and is common when a tumor cannot be completely removed or optimally debulked via surgery.

The standard chemotherapy for ovarian cancer is to use two drugs called carboplatin and paclitaxel. There is research being carried out into whether other drugs may be as or more effective in treating clear cell cancers. New research suggests that in some cases of clear cell cancer which is confined to the ovary, chemotherapy may be avoided.

If a unilateral salpingo-oophorectomy or other surgery is performed, additional chemotherapy, called "adjuvant chemotherapy", can be given (Jayson et al. Lancet. 384: 1376-88; "Ovarian cancer". DynaMed. June 18, 2015). Adjuvant chemotherapy is used in stage 1 cancer typically if the tumor is of a high histologic grade (grade 3) or the highest substage (stage 1c), provided the cancer has been optimally staged during surgery (DynaMed; cancerresearchuk.org). Bevacizumab may be used as an adjuvant chemotherapy if the tumor is not completely removed during surgery or if the cancer is stage IV; it can extend progression-free survival but has not been shown to extend overall survival (DynaMed). Chemotherapy is curative in approximately 20% of advanced ovarian cancers (Hoffman et al. Williams Gynecology (2nd ed.). McGraw Hill Medical. pp. 853-878); it is more often curative with malignant germ cell tumors than epithelial tumors (Williams Gynecology 2012).

Chemotherapy in ovarian cancer typically consists of platins, a group of platinum-based drugs, combined with non-platins. Common therapies can include paclitaxel, cisplatin, topotecan, doxorubicin, epirubicin, and gemcitabine. Carboplatin is typically given in combination with either paclitaxel or docetaxel; the typical combination is carboplatin with paclitaxel (Jayson et al.; DynaMed). Carboplatin is superior to cisplatin in that it is less toxic and has fewer side effects, generally allowing for an improved quality of life in comparison, though both are similarly effective (DynaMed). Three-drug regimens have not been found to be more effective (Jayson et al.), and platins alone or nonplatins alone are less effective than platins and nonplatins in combination (DynaMed). Chemotherapy can be given intravenously or in the peritoneal cavity (Seiden et al. Harrison's Principles of Internal Medicine (18th ed.). McGraw-Hill). Though intraperitoneal chemotherapy is associated with longer progression-free survival and overall survival, it also causes more adverse side effects than intravenous chemotherapy (DynaMed). It is mainly used when the cancer has been optimally debulked. Intraperitoneal chemotherapy can be highly effective because ovarian cancer mainly spreads inside the peritoneal cavity, and higher doses of the drugs can reach the tumors this way (Hoffman et al.).

### Platinum-sensitive or platinum-resistant Cancers

If ovarian cancer recurs, it is considered partially platinum-sensitive or platinum-resistant, based on the time since the last recurrence treated with platins: partially platinum-sensitive cancers recurred 6-12 months after last treatment, and platinum-resistant cancers have an interval of less than 6 months. Second-line chemotherapy can be given after the cancer becomes symptomatic, because no difference in survival is seen between treating asymptomatic (elevated CA-125) and symptomatic recurrences.

For platinum-sensitive tumors, platins are the drugs of choice for second-line chemotherapy, in combination with other cytotoxic agents. Regimens include carboplatin combined with pegylated liposomal doxorubicin, gemcitabine, or paclitaxel (Seiden). Carboplatin-doublet therapy can be combined with paclitaxel for increased efficacy in some cases. Another potential adjuvant therapy for platinum-sensitive recurrences is olaparib, which may improve progression-free survival but has not been shown to improve overall survival (DynaMed). (Olaparib, a PARP inhibitor, was approved by the US FDA for use in BRCA-associated ovarian cancer that had previously been treated with chemotherapy (Yao, Stephanie (19 December 2014). "FDA approves Lynparza to treat advanced ovarian cancer: First LDT companion diagnostic test also approved to identify appropriate patients". U.S. Food and Drug Administration)). For recurrent germ cell tumors, an additional 4 cycles of BEP chemotherapy is the first-line treatment for those that have been treated with surgery or platins.

If the tumor is determined to be platinum-resistant, vincristine, dactinomycin, and cyclophosphamide (VAC) or some combination of paclitaxel, gemcitabine, and oxaliplatin may be used as a second-line therapy (Williams Gynecology 2012).

### Radiation therapy

Dysgerminomas are most effectively treated with radiation (DeCherney et al. Current Diagnosis & Treatment Obstetrics & Gynecology (11th ed.)), though this can cause infertility and is being phased out in favor of chemotherapy (Seiden). Radiation therapy does not improve survival in people with well-differentiated tumors (Seiden).

### Immunotherapy

Immunotherapy is a topic of current research in ovarian cancer. In some cases, the antibody drug bevacizumab, though still a topic of active research, is used to treat advanced cancer along with chemotherapy (cancerresearchuk.org).

### Treatment Selection

The methods described herein can be used for selecting, and then optionally administering, an optimal treatment (e.g., an EGLN1 inhibitor and/or a VHL inhibitor) for a subject. Thus, the methods described herein include methods for the treatment of cancer, particularly melanoma, ovarian cancer (e.g., clear cell ovarian cancer) and/or other form of cancer associated with EGLN1-dependency. Generally, the methods include administering a therapeutically effective amount of a treatment as described herein, to a subject who is in need of, or who has been determined to be in need of, such treatment.

As used in this context, to "treat" means to ameliorate at least one symptom of the cancer. For example, a treatment can result in a reduction in tumor size, tumor growth, cancer cell number, cancer cell growth, or metastasis or risk of metastasis.

For example, the methods can include selecting and/or administering a treatment that includes a therapeutically effective amount of an EGLN1 inhibitor and/or a VHL inhibitor. An EGLN1 inhibitor and/or a VΣ-Π, inhibitor of the instant disclosure may be administered alone to a subject, or, optionally, an EGLN1 inhibitor and/or a VHL inhibitor may be administered in combination with an additional therapeutic agent including platinum agents such as cisplatin or carboplatin.

Exemplary EGLN1 inhibitors include FG-4592 (Roxadustat), FG-2216, Molidustat (BAY 85-3934), IOX2, AKB-6548 (Vadadustat), Daprodustat (GSK-1278863) and FG-4497. Structures of certain of these small molecule EGLN1 inhibitors follow.

### FG-4592 (Roxadustat):

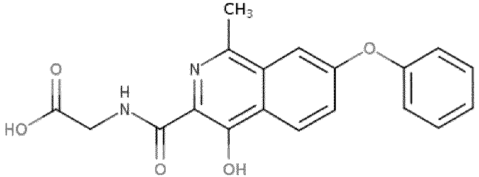

### FG-2216:

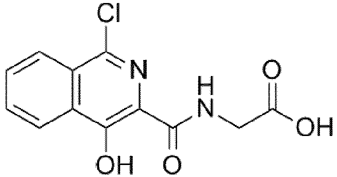

### Molidustat (BAY 85-3934):

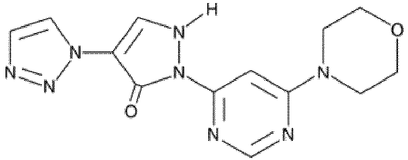

### IOX2:

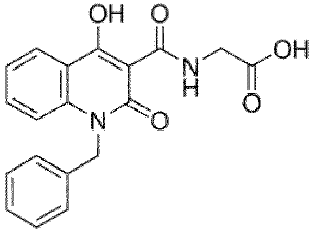

### AKB-6548 (Vadadustat):

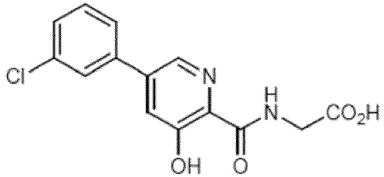

### Daprodustat (GSK-1278863):

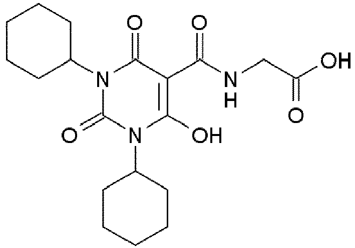

An exemplary VHL inhibitor is VH298, which has the following structure:

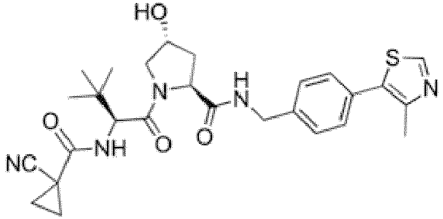

Oligonucleotide inhibitors of EGLN1 and/or VHL are also explicitly contemplated for use herein, including, e.g., antisense oligonucleotides, dsNA agents (including, e.g., siRNAs, hairpin oligonucleotides, etc.), and sgRNA (e.g., implementing CRISPR/Cas9 as a delivery approach for sequence-specific inhibition of EGLN1 and/or VHL). In certain embodiments, sequence-specific oligonucleotide inhibitors of EGLN1 and/or VHL are delivered as naked oligonucleotides, as modified oligonucleotides (e.g., as GalNAc conjugates), within lipid nanoparticles (LNPs), or as components of other art-recognized delivery modalities for oligonucleotide therapeutics. Exemplary description of oligonucleotide inhibitors of EGLN1 can be found, e.g., in U.S. Patent No. 9,193,973, while VHL appears not to have previously been considered as a target for oligonucleotide-mediated knockdown. In certain embodiments, exemplary EGLN1 and/or VHL inhibitor compositions may be given in combination with chemotherapy and/or radiation therapy. An exemplary EGLN1 inhibitor may be an oligonucleotide inhibitor of EGLN1, such as the siRNA formed by the duplex of 5'-CAAGGUACGCAAUAACUGU-3' and 5'-ACAGLTUALTUGCGUACCLTUG-3'. Also specifically contemplated is an analog, derivative, fragment, homolog or variant of the oligonucleotide. Compositions of the present disclosure may be formulated as a liposome preparation. The liposome preparation can comprise liposomes which penetrate the cells of interest, and fuse with the cell membrane, resulting in delivery of the contents of the liposome into the cell. For example, liposomes such as those described in U.S. Patent No. 5,077,211 of Yarosh, U.S. Patent No. 4,621,023 of Redziniak et al. or U.S. Patent No. 4,508,703 of Redziniak et al. can be used.

Exemplary antisense or other oligonucleotide-directed inhibition of EGLN1 and/or VΣ-Π, expression can be assayed in a variety of ways known in the art. For example, EGLN1 and/or VHL mRNA levels can be quantitated by, e.g., Northern blot analysis, competitive polymerase chain reaction (PCR), or real-time PCR (RT-PCR). Real-time quantitative PCR is presently preferred. RNA analysis can be performed on total cellular RNA or poly(A)+ mRNA. Methods of RNA isolation are taught in, for example, Ausubel, F. M. et al.,Current Protocols in Molecular Biology, Volume 1, pp. 4.1.1-4.2.9 and 4.5.1-4.5.3, John Wiley & Sons, Inc., 1993. Northern blot analysis is routine in the art and is taught in, for example, Ausubel, F. M. et al.,Current Protocols in Molecular Biology, Volume 1, pp. 4.2.1-4.2.9, John Wiley & Sons, Inc., 1996. Real-time quantitative (PCR) can be conveniently accomplished using the commercially available ABI PRISM^{™} 7700 Sequence Detection System, available from PE-Applied Biosystems, Foster City, Calif. and used according to manufacturer's instructions.

Protein levels of EGLN1 and/or VΣ-Π, can be quantitated in a variety of ways well known in the art, such as immunoprecipitation, Western blot analysis (immunoblotting), ELISA or fluorescence-activated cell sorting (FACS). Antibodies directed to EGLN1 and/or VHL can be identified and obtained from a variety of sources, such as the MSRS catalog of antibodies (Aerie Corporation, Birmingham, Mich.), or can be prepared via conventional antibody generation methods. Methods for preparation of polyclonal antisera are taught in, for example, Ausubel, F. M. et al.,Current Protocols in Molecular Biology, Volume 2, pp. 11.12.1-11.12.9, John Wiley & Sons, Inc., 1997. Preparation of monoclonal antibodies is taught in, for example, Ausubel, F. M. et al.,Current Protocols in Molecular Biology, Volume 2, pp. 11.4.1-11.11.5, John Wiley & Sons, Inc., 1997.

Immunoprecipitation methods are standard in the art and can be found at, for example, Ausubel, F. M. et al.,Current Protocols in Molecular Biology, Volume 2, pp. 10.16.1-10.16.11, John Wiley & Sons, Inc., 1998. Western blot (immunoblot) analysis is standard in the art and can be found at, for example, Ausubel, F. M. et al.,Current Protocols in Molecular Biology, Volume 2, pp. 10.8.1-10.8.21, John Wiley & Sons, Inc., 1997. Enzyme-linked immunosorbent assays (ELISA) are standard in the art and can be found at, for example, Ausubel, F. M. et al.,Current Protocols in Molecular Biology, Volume 2, pp. 11.2.1-11.2.22, John Wiley & Sons, Inc., 1991

Monoclonal and polyclonal antibodies against EGLN1 and/or VHL have also been described in the art (see, e.g., GenBank Accession No. NP_071334.1 for an anti-EGLN1 antibody and EP 2957571 for an anti-VHL monoclonal antibody). Compounds of the present invention that suppress EGLN1 and/or VHL helicase gene expression or the function (activity) of a protein encoded by that gene may be natural or synthetic compounds. Typically, the compounds can be produced, obtained or isolated using methods known to those skilled in the art. Such compounds include, for example, compounds comprising a single molecule, such as organic compounds, inorganic compounds, nucleic acids, proteins, peptides, and sugars; and libraries of compounds, expression products of gene libraries, cell extracts, cell culture supernatants, the products of fermenting microorganisms, marine organism extracts, plant extracts, and compounds purified or isolated from such extracts.

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a therapeutic compound (i.e., an effective dosage) depends on the therapeutic compounds selected. The compositions can be administered from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the therapeutic compounds described herein can include a single treatment or a series of treatments.

Dosage, toxicity and therapeutic efficacy of the therapeutic compounds can be determined by standard pharmacological procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of an EGLN1 and/or VHL inhibitor which achieves a half-maximal inhibition of symptoms) as determined in cell culture. Such information can be used to more accurately determine useful doses in humans. Levels in plasma may be measured, for example, by high performance liquid chromatography.

### Combination Treatments

The compositions and methods of the present disclosure may be used in the context of a number of therapeutic or prophylactic applications. In order to increase the effectiveness of a treatment with the compositions of the present disclosure, e.g., an EGLN1 and/or VHL inhibitor selected and/or administered as a single agent, or to augment the efficacy of another therapy (second therapy), it may be desirable to combine these compositions and methods with one another, or with other agents and methods effective in the treatment, amelioration, or prevention of diseases and pathologic conditions, for example, EGLN1-dependent cancers, e.g., an EGLN1-dependent melanoma, an EGLN1-dependent ovarian cancer (optionally an EGLN1-dependent clear cell ovarian cancer), etc.

Administration of a composition of the present disclosure to a subject will follow general protocols for the administration described herein, and the general protocols for the administration of a particular secondary therapy will also be followed, taking into account the toxicity, if any, of the treatment. It is expected that the treatment cycles would be repeated as necessary. It also is contemplated that various standard therapies may be applied in combination with the described therapies.

### Pharmaceutical Compositions

Agents of the present disclosure can be incorporated into a variety of formulations for therapeutic use (e.g., by administration) or in the manufacture of a medicament (e.g., for treating or preventing an EGLN1-dependent cancer) by combining the agents with appropriate pharmaceutically acceptable carriers or diluents, and may be formulated into preparations in solid, semi-solid, liquid or gaseous forms. Examples of such formulations include, without limitation, tablets, capsules, powders, granules, ointments, solutions, suppositories, injections, inhalants, gels, microspheres, and aerosols.

Pharmaceutical compositions can include, depending on the formulation desired, pharmaceutically-acceptable, non-toxic carriers of diluents, which are vehicles commonly used to formulate pharmaceutical compositions for animal or human administration. The diluent is selected so as not to affect the biological activity of the combination. Examples of such diluents include, without limitation, distilled water, buffered water, physiological saline, PBS, Ringer's solution, dextrose solution, and Hank's solution. A pharmaceutical composition or formulation of the present disclosure can further include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

Further examples of formulations that are suitable for various types of administration can be found in Remington's Pharmaceutical Sciences, Mace Publishing Company, Philadelphia, PA, 17th ed. (1985). For a brief review of methods for drug delivery, see, Langer, Science 249: 1527-1533 (1990).

For oral administration, the active ingredient can be administered in solid dosage forms, such as capsules, tablets, and powders, or in liquid dosage forms, such as elixirs, syrups, and suspensions. The active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate. Examples of additional inactive ingredients that may be added to provide desirable color, taste, stability, buffering capacity, dispersion or other known desirable features are red iron oxide, silica gel, sodium lauryl sulfate, titanium dioxide, and edible white ink.

Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

Formulations suitable for parenteral administration include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts of amines, carboxylic acids, and other types of compounds, are well known in the art. For example, S. M. Berge, et al. describe pharmaceutically acceptable salts in detail in J Pharmaceutical Sciences 66 (1977): 1-19. The salts can be prepared in situ during the final isolation and purification of the compounds of the application, or separately by reacting a free base or free acid function with a suitable reagent, as described generally below. For example, a free base function can be reacted with a suitable acid. Furthermore, where the compounds to be administered of the application carry an acidic moiety, suitable pharmaceutically acceptable salts thereof may, include metal salts such as alkali metal salts, e.g. sodium or potassium salts; and alkaline earth metal salts, e.g. calcium or magnesium salts. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Additionally, as used herein, the term "pharmaceutically acceptable ester" refers to esters that hydrolyze in vivo and include those that break down readily in the human body to leave the parent compound (e.g., an FDA-approved compound where administered to a human subject) or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moeity advantageously has not more than 6 carbon atoms. Examples of particular esters include formates, acetates, propionates, butyrates, acrylates and ethyl succinates.

Furthermore, the term "pharmaceutically acceptable prodrugs" as used herein refers to those prodrugs of the certain compounds of the present application which are, within the scope of sound medical judgment, suitable for use in contact with the issues of humans and lower animals with undue toxicity, irritation, allergic response, and the like, commensurate with a reasonable benefit/risk ratio, and effective for their intended use, as well as the zwitterionic forms, where possible, of the compounds of the application. The term "prodrug" refers to compounds that are rapidly transformed in vivo to yield the parent compound of an agent of the instant disclosure, for example by hydrolysis in blood. A thorough discussion is provided in T. Higuchi and V. Stella, Prodrugs as Novel Delivery Systems, Vol. 14 of the A.C.S. Symposium Series, and in Edward B. Roche, ed., Bioreversible Carriers in Drug Design, American Pharmaceutical Association and Pergamon Press, (1987).

The components used to formulate the pharmaceutical compositions are preferably of high purity and are substantially free of potentially harmful contaminants (e.g., at least National Food (NF) grade, generally at least analytical grade, and more typically at least pharmaceutical grade). Moreover, compositions intended for in vivo use are usually sterile. To the extent that a given compound must be synthesized prior to use, the resulting product is typically substantially free of any potentially toxic agents, particularly any endotoxins, which may be present during the synthesis or purification process. Compositions for parental administration are also sterile, substantially isotonic and made under GMP conditions.

Formulations may be optimized for retention and stabilization in a subject and/or tissue of a subject, e.g., to prevent rapid clearance of a formulation by the subject. Stabilization techniques include cross-linking, multimerizing, or linking to groups such as polyethylene glycol, polyacrylamide, neutral protein carriers, etc. in order to achieve an increase in molecular weight.

Other strategies for increasing retention include the entrapment of the agent, such as an EGLN1 and/or VHL inhibitor, in a biodegradable or bioerodible implant. The rate of release of the therapeutically active agent is controlled by the rate of transport through the polymeric matrix, and the biodegradation of the implant. The transport of drug through the polymer barrier will also be affected by compound solubility, polymer hydrophilicity, extent of polymer cross-linking, expansion of the polymer upon water absorption so as to make the polymer barrier more permeable to the drug, geometry of the implant, and the like. The implants are of dimensions commensurate with the size and shape of the region selected as the site of implantation. Implants may be particles, sheets, patches, plaques, fibers, microcapsules and the like and may be of any size or shape compatible with the selected site of insertion.

The implants may be monolithic, i.e. having the active agent homogenously distributed through the polymeric matrix, or encapsulated, where a reservoir of active agent is encapsulated by the polymeric matrix. The selection of the polymeric composition to be employed will vary with the site of administration, the desired period of treatment, patient tolerance, the nature of the disease to be treated and the like. Characteristics of the polymers will include biodegradability at the site of implantation, compatibility with the agent of interest, ease of encapsulation, a half-life in the physiological environment.

Biodegradable polymeric compositions which may be employed may be organic esters or ethers, which when degraded result in physiologically acceptable degradation products, including the monomers. Anhydrides, amides, orthoesters or the like, by themselves or in combination with other monomers, may find use. The polymers will be condensation polymers. The polymers may be cross-linked or non-cross-linked. Of particular interest are polymers of hydroxyaliphatic carboxylic acids, either homo- or copolymers, and polysaccharides. Included among the polyesters of interest are polymers of D-lactic acid, L-lactic acid, racemic lactic acid, glycolic acid, polycaprolactone, and combinations thereof. By employing the L-lactate or D- lactate, a slowly biodegrading polymer is achieved, while degradation is substantially enhanced with the racemate. Copolymers of glycolic and lactic acid are of particular interest, where the rate of biodegradation is controlled by the ratio of glycolic to lactic acid. The most rapidly degraded copolymer has roughly equal amounts of glycolic and lactic acid, where either homopolymer is more resistant to degradation. The ratio of glycolic acid to lactic acid will also affect the brittleness of in the implant, where a more flexible implant is desirable for larger geometries. Among the polysaccharides of interest are calcium alginate, and functionalized celluloses, particularly carboxymethylcellulose esters characterized by being water insoluble, a molecular weight of about 5 kD to 500 kD, etc. Biodegradable hydrogels may also be employed in the implants of the individual instant disclosure. Hydrogels are typically a copolymer material, characterized by the ability to imbibe a liquid. Exemplary biodegradable hydrogels which may be employed are described in Heller in: Hydrogels in Medicine and Pharmacy, N. A. Peppes ed., Vol. ΠI, CRC Press, Boca Raton, Fla., 1987, pp 137-149.

### Pharmaceutical Dosages

Pharmaceutical compositions of the present disclosure containing an agent described herein may be used (e.g., administered to an individual, such as a human individual, in need of treatment with an EGLN1 and/or VΣ-Π, inhibitor) in accord with known methods, such as oral administration, intravenous administration as a bolus or by continuous infusion over a period of time, by intramuscular, intraperitoneal, intracerobrospinal, intracranial, intraspinal, subcutaneous, intraarticular, intrasynovial, intrathecal, topical, or inhalation routes.

Dosages and desired drug concentration of pharmaceutical compositions of the present disclosure may vary depending on the particular use envisioned. The determination of the appropriate dosage or route of administration is well within the skill of an ordinary artisan. Animal experiments provide reliable guidance for the determination of effective doses for human therapy. Interspecies scaling of effective doses can be performed following the principles described in Mordenti, J. and Chappell, W. "The Use of Interspecies Scaling in Toxicokinetics," In Toxicokinetics and New Drug Development, Yacobi et al., Eds, Pergamon Press, New York 1989, pp.42-46.

For *in vivo* administration of any of the agents of the present disclosure, normal dosage amounts may vary from about 10 ng/kg up to about 100 mg/kg of an individual's and/or subject's body weight or more per day, depending upon the route of administration. In some embodiments, the dose amount is about 1 mg/kg/day to 10 mg/kg/day. For repeated administrations over several days or longer, depending on the severity of the disease, disorder, or condition to be treated, the treatment is sustained until a desired suppression of symptoms is achieved.

An effective amount of an agent of the instant disclosure may vary, e.g., from about 0.001 mg/kg to about 1000 mg/kg or more in one or more dose administrations for one or several days (depending on the mode of administration). In certain embodiments, the effective amount per dose varies from about 0.001 mg/kg to about 1000 mg/kg, from about 0.01 mg/kg to about 750 mg/kg, from about 0.1 mg/kg to about 500 mg/kg, from about 1.0 mg/kg to about 250 mg/kg, and from about 10.0 mg/kg to about 150 mg/kg.

An exemplary dosing regimen may include administering an initial dose of an agent of the disclosure of about 200 µg/kg, followed by a weekly maintenance dose of about 100 µg/kg every other week. Other dosage regimens may be useful, depending on the pattern of pharmacokinetic decay that the physician wishes to achieve. For example, dosing an individual from one to twenty-one times a week is contemplated herein. In certain embodiments, dosing ranging from about 3 µg/kg to about 2 mg/kg (such as about 3 µg/kg, about 10 µg/kg, about 30 µg/kg, about 100 µg/kg, about 300 µg/kg, about 1 mg/kg, or about 2 mg/kg) may be used. In certain embodiments, dosing frequency is three times per day, twice per day, once per day, once every other day, once weekly, once every two weeks, once every four weeks, once every five weeks, once every six weeks, once every seven weeks, once every eight weeks, once every nine weeks, once every ten weeks, or once monthly, once every two months, once every three months, or longer. Progress of the therapy is easily monitored by conventional techniques and assays. The dosing regimen, including the agent(s) administered, can vary over time independently of the dose used.

Pharmaceutical compositions described herein can be prepared by any method known in the art of pharmacology. In general, such preparatory methods include the steps of bringing the agent or compound described herein (i.e., the "active ingredient") into association with a carrier or excipient, and/or one or more other accessory ingredients, and then, if necessary and/or desirable, shaping, and/or packaging the product into a desired single- or multi-dose unit.

Pharmaceutical compositions can be prepared, packaged, and/or sold in bulk, as a single unit dose, and/or as a plurality of single unit doses. A "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition described herein will vary, depending upon the identity, size, and/or condition of the subject treated and further depending upon the route by which the composition is to be administered. The composition may comprise between 0.1% and 100% (w/w) active ingredient.

Pharmaceutically acceptable excipients used in the manufacture of provided pharmaceutical compositions include inert diluents, dispersing and/or granulating agents, surface active agents and/or emulsifiers, disintegrating agents, binding agents, preservatives, buffering agents, lubricating agents, and/or oils. Excipients such as cocoa butter and suppository waxes, coloring agents, coating agents, sweetening, flavoring, and perfuming agents may also be present in the composition.

Exemplary diluents include calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and mixtures thereof.

Exemplary granulating and/or dispersing agents include potato starch, corn starch, tapioca starch, sodium starch glycolate, clays, alginic acid, guar gum, citrus pulp, agar, bentonite, cellulose, and wood products, natural sponge, cation-exchange resins, calcium carbonate, silicates, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and mixtures thereof.

Exemplary surface active agents and/or emulsifiers include natural emulsifiers (e.g., acacia, agar, alginic acid, sodium alginate, tragacanth, chondrux, cholesterol, xanthan, pectin, gelatin, egg yolk, casein, wool fat, cholesterol, wax, and lecithin), colloidal clays (e.g., bentonite (aluminum silicate) and Veegum (magnesium aluminum silicate)), long chain amino acid derivatives, high molecular weight alcohols (e.g., stearyl alcohol, cetyl alcohol, oleyl alcohol, triacetin monostearate, ethylene glycol distearate, glyceryl monostearate, and propylene glycol monostearate, polyvinyl alcohol), carbomers (e.g., carboxy polymethylene, polyacrylic acid, acrylic acid polymer, and carboxyvinyl polymer), carrageenan, cellulosic derivatives (e.g., carboxymethylcellulose sodium, powdered cellulose, hydroxymethyl cellulose, hydroxypropyl cellulose, hydroxypropyl methylcellulose, methylcellulose), sorbitan fatty acid esters (e.g., polyoxyethylene sorbitan monolaurate (Tween^{®} 20), polyoxyethylene sorbitan (Tween^{®} 60), polyoxyethylene sorbitan monooleate (Tween^{®} 80), sorbitan monopalmitate (Span^{®} 40), sorbitan monostearate (Span^{®} 60), sorbitan tristearate (Span^{®} 65), glyceryl monooleate, sorbitan monooleate (Span^{®} 80), polyoxyethylene esters (e.g., polyoxyethylene monostearate (Myij^{®} 45), polyoxyethylene hydrogenated castor oil, polyethoxylated castor oil, polyoxymethylene stearate, and Solutol^{®}), sucrose fatty acid esters, polyethylene glycol fatty acid esters (e.g., Cremophor^{®}), polyoxyethylene ethers, (e.g., polyoxyethylene lauryl ether (Brij^{®} 30)), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic^{®} F-68, Poloxamer P-188, cetrimonium bromide, cetylpyridinium chloride, benzalkonium chloride, docusate sodium, and/or mixtures thereof.

Exemplary binding agents include starch (e.g., cornstarch and starch paste), gelatin, sugars (e.g., sucrose, glucose, dextrose, dextrin, molasses, lactose, lactitol, mannitol, etc.), natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinyl-pyrrolidone), magnesium aluminum silicate (Veegum^{®}), and larch arabogalactan), alginates, polyethylene oxide, polyethylene glycol, inorganic calcium salts, silicic acid, polymethacrylates, waxes, water, alcohol, and/or mixtures thereof.

Exemplary preservatives include antioxidants, chelating agents, antimicrobial preservatives, antifungal preservatives, antiprotozoan preservatives, alcohol preservatives, acidic preservatives, and other preservatives. In certain embodiments, the preservative is an antioxidant. In other embodiments, the preservative is a chelating agent.

Exemplary antioxidants include alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite.

Exemplary chelating agents include ethylenediaminetetraacetic acid (EDTA) and salts and hydrates thereof (e.g., sodium edetate, disodium edetate, trisodium edetate, calcium disodium edetate, dipotassium edetate, and the like), citric acid and salts and hydrates thereof (e.g., citric acid monohydrate), fumaric acid and salts and hydrates thereof, malic acid and salts and hydrates thereof, phosphoric acid and salts and hydrates thereof, and tartaric acid and salts and hydrates thereof. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal.

Exemplary antifungal preservatives include butyl paraben, methyl paraben, ethyl paraben, propyl paraben, benzoic acid, hydroxybenzoic acid, potassium benzoate, potassium sorbate, sodium benzoate, sodium propionate, and sorbic acid.

Exemplary alcohol preservatives include ethanol, polyethylene glycol, phenol, phenolic compounds, bisphenol, chlorobutanol, hydroxybenzoate, and phenylethyl alcohol.

Exemplary acidic preservatives include vitamin A, vitamin C, vitamin E, beta-carotene, citric acid, acetic acid, dehydroacetic acid, ascorbic acid, sorbic acid, and phytic acid.

Other preservatives include tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant^{®} Plus, Phenonip^{®}, methylparaben, Germall^{®} 115, Germaben^{®} II, Neolone^{®}, Kathon^{®}, and Euxyl^{®}.

Exemplary buffering agents include citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and mixtures thereof.

Exemplary lubricating agents include magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and mixtures thereof.

Exemplary natural oils include almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary synthetic oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and mixtures thereof.

Liquid dosage forms for oral and parenteral administration include pharmaceutically acceptable emulsions, microemulsions, solutions, suspensions, syrups and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (e.g., cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can include adjuvants such as wetting agents, emulsifying and suspending agents, sweetening, flavoring, and perfuming agents. In certain embodiments for parenteral administration, the conjugates described herein are mixed with solubilizing agents such as Cremophor^{®}, alcohols, oils, modified oils, glycols, polysorbates, cyclodextrins, polymers, and mixtures thereof.

Injectable preparations, for example, sterile injectable aqueous or oleaginous suspensions can be formulated according to the known art using suitable dispersing or wetting agents and suspending agents. The sterile injectable preparation can be a sterile injectable solution, suspension, or emulsion in a nontoxic parenterally acceptable diluent or solvent, for example, as a solution in 1,3-butanediol. Among the acceptable vehicles and solvents that can be employed are water, Ringer's solution, U.S.P., and isotonic sodium chloride solution. In addition, sterile, fixed oils are conventionally employed as a solvent or suspending medium. For this purpose any bland fixed oil can be employed including synthetic mono- or di-glycerides. In addition, fatty acids such as oleic acid are used in the preparation of inj ectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution, which, in turn, may depend upon crystal size and crystalline form. Alternatively, delayed absorption of a parenterally administered drug form may be accomplished by dissolving or suspending the drug in an oil vehicle.

Compositions for rectal or vaginal administration are typically suppositories which can be prepared by mixing the conjugates described herein with suitable non-irritating excipients or carriers such as cocoa butter, polyethylene glycol, or a suppository wax which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, the active ingredient is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets, and pills, the dosage form may include a buffering agent.

Solid compositions of a similar type can be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the art of pharmacology. They may optionally comprise opacifying agents and can be of a composition that they release the active ingredient(s) only, or preferentially, in a certain part of the intestinal tract, optionally, in a delayed manner. Examples of encapsulating compositions which can be used include polymeric substances and waxes. Solid compositions of a similar type can be employed as fillers in soft and hard-filled gelatin capsules using such excipients as lactose or milk sugar as well as high molecular weight polethylene glycols and the like.

The active ingredient can be in a micro-encapsulated form with one or more excipients as noted above. The solid dosage forms of tablets, dragees, capsules, pills, and granules can be prepared with coatings and shells such as enteric coatings, release controlling coatings, and other coatings well known in the pharmaceutical formulating art. In such solid dosage forms the active ingredient can be admixed with at least one inert diluent such as sucrose, lactose, or starch. Such dosage forms may comprise, as is normal practice, additional substances other than inert diluents, e.g., tableting lubricants and other tableting aids such as magnesium stearate and microcrystalline cellulose. In the case of capsules, tablets and pills, the dosage forms may comprise buffering agents. They may optionally comprise opacifying agents and can be of a composition that they release the active ingredient(s) only, or preferentially, in a certain part of the intestinal tract, optionally, in a delayed manner. Examples of encapsulating agents which can be used include polymeric substances and waxes.

Dosage forms for topical and/or transdermal administration of an agent (e.g., an EGLN1 and/or VHL inhibitor) described herein may include ointments, pastes, creams, lotions, gels, powders, solutions, sprays, inhalants, and/or patches. Generally, the active ingredient is admixed under sterile conditions with a pharmaceutically acceptable carrier or excipient and/or any needed preservatives and/or buffers as can be required. Additionally, the present disclosure contemplates the use of transdermal patches, which often have the added advantage of providing controlled delivery of an active ingredient to the body. Such dosage forms can be prepared, for example, by dissolving and/or dispensing the active ingredient in the proper medium. Alternatively or additionally, the rate can be controlled by either providing a rate controlling membrane and/or by dispersing the active ingredient in a polymer matrix and/or gel.

Suitable devices for use in delivering intradermal pharmaceutical compositions described herein include short needle devices. Intradermal compositions can be administered by devices which limit the effective penetration length of a needle into the skin. Alternatively or additionally, conventional syringes can be used in the classical mantoux method of intradermal administration. Jet injection devices which deliver liquid formulations to the dermis via a liquid jet injector and/or via a needle which pierces the stratum corneum and produces a jet which reaches the dermis are suitable. Ballistic powder/particle delivery devices which use compressed gas to accelerate the compound in powder form through the outer layers of the skin to the dermis are suitable.

Formulations suitable for topical administration include, but are not limited to, liquid and/or semi-liquid preparations such as liniments, lotions, oil-in-water and/or water-in-oil emulsions such as creams, ointments, and/or pastes, and/or solutions and/or suspensions. Topically administrable formulations may, for example, comprise from about 1% to about 10% (w/w) active ingredient, although the concentration of the active ingredient can be as high as the solubility limit of the active ingredient in the solvent. Formulations for topical administration may further comprise one or more of the additional ingredients described herein.

A pharmaceutical composition described herein can be prepared, packaged, and/or sold in a formulation suitable for pulmonary administration via the buccal cavity. Such a formulation may comprise dry particles which comprise the active ingredient and which have a diameter in the range from about 0.5 to about 7 nanometers, or from about 1 to about 6 nanometers. Such compositions are conveniently in the form of dry powders for administration using a device comprising a dry powder reservoir to which a stream of propellant can be directed to disperse the powder and/or using a self-propelling solvent/powder dispensing container such as a device comprising the active ingredient dissolved and/or suspended in a low-boiling propellant in a sealed container. Such powders comprise particles wherein at least 98% of the particles by weight have a diameter greater than 0.5 nanometers and at least 95% of the particles by number have a diameter less than 7 nanometers. Alternatively, at least 95% of the particles by weight have a diameter greater than 1 nanometer and at least 90% of the particles by number have a diameter less than 6 nanometers. Dry powder compositions may include a solid fine powder diluent such as sugar and are conveniently provided in a unit dose form.

Low boiling propellants generally include liquid propellants having a boiling point of below 65° F. at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid non-ionic and/or solid anionic surfactant and/or a solid diluent (which may have a particle size of the same order as particles comprising the active ingredient).

Pharmaceutical compositions described herein formulated for pulmonary delivery may provide the active ingredient in the form of droplets of a solution and/or suspension. Such formulations can be prepared, packaged, and/or sold as aqueous and/or dilute alcoholic solutions and/or suspensions, optionally sterile, comprising the active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. The droplets provided by this route of administration may have an average diameter in the range from about 0.1 to about 200 nanometers.

Formulations described herein as being useful for pulmonary delivery are useful for intranasal delivery of a pharmaceutical composition described herein. Another formulation suitable for intranasal administration is a coarse powder comprising the active ingredient and having an average particle from about 0.2 to 500 micrometers. Such a formulation is administered by rapid inhalation through the nasal passage from a container of the powder held close to the nares.

Formulations for nasal administration may, for example, comprise from about as little as 0.1% (w/w) to as much as 100% (w/w) of the active ingredient, and may comprise one or more of the additional ingredients described herein. A pharmaceutical composition described herein can be prepared, packaged, and/or sold in a formulation for buccal administration. Such formulations may, for example, be in the form of tablets and/or lozenges made using conventional methods, and may contain, for example, 0.1 to 20% (w/w) active ingredient, the balance comprising an orally dissolvable and/or degradable composition and, optionally, one or more of the additional ingredients described herein. Alternately, formulations for buccal administration may comprise a powder and/or an aerosolized and/or atomized solution and/or suspension comprising the active ingredient. Such powdered, aerosolized, and/or aerosolized formulations, when dispersed, may have an average particle and/or droplet size in the range from about 0.1 to about 200 nanometers, and may further comprise one or more of the additional ingredients described herein.

A pharmaceutical composition described herein can be prepared, packaged, and/or sold in a formulation for ophthalmic administration. Such formulations may, for example, be in the form of eye drops including, for example, a 0.1-1.0% (w/w) solution and/or suspension of the active ingredient in an aqueous or oily liquid carrier or excipient. Such drops may further comprise buffering agents, salts, and/or one or more other of the additional ingredients described herein. Other opthalmically-administrable formulations which are useful include those which comprise the active ingredient in microcrystalline form and/or in a liposomal preparation. Ear drops and/or eye drops are also contemplated as being within the scope of this disclosure.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation.

Drugs provided herein can be formulated in dosage unit form for ease of administration and uniformity of dosage. It will be understood, however, that the total daily usage of the agents described herein will be decided by a physician within the scope of sound medical judgment. The specific therapeutically effective dose level for any particular subject or organism will depend upon a variety of factors including the disease being treated and the severity of the disorder; the activity of the specific active ingredient employed; the specific composition employed; the age, body weight, general health, sex, and diet of the subject; the time of administration, route of administration, and rate of excretion of the specific active ingredient employed; the duration of the treatment; drugs used in combination or coincidental with the specific active ingredient employed; and like factors well known in the medical arts.

The agents and compositions provided herein can be administered by any route, including enteral (e.g., oral), parenteral, intravenous, intramuscular, intra-arterial, intramedullary, intrathecal, subcutaneous, intraventricular, transdermal, interdermal, rectal, intravaginal, intraperitoneal, topical (as by powders, ointments, creams, and/or drops), mucosal, nasal, bucal, sublingual; by intratracheal instillation, bronchial instillation, and/or inhalation; and/or as an oral spray, nasal spray, and/or aerosol. Specifically contemplated routes are oral administration, intravenous administration (e.g., systemic intravenous injection), regional administration via blood and/or lymph supply, and/or direct administration to an affected site. In general, the most appropriate route of administration will depend upon a variety of factors including the nature of the agent (e.g., its stability in the environment of the gastrointestinal tract), and/or the condition of the subject (e.g., whether the subject is able to tolerate oral administration). In certain embodiments, the agent or pharmaceutical composition described herein is suitable for oral delivery or intravenous injection to a subject.

The exact amount of an agent required to achieve an effective amount will vary from subject to subject, depending, for example, on species, age, and general condition of a subject, severity of the side effects or disorder, identity of the particular agent, mode of administration, and the like. An effective amount may be included in a single dose (e.g., single oral dose) or multiple doses (e.g., multiple oral doses). In certain embodiments, when multiple doses are administered to a subject or applied to a tissue or cell, any two doses of the multiple doses include different or substantially the same amounts of an agent (e.g., an EGLN1 and/or VHL inhibitor) described herein.

As noted elsewhere herein, a drug of the instant disclosure may be administered *via* a number of routes of administration, including but not limited to: subcutaneous, intravenous, intrathecal, intramuscular, intranasal, oral, transepidermal, parenteral, by inhalation, or intracerebroventricular.

The term "injection" or "injectable" as used herein refers to a bolus injection (administration of a discrete amount of an agent for raising its concentration in a bodily fluid), slow bolus injection over several minutes, or prolonged infusion, or several consecutive injections/infusions that are given at spaced apart intervals.

In some embodiments of the present disclosure, a formulation as herein defined is administered to the subject by bolus administration.

A drug or other therapy of the instant disclosure is administered to the subject in an amount sufficient to achieve a desired effect at a desired site (e.g., reduction of cancer size, cancer cell abundance, symptoms, etc.) determined by a skilled clinician to be effective. In some embodiments of the disclosure, the agent is administered at least once a year. In other embodiments of the disclosure, the agent is administered at least once a day. In other embodiments of the disclosure, the agent is administered at least once a week. In some embodiments of the disclosure, the agent is administered at least once a month.

Additional exemplary doses for administration of an agent of the disclosure to a subject include, but are not limited to, the following: 1-20 mg/kg/day, 2-15 mg/kg/day, 5-12 mg/kg/day, 10 mg/kg/day, 1-500 mg/kg/day, 2-250 mg/kg/day, 5-150 mg/kg/day, 20-125 mg/kg/day, 50-120 mg/kg/day, 100 mg/kg/day, at least 10 µg/kg/day, at least 100 µg/kg/day, at least 250 µg/kg/day, at least 500 µg/kg/day, at least 1 mg/kg/day, at least 2 mg/kg/day, at least 5 mg/kg/day, at least 10 mg/kg/day, at least 20 mg/kg/day, at least 50 mg/kg/day, at least 75 mg/kg/day, at least 100 mg/kg/day, at least 200 mg/kg/day, at least 500 mg/kg/day, at least 1 g/kg/day, and a therapeutically effective dose that is less than 500 mg/kg/day, less than 200 mg/kg/day, less than 100 mg/kg/day, less than 50 mg/kg/day, less than 20 mg/kg/day, less than 10 mg/kg/day, less than 5 mg/kg/day, less than 2 mg/kg/day, less than 1 mg/kg/day, less than 500 µg/kg/day, and less than 500 µg/kg/day.

In certain embodiments, when multiple doses are administered to a subject or applied to a tissue or cell, the frequency of administering the multiple doses to the subject or applying the multiple doses to the tissue or cell is three doses a day, two doses a day, one dose a day, one dose every other day, one dose every third day, one dose every week, one dose every two weeks, one dose every three weeks, or one dose every four weeks. In certain embodiments, the frequency of administering the multiple doses to the subject or applying the multiple doses to the tissue or cell is one dose per day. In certain embodiments, the frequency of administering the multiple doses to the subject or applying the multiple doses to the tissue or cell is two doses per day. In certain embodiments, the frequency of administering the multiple doses to the subject or applying the multiple doses to the tissue or cell is three doses per day. In certain embodiments, when multiple doses are administered to a subject or applied to a tissue or cell, the duration between the first dose and last dose of the multiple doses is one day, two days, four days, one week, two weeks, three weeks, one month, two months, three months, four months, six months, nine months, one year, two years, three years, four years, five years, seven years, ten years, fifteen years, twenty years, or the lifetime of the subject, tissue, or cell. In certain embodiments, the duration between the first dose and last dose of the multiple doses is three months, six months, or one year. In certain embodiments, the duration between the first dose and last dose of the multiple doses is the lifetime of the subject, tissue, or cell. In certain embodiments, a dose (e.g., a single dose, or any dose of multiple doses) described herein includes independently between 0.1 µg and 1 µg, between 0.001 mg and 0.01 mg, between 0.01 mg and 0.1 mg, between 0.1 mg and 1 mg, between 1 mg and 3 mg, between 3 mg and 10 mg, between 10 mg and 30 mg, between 30 mg and 100 mg, between 100 mg and 300 mg, between 300 mg and 1,000 mg, or between 1 g and 10 g, inclusive, of an agent (e.g., an EGLN1 and/or VHL inhibitor) described herein. In certain embodiments, a dose described herein includes independently between 1 mg and 3 mg, inclusive, of an agent (e.g., an EGLN1 and/or VΣ-Π, inhibitor) described herein. In certain embodiments, a dose described herein includes independently between 3 mg and 10 mg, inclusive, of an agent (e.g., an EGLN1 and/or VHL inhibitor) described herein. In certain embodiments, a dose described herein includes independently between 10 mg and 30 mg, inclusive, of an agent (e.g., an EGLN1 and/or VHL inhibitor) described herein. In certain embodiments, a dose described herein includes independently between 30 mg and 100 mg, inclusive, of an agent (e.g., an EGLN1 and/or VΣ-Π, inhibitor) described herein.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult. In certain embodiments, a dose described herein is a dose to an adult human whose body weight is 70 kg.

It will be also appreciated that an agent (e.g., an EGLN1 and/or VHL inhibitor) or composition, as described herein, can be administered in combination with one or more additional pharmaceutical agents (e.g., therapeutically and/or prophylactically active agents), which are different from the agent or composition and may be useful as, e.g., combination therapies.

The agents or compositions can be administered in combination with additional pharmaceutical agents that improve their activity (e.g., activity (e.g., potency and/or efficacy) in treating a disease (e.g., an EGLN1-dependent cancer) in a subject in need thereof, in preventing a disease in a subject in need thereof, in reducing the risk of developing a disease in a subject in need thereof, in inhibiting the replication of a virus, in killing a virus, etc. in a subject or cell. In certain embodiments, a pharmaceutical composition described herein including an agent (e.g., an EGLN1 and/or VHL inhibitor) described herein and an additional pharmaceutical agent shows a synergistic effect that is absent in a pharmaceutical composition including one of the agent and the additional pharmaceutical agent, but not both.

In some embodiments of the disclosure, a therapeutic agent distinct from a first therapeutic agent of the disclosure is administered prior to, in combination with, at the same time, or after administration of the agent of the disclosure. In some embodiments, the second therapeutic agent is selected from the group consisting of a chemotherapeutic, an immunotherapy (e.g., an agent for immune checkpoint blockade such as a PD-1 inhibitor, optionally with or without one or more CTLA-4 inhibitors), an antioxidant, an antiinflammatory agent, an antimicrobial, a steroid, etc.

The agent or composition can be administered concurrently with, prior to, or subsequent to one or more additional pharmaceutical agents, which may be useful as, e.g., combination therapies. Pharmaceutical agents include therapeutically active agents. Pharmaceutical agents also include prophylactically active agents. Pharmaceutical agents include small organic molecules such as drug compounds (e.g., compounds approved for human or veterinary use by the U.S. Food and Drug Administration as provided in the Code of Federal Regulations (CFR)), peptides, proteins, carbohydrates, monosaccharides, oligosaccharides, polysaccharides, nucleoproteins, mucoproteins, lipoproteins, synthetic polypeptides or proteins, small molecules linked to proteins, glycoproteins, steroids, nucleic acids, DNAs, RNAs, nucleotides, nucleosides, oligonucleotides, antisense oligonucleotides, lipids, hormones, vitamins, and cells. In certain embodiments, the additional pharmaceutical agent is a pharmaceutical agent useful for treating and/or preventing a disease described herein. Each additional pharmaceutical agent may be administered at a dose and/or on a time schedule determined for that pharmaceutical agent. The additional pharmaceutical agents may also be administered together with each other and/or with the agent or composition described herein in a single dose or administered separately in different doses. The particular combination to employ in a regimen will take into account compatibility of the agent described herein with the additional pharmaceutical agent(s) and/or the desired therapeutic and/or prophylactic effect to be achieved. In general, it is expected that the additional pharmaceutical agent(s) in combination be utilized at levels that do not exceed the levels at which they are utilized individually. In some embodiments, the levels utilized in combination will be lower than those utilized individually.

The additional pharmaceutical agents include, but are not limited to, additional EGLN1 and/or VHL inhibitors, other anti-cancer agents, immunotherapy and/or immunomodulatory agents, anti-proliferative agents, cytotoxic agents, anti-angiogenesis agents, anti-inflammatory agents, immunosuppressants, anti-bacterial agents, anti-viral agents, cardiovascular agents, cholesterol-lowering agents, anti-diabetic agents, anti-allergic agents, contraceptive agents, and pain-relieving agents. In certain embodiments, the additional pharmaceutical agent is an anti-proliferative agent. In certain embodiments, the additional pharmaceutical agent is an anti-cancer agent. In certain embodiments, the additional pharmaceutical agent is an anti-viral agent. In certain embodiments, the additional pharmaceutical agent is selected from the group consisting of epigenetic or transcriptional modulators (e.g., DNA methyltransferase inhibitors, histone deacetylase inhibitors (HDAC inhibitors), lysine methyltransferase inhibitors), antimitotic drugs (e.g., taxanes and vinca alkaloids), hormone receptor modulators (e.g., estrogen receptor modulators and androgen receptor modulators), cell signaling pathway inhibitors (e.g., tyrosine kinase inhibitors), modulators of protein stability (e.g., proteasome inhibitors), Hsp90 inhibitors, glucocorticoids, all-trans retinoic acids, and other agents that promote differentiation. In certain embodiments, the agents described herein or pharmaceutical compositions can be administered in combination with an anti-cancer therapy including, but not limited to, surgery, radiation therapy, transplantation (e.g., stem cell transplantation, bone marrow transplantation), immunotherapy, and chemotherapy.

Dosages for a particular agent of the instant disclosure may be determined empirically in individuals who have been given one or more administrations of the agent.

Administration of an agent of the present disclosure can be continuous or intermittent, depending, for example, on the recipient's physiological condition, whether the purpose of the administration is therapeutic or prophylactic, and other factors known to skilled practitioners. The administration of an agent may be essentially continuous over a preselected period of time or may be in a series of spaced doses.

Guidance regarding particular dosages and methods of delivery is provided in the literature; see, for example, U.S. Patent Nos. 4,657,760; 5,206,344; or 5,225,212. It is within the scope of the instant disclosure that different formulations will be effective for different treatments and different disorders, and that administration intended to treat a specific organ or tissue may necessitate delivery in a manner different from that to another organ or tissue. Moreover, dosages may be administered by one or more separate administrations, or by continuous infusion. For repeated administrations over several days or longer, depending on the condition, the treatment is sustained until a desired suppression of disease symptoms occurs. However, other dosage regimens may be useful. The progress of this therapy is easily monitored by conventional techniques and assays.

### Kits

The instant disclosure also provides kits containing agents of this disclosure for use in the methods of the present disclosure. Kits of the instant disclosure may include one or more containers comprising an agent (e.g., an EGLN1 and/or VHL inhibitor) of this disclosure and/or may contain agents (e.g., oligonucleotide primers, probes, etc.) for identifying a cancer or subject as EGLN1-dependent. In some embodiments, the kits further include instructions for use in accordance with the methods of this disclosure. In some embodiments, these instructions comprise a description of administration of the agent to treat or diagnose, e.g., an EGLN1-dependent cancer, according to any of the methods of this disclosure. In some embodiments, the instructions comprise a description of how to detect an EGLN1-dependent cancer, for example in an individual, in a tissue sample, or in a cell. The kit may further comprise a description of selecting an individual suitable for treatment based on identifying whether that subject has an EGLN1-dependent cancer.

The instructions generally include information as to dosage, dosing schedule, and route of administration for the intended treatment. The containers may be unit doses, bulk packages (e.g., multi-dose packages) or sub-unit doses. Instructions supplied in the kits of the instant disclosure are typically written instructions on a label or package insert (e.g., a paper sheet included in the kit), but machine-readable instructions (e.g., instructions carried on a magnetic or optical storage disk) are also acceptable.

The label or package insert indicates that the composition is used for treating, e.g., an EGLN1-dependent cancer, in a subject. Instructions may be provided for practicing any of the methods described herein.

The kits of this disclosure are in suitable packaging. Suitable packaging includes, but is not limited to, vials, bottles, jars, flexible packaging (e.g., sealed Mylar or plastic bags), and the like. Also contemplated are packages for use in combination with a specific device, such as an inhaler, nasal administration device (e.g., an atomizer) or an infusion device such as a minipump. A kit may have a sterile access port (for example the container may be an intravenous solution bag or a vial having a stopper pierceable by a hypodermic injection needle). The container may also have a sterile access port (e.g., the container may be an intravenous solution bag or a vial having a stopper pierceable by a hypodermic injection needle). In certain embodiments, at least one active agent in the composition is an EGLN1 and/or VHL inhibitor. The container may further comprise a second pharmaceutically active agent.

Kits may optionally provide additional components such as buffers and interpretive information. Normally, the kit comprises a container and a label or package insert(s) on or associated with the container.

The practice of the present disclosure employs, unless otherwise indicated, conventional techniques of chemistry, molecular biology, microbiology, recombinant DNA, genetics, immunology, cell biology, cell culture and transgenic biology, which are within the skill of the art. See, e.g., Maniatis et al., 1982, Molecular Cloning (Cold Spring Harbor Laboratory Press, Cold Spring Harbor, N.Y.); Sambrook et al., 1989, Molecular Cloning, 2nd Ed. (Cold Spring Harbor Laboratory Press, Cold Spring Harbor, N.Y.); Sambrook and Russell, 2001, Molecular Cloning, 3rd Ed. (Cold Spring Harbor Laboratory Press, Cold Spring Harbor, N.Y.); Ausubel et al., 1992), Current Protocols in Molecular Biology (John Wiley & Sons, including periodic updates); Glover, 1985, DNA Cloning (IRL Press, Oxford); Anand, 1992; Guthrie and Fink, 1991; Harlow and Lane, 1988, Antibodies, (Cold Spring Harbor Laboratory Press, Cold Spring Harbor, N.Y.); Jakoby and Pastan, 1979; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I- IV (D. M. Weir and C. C. Blackwell, eds., 1986); Riott, Essential Immunology, 6th Edition, Blackwell Scientific Publications, Oxford, 1988; Hogan et al., Manipulating the Mouse Embryo, (Cold Spring Harbor Laboratory Press, Cold Spring Harbor, N.Y., 1986); Westerfield, M., The zebrafish book. A guide for the laboratory use of zebrafish (Danio rerio), (4th Ed., Univ. of Oregon Press, Eugene, 2000).

Unless otherwise defined, all technical and scientific terms used herein have the same meaning as commonly understood by one of ordinary skill in the art to which this disclosure belongs. Although methods and materials similar or equivalent to those described herein can be used in the practice or testing of the present disclosure, suitable methods and materials are described below. In case of conflict, the present specification, including definitions, will control. In addition, the materials, methods, and examples are illustrative only and not intended to be limiting.

Reference will now be made in detail to exemplary embodiments of the disclosure. While the disclosure will be described in conjunction with the exemplary embodiments, it will be understood that it is not intended to limit the disclosure to those embodiments. To the contrary, it is intended to cover alternatives, modifications, and equivalents as may be included within the spirit and scope of the disclosure as defined by the appended claims. Standard techniques well known in the art or the techniques specifically described below were utilized.

### EXAMPLES

### Example 1: Materials and Methods

### Analysis of Dependency Data

Gene dependency data from Project Achilles, including data from the screening of 501 cancer cell lines by RNAi (~94k shRNAs, ~5 shRNAs/gene) and 436 cancer cell lines with CRISPR-Cas9 (~70k sgRNAs, ~4 sgRNAs/gene) (1-5). EGLN1 was originally identified as an interesting target to pursue using the DEMETER six sigma dependencies (2). Further analyses were performed using DEMETER2 RNAi (20, 54) and CERES CRISPR/Cas9 dependency data (7, 19, 54, 55). All significant findings have been summarized in the supplemental tables.

### Identifying Features Associated with EGLN1 Dependency

Multiple approaches were employed to find relationships between EGLN1 dependency and mRNA expression, copy-number, mutation, or dependency data (see below "CCLE Omics Data" section for details). To identify genes whose mutation was associated with EGLN1 dependency, two-sample t-tests were used to measure the mean-difference of dependency between mutant and WT cell lines for each gene and type of mutation (damaging or missense). Only mutations present in at least 5 cell lines were included in the analysis. To identify other continuous-valued (expression, copy-number, dependency) features associated with EGLN1 dependency, the R package Limma was used (66). Linear model coefficients were used to assess the strength of association between each feature and EGLN1 dependency, P-values were determined using empirical-Bayes moderated t-statistics (analyzing each data type separately). Note that this method was also used to identify top dependencies associated with HIF1A expression in FIG. 8M.

### CCLE Omics Data

The following CCLE (55) datasets were used, which are available for download at depmap.org/portal/download/all/.
mRNA Expression: CCLE_DepMap_18Q2_RNAseq_RPKM_20180502.gct
Copy-number: public_18Q2_gene_cn_v2.csv
Damaging/Non-damaging Mutations: CCLE_DepMap_18Q2_maf_ 20180502.txt (genes are classified as having a "damaging mutation" if isDeleterious is "TRUE" for any of its mutations).

### Identifying Gene Sets Associated with EGLN1 Dependency

Using the GenePattern software platform developed by the Broad Institute, single-sample GSEA (ssGSEA) was run on ovarian mRNA expression to calculate the activity level of each HALLMARK gene set in each ovarian cell line (using the Hallmark collection from the Molecular Signatures Database). Gene sets whose ssGSEA values were associated with EGLN1 dependency were then identified using linear model analysis as mentioned above.

### Lineage Enrichment Analysis

Using a one-sided Fisher's exact test, the positive enrichment of EGLN1-dependent lines in a given lineage compared to all other lineages was measured. CRISPR EGLN1-dependencies were defined as cell lines with at least a 50% probability of being dependent, which leveraged the CERES probabilities of dependency that were published alongside the CERES effect scores (19). Lineages represented by fewer than 5 lines were filtered out and false discovery rates were calculated to correct for multiple hypothesis testing.

### Cell Culture Conditions

All cell lines were provided from stocks curated by the Cancer Dependency Map project at the Broad Institute and originally obtained from the Cancer Cell Line Encyclopedia (broadinstitute.org/ccle) unless otherwise indicated. All cell lines were fingerprinted using one of two genotyping platforms, Sequenom or Fluidigm. Mycoplasm testing was performed when cell lines were obtained from CCLE. Cell lines expressing pLX _311-cas9 were generated by the Project Achilles for use in the genome-wide pooled CRISPR screening data used to inform this project. ES2, OVTOKO, TOV112D, HEYA8, OVISE were cultured in RPMI-1640+10% FBS. OVK18 was cultured in MEM+10% FBS. COV434 was cultured in DMEM+10%FBS+2mM Glutamine. TOV21G was cultured in MCDB105:Medium199 (1:1)+15% FBS. JHOC5 was cultured in DMEM:F12 (1:1)+10% FBS+0.1mM NEAA.. All media were supplemented with penicillinstreptomycin.

### Arrayed Lentivirus Production

293T cells were seeded in 6 well plates at 1.5×10⁶ per well (2mL volume) 24 h pretransfection. Transfection was performed using TransIT-LTI Transfection Reagent (Mirus). 8.25uL of LT1 was diluted in 75uL of Opti-Mem (Corning) for each well and incubated at room temperature for 5 min. The diluted LTI mixture was added to a mixture of 1250 ng PsPAX2, 250ng VSVG, and 1250 ng lentivector DNA suspended in Opti-MEM and incubated at room temperature for 20 - 30 minutes. The transfection mixture was then added to the plate of cells, spun at 1000g for 30 min, and incubated at standard cell culture conditions (37°C, 5% CO2) for 6 - 8 hours. Media were then changed to high serum media (DMEM + 30% FBS) and incubated until harvest after 48-72 h. Virus was stored at -80°C until use in the experiments.

### Immunoblots

Cells were infected with lentiviruses expressing sgRNAs targeting EGLN1 and selected with puromycin at a concentration of 4ug/mL for 48-72 h or until all uninfected cells were dead. Whole cell lysates were prepared using RIPA buffer (Sigma-Aldrich) supplemented with EDTA-free Protease Inhibitor Cocktail (Roche), 1mM Sodium Orthovanadate (NEB), and 5mM Sodium Fluoride (NEB). Protein levels were quantified using the Pierce BCA assay kit (Thermo Fisher Scientific). Protein lysates were run on 4-12% Bis-Tris Pre-Cast gels (Thermo Fisher Scientific) and proteins transferred to a PVDF membrane using the iBlot 2 system (Thermo Fisher Scientific). HIF1a levels were detected using a mouse monoclonal anti-HIF1a Antibody at 1:1000 dilution (BD Biosciences #610958) and a LICOR-compatible anti-mouse IR secondary antibody (LICOR #926-68020) at 1:5000 dilution. EGLN1 levels were detected using a rabbit monoclonal EGLN1 antibody (Cell Signaling #3293) at 1:1000 and a LICOR-compatible anti-rabbit IR secondary antibody (#926-32211) at 1:5000 dilution. VHL levels were detected using a rabbit monoclonal VHL antibody (Cell Signaling #685475) and a LICOR-compatible anti-rabbit IR secondary antibody. GAPDH levels were detected using a rabbit monoclonal GAPDH antibody (Cell Signaling 14C10 #2118) at 1:1000 and the same LICOR-compatible anti-rabbit IR secondary antibody at 1:5000 dilution.

### EGLN1 and VHL inhibitors

EGLN1 inhibitors [IOX2 (Catalog No. S2919), Roxadustat FG-4592 (Catalog No. S1007), Molidustat Bay-85-3934 (Catalog No. S8138), FG-2216 (Catalog No. S7979), and Daprodustat GSK1278863 (Catalog No. S8171] were obtained from Selleck Chemicals (selleckchem.com). The VHL inhibitor VH298 was obtained from Tocris Bioscience (Catalog No. 6156). Compounds were dissolved in DMSO per manufacturer's instructions to an initial stock concentration of 10 mM or formulated in 50% PEG for microdevice experiments.

### Luciferase Competition Assay

Infections to make cell lines expressing ORFs for Firefly Luciferase 2P (gateway cloned into pLX_313 from Promega pGL4.11[FLuc2P]) and Renilla Luciferase (pLX_313) were performed by centrifuging freshly seeded plates containing cells with lentiviral particles and 4 µg/mL polybrene for two hours at 2000 rpm. Cell lines expressing pLX_311-cas9 were infected with pLX_313-FireflyLuc2P and parental cell lines were infected with pLX_313-RenillaLuc. Firefly and Renilla expressing cells were mixed 1:1 in wells of a 96 well plate containing 4ug/mL polybrene. After mixing the cell population, sgRNA lentivirus was added. Both firefly and Renilla expressing cells were infected with the sgRNA but only the firefly expressing cells had Cas9. Freshly seeded plates were spun at 2000 rpm for 30 min and incubated overnight at standard cell culture conditions. The following day, infected cells were selected using 4 µg/mL puromycin for 48 h. Every 3-4 days following the selection period, the plate was split into two new plates. One plate was read 24 h later using reagents from a Dual-Glo Luciferase Assay Kit (Promega). The other plate continued until the next time point. Luciferase signal was quantified using an Envision Plate Reader. Data were expressed as the fold change of the ratio of Firefly Luciferase signal to Renilla Luciferase signal normalized to the signal ratio post-selection from an infection with the empty sgRNA delivery vector (xPR_003) and averaged across 6 biological replicates. Results are representative of two independent experiments.

### CellTiter-Glo Viability Assays

CellTiter-Glo (CTG) viability assays were performed per the manufacturer's instructions. Briefly, 5,000-10,000 cells were seeded per well in a 96 well plate then treated with either DMSO or EGLN1 inhibitor in doses indicated. Following 3-6 days treatment (retreated at day 4) CTG compound was added and viability was measured. Data presented are the average of three technical replicates. Data are representative of three independent experiments.

### Proliferation Assays

Non-Cas9 expressing cells were plated on a 12 well or 6 well plate in triplicate with varying concentrations of FG-4592. Every 3-4 days cells were trypsinized, counted using Nexcelom Cellometer, and reseeded at equal densities with the same concentrations of FG-4592, IOX2, BAY85-3934 or VH298. Data presented are the average of three technical replicate wells when available, excluding wells where there were no live cells to passage. Data are representative of two independent experiments.

### Flow Cytometry Analysis

Flow cytometry was performed at the Broad Institute Flow Cytometry core using CFSE (5(6)-Carboxyfluorescein N-hydroxysuccinimidyl ester)- Cell Labeling Kit (Abcam ab113853) to label dividing cells with CFSE and analyzed on FL-1 Excitation(max) 492nm, Emission(max) 517nm. Apoptosis was detected using PE Annexin V Apoptosis Detection Kit 1 (BD Biosciences 559763). Dual labeling Annexin V with 7-Amino-Actinomycin (7-AAD) allowed quantification of apoptosis. Data are presented as the average of two technical replicates and are representative of three independent experiments. Data were analyzed using FlowJo data analysis.

### Hypoxia Response Element

The Hypoxia Response Element (HRE) was designed based on previous publications (18, 19) and was cloned with a luciferase reporter. The construct was then stably transfected to EGLN1 dependent and independent cell lines. Following transfection cells were treated with EGLN1 inhibitor for up to 6 days and luciferase activity was measured as a readout of HIF1A activity.

### Colony Formation Assay Dose Response Curves

Cells were plated at a low density (250 - 1000 cells/well) with varying concentrations of FG-4592 (Selleck) on 24 well plates. Media and drug were refreshed every 3-4 days for 10 days. Cells were fixed on the plate with 10% neutral buffered formalin (Thermo Fisher Scientific) and stained with 0.1% crystal violet in 10% ethanol. After plates were washed and dried, dye was extracted using 10% acetic acid. Absorbance of extracted dye samples at 595nm was quantified using a Molecular Devices SpectraMax M5 Plate Reader. Data presented have been normalized to the DMSO treated sample. Data are representative of three independent experiments.

### Animal Models

Female Athymic^{Nu/Nu} mice (8-10 weeks old) were used, were obtained from Jackson Laboratories and had access to food and water in appropriate housing conditions. All animal procedures performed at the Broad Institute were approved by the Institutional Animal Care and Use Committee (IACUC) and according to institutional regulations.

### In Vivo Transplantation

Cells were infected with lentivirus expression control sgChr2-1 or sgEGLN1-9. Following 24 h of selection cells were subcutaneously implanted into recipient mice (athymic^{Nu/Nu}, Jackson Laboratories) in 50% matrigel at a concentration of 250,000 cells (ES2) or 400,000 cells (OVISE). Mice were implanted with sgChr2-1 and sgEGLN1-9 at opposite side flanks to control for any variability between mice. Following the detection of tumors, tumor size was quantified at least once per week using a caliper measuring tumor dimensions. Mice were euthanized at the end of the experiment. Tumors were harvested from the euthanized mice and dissociated in cell culture and further saved for RNA, protein or frozen cell storage.

### RT-PCR

ES2-formed tumors harvested from implanted cells were isolated from total tumor. Total RNA was isolated using QIAGEN miRNeasy Mini Kits. Reverse transcription for RNA samples was performed using Thermo Fisher Superscript III First-Strand Synthesis System (Thermo Fisher #18080-051). RT-PCR was performed on the QuantStudio 6 Flex (Applied Biosystems) using SYBR Green Master Mix available from Thermo Fisher (Thermo Fisher #4367659) with probes against EGLN1 deletion site in triplicate. Comparative CT was chosen for analysis and was normalized against RPS17 as an internal control.

### RNA Sequencing

RNA sequencing was performed following 5-day treatment of EGLN1-dependent and independent cell lines in triplicate with either EGLN1 inhibitor or DMSO (control). Cell lines also had EGLN1 KO or Chr2-1 KO (control) and were allowed to grow for 7 days before cell pellets were collected and RNA was isolated using Qiagen Trizol (Qiazol) RNA extraction kit. Purified RNA was provided to RNA sequencing core at the Broad Institute and sequencing was performed on the Illumina HiSeq 2000 or HiSeq 25000 instruments with coverage of 100 million paired 101 nucleotides-long reads per sample. RNA reads were aligned using TopHat version 1.4 and Gene RPKM values were calculated using GTEx project pipeline.

### Microdevice manufacturing and implantation

Devices were manufactured as previously described (42). Briefly, microscale devices at diameter 820 µM and length 3 mm were manufactured and pure powder compounds were formulated in 50% PEG. Mice were subcutaneously delivered ovarian cancer cells and tumors were allowed to grow to 1 cm². Mice were anesthetized during implantation using 1% isoflurane. A 19-guage spinal biopsy needle (Angiotech) was inserted into the tumor and served as the location for the device to be implanted.

### Immunohistochemistry

Immunohistochemistry was performed on 4 µm serial sections from formalin-fixed, paraffinembedded (FFPE) tumor tissues. The samples were deparaffinized, rehydrated, and pretreated for antigen retrieval by microwave treatment. Immunostaining was done using cell proliferation marker Ki67 and cleaved caspase 3 (CCE) at the Koch Institute Microscopy Core Facility.

### Statistical Analysis

All experiments presented herein are shown either as the average of at least three samples repeated twice and analyzed either with standard student's t-test or ANOVA as appropriate. Computational tests such as Gene Set Enrichment Analysis and TCGA statistical analysis were performed using computational software tools developed at the Broad Institute and Dana Farber Cancer Institute.

### Example 2: Genome-Wide Interrogation of Human Cancers Identified EGLN1 as a Druggable Preferential Dependency, in Clear Cell Ovarian Cancers and Melanoma

Genes that are essential for cell viability in a context-specific manner, in contrast to panessential genes, represent potential cancer dependencies. To identify such genes, genome-scale loss-of-function screens were performed using RNAi and CRISPR-Cas9 technologies in hundreds of human cancer cell lines (2, 3, 5). Data derived from screening 501 human cancer cell lines with RNAi were analyzed, and 762 genes were identified that were essential for the proliferation/survival of a subset of cell lines at a level of 6 standard deviations from the mean dependency score; a stringent metric to find such differential dependencies (2, 3, 5). Of these 762 genes, 153 of them were found to be classified as druggable based on previous annotations (2) (FIG. 1A, Supplementary Table 2)(2). Among the druggable genes, 15 were targets of molecules that are either approved or in clinical trials. As expected, most of these compounds have been developed for oncology indications, providing proof of concept of using this approach in identifying cancer targets. In addition, one gene, Egl-9 Family Hypoxia Inducible Factor 1 (EGLN1) was identified for which small molecule inhibitors are in phase II and III clinical trials to treat anemia in patients with chronic kidney disease (NCT03263091, NCT03303066, clinicaltrials.gov). This target was selected for further investigation as a candidate novel oncology therapeutic target.

To validate EGLN1 dependency with an orthogonal technology to RNAi, data derived from screening 436 cell lines using a genome-scale CRISPR-Cas9 library (7, 18) were analyzed. EGLN1 also scored as a preferential dependency in both CRISPR and RNAi datasets (FIGs. 1B and 8C) (19, 20). Indeed, the concordance between EGLN1 dependency in cell lines screened by CRISPR and RNAi was highly significant (FIG. 1C, Pearson correlation 0.512, p<10⁻¹⁷).

Since *EGLN1* is one of three family members, other EGLN family members (*EGLN2* and *EGLN3)* were also examined for any dependencies in any of the cell lines. Among the EGLN family members, *EGLN1* was identified as the strongest preferential dependency in both CRISPR and RNAi datasets (FIGs. 1A-1C). Furthermore, few cell lines were found to be dependent on *EGLN1* that were also identified as dependent on *EGLN2* or *EGLN3.*

Co-dependencies derived from the Achilles datasets have previously been used to identify genes that have similar function (2, 11-13, 21, 22). Here, this method has been applied to gain insight into the molecular basis of *EGLN1* dependency. Specifically, linear models were constructed to identify co-dependency relationships between *EGLN1* and every other gene. In the CRISPR-Cas9 screens, it was found that VHL was the strongest and most significantly associated dependency, while *HIF1AN* (Hypoxia Inducible Factor 1 Alpha Subunit Inhibitor) was among the top hits in both CRISPR-Cas9 and RNAi screens, with *HIF1A* one of the strongest negatively associated hits (FIGs. 1D and 8D). These observations indicated that *EGLN1* dependency was related to its canonical function in the HIF pathway. To further investigate this association with members of the HIF pathway, the correlations between dependency profiles of every pair of genes in the pathway (*VHL, HIF1AN, HIF1A, HIF2A, EGLN1, EGLN2* and *EGLN3)* were calculated. It was thereby identified that the strongest correlation within the pathway existed between EGLN1 dependency and VHL dependency, followed by *EGLN1* dependency and *HIF1AN* dependency (Hypoxia Inducible Factor 1 Alpha Subunit Inhibitor), in CRISPR datasets (FIG. 1E).

With the goal of understanding why some cells are more dependent upon EGLN1 than others, genomic features including gene expression, copy number alterations and mutations that related to EGLN1 dependency were searched for in the CRISPR and RNAi datasets (FIGs 8E-8L, Supplementary Table 3, Supplementary Table 4). Higher levels of *HIF1A* expression were identified as among the most significantly associated gene expression features. In addition, in the reverse analysis, EGLN1 dependency was the top correlated dependency with *HIF1A* expression (FIG. 8M). In particular, cell lines that expressed lower levels of *HIF1A* were not dependent on *EGLN1,* which indicated that high levels of expression were required for a dependency (FIG. 1F). Finally, gene sets that correlated with *EGLN1* dependency were investigated and hypoxia-related gene sets were identified among the top correlated gene sets (FIGs. 8N and 8O). Taken together, these observations were consistent with the conclusion that EGLN1 regulation of HIF1A was the downstream event responsible for the dependency.

### Example 3: EGLN1 Dependency was Enriched in Ovarian Clear Cell Carcinoma and Melanoma

To investigate whether EGLN1 dependency was enriched in specific cancer types, a lineage enrichment analysis was performed using the Fisher's exact test. Such analysis identified that melanoma and ovarian cancer cell lines were significantly enriched for EGLN1 dependencies in the CRISPR-Cas9 and RNAi datasets (FIG. 9A, FDR<0.1). Specifically, among the ovarian cancer cell lines, 13 of 33 (39%) ovarian cancer cell lines were dependent on EGLN1 (FIGs. 2A and 2B). Similarly, these cell lines were also found to be dependent on *EGLN1* in the RNAi dataset, and cell lines in both datasets showed strongly correlated *EGLN1* dependencies (FIG. 9C). Because ovarian cancer represents a heterogeneous disease comprised of four major different subtypes (23-33), *EGLN1* dependency was examined in individual subtypes. To determine whether EGLN1 dependency was enriched in a specific subtype, the ovarian cancer cell lines were grouped by historical pathological, molecular and histological subtypes (Supplementary Table 5, adapted from (28, 31, 33)). In such studies, *EGLN1* dependency was identified as significantly enriched in ovarian clear cell carcinoma lines, as compared to every other ovarian subtype (FIG. 2C, p<0.05; FIG. 9D, p<0.05).

It was next evaluated whether the association of *EGLN1* dependency with high expression levels of *HIF1A* remained significant in ovarian cell lines. It was found that EGLN1-dependent ovarian cancer cells expressed *HIF1A* at significantly higher levels than non-EGLN1-dependent cells in the CRISPR dataset (FIG. 2D, p < 0.005). A similar relationship was found between *EGLN1* dependency scores and *HIF1A* expression in the RNAi dataset (FIG. 9E). In particular, when subdivided by histological subtypes, it was found that ovarian clear cell lines showed the highest group expression of *HIF1A* in CRISPR (FIG. 2E, p<0.05) and RNAi datasets (FIG. 9E). These results are consistent with previous observations that *HIF1A* is overexpressed or over-activated in ovarian clear cell cancers, as compared to other ovarian cancer subtypes (34, 35).

Higher levels of *HIF1A* and a hypoxia gene set were identified as positively correlated with *EGLN1* dependency within the ovarian cell lines after performing single-sample Gene Set Enrichment Analysis (ssGSEA, FIGs. 2F and 9F), which indicated that in ovarian cell lines *EGLN1* dependency was also related to its known function in the HIF1A pathway.

### Example 4: The EGLN1 Dependency Disclosed Herein Validated

To validate and characterize the observed *EGLN1* dependency, a series of 10 sgRNAs were designed, to identify sgRNAs that effectively deleted *EGLN1.* An EGLN1-dependent cancer cell line, ES2 (FIG. 3A), and an EGLN1-independent cell line, TOV112D (FIG. 10A), were selected from the panel of cell lines screened in Project Achilles. Three sgRNAs (EGLN1-1, EGLN1-8, and EGLN1-9) were chosen for subsequent *EGLN1* knockout experiments. It was observed that deleting *EGLN1* led to an increase in HIF1A and subsequently phosphorylated HIF1A (36, 37). Accordingly, it was concluded that these selected *EGLN1* sgRNAs effectively targeted EGLN1.

To assess the consequences of EGLN1 inhibition, cell proliferation was measured. Expression of the negative control sgChr2-1 (sgRNA designed to target an intergenic region on Chromosome 2) did not affect cell proliferation. In contrast, depletion of EGLN1 using sgRNAN EGLN1-9 led to a significant (p<0.05) decrease in cell proliferation in the EGLN1-dependent cell line ES2 (FIG. 3B) but did not affect proliferation of the EGLN1-independent cell line, TOV112D (FIG. 3C).

It was then hypothesized that EGLN1-dependent cells would also be dependent on VHL. To test this, VHL-knockout cells were generated using VBL-specific sgRNAs and loss of VHL was confirmed by immunoblotting (Supplementary Figure FIG. 10B). Notably, VBL-deficient cells showed a significant (p<0.05) decrease in population doublings (FIG. 3D).

To assess the effects of depleting EGLN1 in a longer-term assay, a competition experiment was performed in which it was assessed whether cells harboring an EGLN1 knockout would be depleted over time, as directly compared to cells expressing a control sgRNA (FIG. 3E). Specifically, different sgRNAs were introduced in Renilla or Firefly+Cas9 expressing cells, and the consequences of expressing each sgRNA were then determined by assessing the Firefly/Renilla ratio over time. The competition assay was performed in three EGLN1-dependent cell lines (ES2, OVTOKO and OVISE) and two cell lines not dependent on EGLN1 (TOV112D and HEYA8). As a positive control for depletion of the cell population, sgRNAs targeting the RNA Polymerase II Subunit D (sgPOLR2D) and RNA Polymerase I Subunit C (sgPOLR1C) were used. As negative controls, sgChr2-1 and sgLacZ were used. It was thereby identified in ES2, OVTOKO and OVISE (EGLN1-dependent) that cells expressing an EGLN1-targeting sgRNA were depleted over time at a rate similar to cells expressing sgRNA targeting POIR2D or POLR1C (FIG. 3E, top). Furthermore, when this same experiment was performed in EGLN1-independent cell lines, no difference was identified between cells expressing the negative control sgRNA or sgRNA targeting EGLN1 (FIG. 3E, bottom). These observations confirmed that depletion of EGLN1 led to decreased cell proliferation in a subset of ovarian cancer cell lines.

### Example 5: Pharmacological Inhibition of EGLN1 or VHL Inhibited Cell Proliferation in a Subset of Cell Lines

EGLN1 is a prolyl hydroxylase, and several PHD inhibitors have been described (38, 40) as preventing the degradation of HIF1A. Specifically, compound FG-4592, also known as Roxadustat, is currently being evaluated for the treatment of anemia in dialysis-dependent chronic kidney disease (CKD) and non-dialysis dependent CKD (40-43). It was found herein that exposure of both ES2 and TOV112D to FG-4592 and the compound Molidustat (Bay 85-3934) led to increased HIF1A levels and phosphorylated HIF1A in a concentration-dependent manner (FIGs. 4A and 11A-11C). This accumulation was observed at 5-10 µM and peaked at 20-40 µM, consistent with the reported effective concentrations of both compounds (43, 44). These concentrations (5 µM-40 µM) were used for all subsequent experiments to test the effect of pharmacological inhibition of EGLN1 in EGLN1-dependent cells.

HIF1A activity was also tested using a hypoxia-response element (HRE) fused to a luciferase reporter (FIG. 4B) (45). Following treatment with FG-4592, HIF1A activity was identified as increased in both EGLN1-dependent cell lines and cell lines not dependent on EGLN1. However, when HIF1A activity levels were compared between both cell lines, the EGLN1-dependent cell line exhibited higher HIF1A activity (FIG. 4B). This finding indicated that pharmacological inhibition of EGLN1 in an EGLN1-dependent cell line induced higher activity of HIF1A than in the cell line not dependent on EGLN1. It was also found that FG-4592 reduced viability in ES2 over 48 hours, while this treatment had no effect on TOV112D via CellTiter-Glo (CTG) assay in a dose-dependent manner (FIG. 4C). To confirm these observations, several other EGLN1 inhibitors were tested, including FG-2216 (Fibrogen) and Daprodustat (GSK1278863 GlaxoSmithKline). The EGLN1-dependent cell line ES2 showed sensitivity to all of these EGLN1 inhibitors (FIG. 11D). These observations confirmed that inhibition of EGLN1 activity recapitulated the effects observed by deleting *EGLN1.*

To investigate if the observed decrease in cell number was due to apoptosis or cell cycle arrest, cells were treated for three days with FG-4592 and then labeled with proliferation agent CFSE (Invitrogen). Proliferation was then analyzed by flow cytometry. Higher doses of FG-4592 reduced proliferation of ES2 cells but did not exert a similar effect on TOV112D cells (FIG. 11E). Furthermore, a slight increase in apoptosis was found following three days of EGLN1 inhibition, which indicated an initial cytotoxic effect (FIG. 11F) ). Thus, short-term pharmacological inhibition of EGLN1 resulted in decreased proliferation. To evaluate the effect of FG-4592 on long-term proliferation, both sensitive and resistant cells were treated for up to 30 days with FG-4592. Treatment with FG-4592 significantly reduced long term proliferation in sensitive cell lines (FIGs. 4D, 11G and 11H, p<0.01) but not insensitive cell lines (FIG. 4E). Together, these observations demonstrated that EGLN1 inhibition reduced cell proliferation.

To further understand EGLN1 dependency, the effect of EGLN1 inhibition was examined in a low cell density colony formation assay. EGLN1-dependent cell lines were identified as sensitive to EGLN1 inhibition (FIGs. 4F and 11I) in a dose-dependent measurement. When cell lines that did not exhibit dependence on EGLN1 were examined, increasing concentrations of FG-4592 did not have the same effect as that observed in EGLN1-dependent cell lines (FIGs. 4G and 11I)). Together, these results indicated that EGLN1-dependent cell lines were also dependent on EGLN1 in colonyformation cell viability assays.

It was hypothesized that EGLN1-sensitivity to small molecule inhibition would persist in the context of hypoxia. To test this hypothesis, colony-forming formation assays were performed in the context of normal culture conditions (normoxia) or in a hypoxic incubator at 5% oxygen (hypoxia). It was found that EGLN1-dependent cells were sensitive to EGLN1 small molecule inhibitor FG-4592 in a dose-dependent manner in hypoxia and normoxia (FIG. 11J). Increased sensitivity to EGLN1 inhibition was not observed in 5% oxygen with the EGLN1-insensitive cell line TOV112D, which indicated that EGLN1 dependency persisted under reduced oxygen concentrations *in vitro.*

Several inhibitors developed for clinical trials have the ability to target EGLN2 and EGLN3. To confirm that the effect disclosed herein was related to EGLN1 inhibition, the more selective EGLN1 inhibitor IOX2 was used. In a short-term viability assay, it was found that IOX2-mediated inhibition of EGLN1 resembled FG-4592 inhibition of EGLN1 (FIGs. 12A and 12B). Furthermore, when colony growth was assessed, it was found that EGLN1-dependent cell lines exhibited reduced dose-dependent colony growth, as compared to cell lines that were not dependent on EGLN1 (FIG. 12C). When the effect of IOX2 on long-term proliferation was assessed, it was found that EGLN1-dependent cell lines showed decreased proliferation over time (Supplementary Figure 5D-5F). These findings indicated that the effects observed with FG-4592 and other EGLN clinical inhibitors were predominantly through inhibition of EGLN1, not EGLN2 or EGLN3.

It was further considered that pharmacological inhibition of VHL should mimic pharmacological inhibition of EGLN1. To test this, VH298 (Tocris) was administered to cells. VH298 is a chemical probe that has recently been reported to block the interaction of VHL and HIF1A downstream of hydroxylation of HIF1A by EGLN1 (39), leading to HIF1A stabilization (FIG. 12G) and activation of HIF target genes. Treatment of ES2 cells with VH298 substantially decreased cell proliferation (FIG. 4H) while treatment of TOV112D cells slightly decreased proliferation (FIG. 4I). This effect was observed in several other EGLN1-dependent cell lines (FIGs. 12H and 12I). These findings indicated that EGLN1-dependent cells were more sensitive to VHL inhibition than EGLN1-independent cells.

### Example 6: Deletion of HIF1A Rescued EGLN1 and VHL Dependency

Based on the above-described observation that EGLN1 dependency was associated with higher expression of HIF1A, it was hypothesized that depleting EGLN1 would affect HIF1A targets and pathways. As a test of this hypothesis, RNA-sequencing was therefore performed in the EGLN1-dependent cell lines ES2 and OVISE with and without *EGLN1.* Among the genes that were differentially expressed in these cell lines (Supplementary Table 6, FIG. 13A), several genes that act downstream of the EGLN1-HIF1A pathway were identified. Gene Set Enrichment Analysis (GSEA) was next performed to identify significant changes in the EGLN1 knockout population. Within the top scoring pathways, there was significant enrichment in hypoxia and HIF1A-related pathways (FIGs. 5A and 13B). Together, these observations indicated that loss of EGLN1 strongly affected HIF1A-related pathways.

As HIF1A pathways were significantly affected by EGLN1 inhibition, it was hypothesized that knockout of *HIF1A* might rescue EGLN1 dependency. To test this hypothesis, several sgRNAs targeting *HIF1A* were designed and three were selected for further use that efficiently depleted HIF1A in EGLN1-sensitive cell line ES2 (FIG. 5B). sgRNA-mediated deletion of *HIF1A* failed to affect the proliferation of EGLN1-insensitive cell lines. However, such depletion of HIF1A rescued EGLN1-sensitivity to FG-4592 in ES2 (FIG. 5C) and OVISE (FIG. 5D) (p<0.05) cell lines. These observations indicated that the observed dependence of EGLN1 required HIF1A.

Since *HIF1A* knockout rescued EGLN1 dependency, it was next hypothesized that HIF1A knockout might also rescue VHL dependency. When compared to DMSO-treated control (FIG. 5E), knockout of HIF1A rescued cell proliferation blocked by VHL inhibitor VH298 (FIG. 5F, p<0.05), which indicated that VBL-dependency functioned through the regulation of HIF1A.

### Example 7: Genetic and Pharmacological Inhibition of EGLN1 Reduced Tumor Growth

It was then tested whether depletion of EGLN1 affected tumor growth *in vivo.* Specifically, cells expressing sgRNAs against EGLN1 or Chr2-1 (negative control) were employed, and subcutaneously implanted into recipient mice (FIG. 6A). Control and knockout cells were respectively implanted into opposite flanks of the same mouse to control for any tumor variation between mice. Knockout of EGLN1 significantly reduced tumor size over time in ES2 (FIG. 6B). Furthermore, OVISE tumor cells with deleted EGLN1 grew slower than OVISE tumors in which EGLN1 was expressed (FIG. 6C). When RNA expression of a subset of the tumors that had grown in ES2 cells with inhibited EGLN1 was examined, a correlation with EGLN1 expression and tumor size (FIG. 6D) was observed, which indicated that inactivation of the EGLN1 gene significantly inhibited tumor growth.

To determine if pharmacological inhibition of EGLN1 also inhibited tumor growth, an implantable microdevice was employed to directly deliver multiple compounds to different parts of the tumor (46). By inserting this microdevice directly into the tumor, the effect of FG-4592, Bay 85-3934 and VH298 in tumors was assessed *in vivo.* Specifically, ES2 cells were implanted with intact HIF1A (ES2) or HIF1A knockout (ES2 HIF1A KO) in immunodeficient mice and the tumors were allowed to grow to 1 cm² before implanting a microdevice containing the EGLN1 inhibitor FG-4592 and VHL inhibitor VH298. Following implantation, the tumors were collected after 48 hours and the tumors were sectioned and stained for proliferation with an antibody against Ki-67, a cellular marker for proliferating cells or Cleaved Caspase-3, a cellular marker for apoptosis. As expected, local delivery of PEG (polyethylene glycol, the vehicle used for drug formulation) had no effect on cell proliferation or apoptosis demonstrated by clear immunostaining of Ki-67 (FIGs. 6E and 14, top) in both HIF1A WT and HIF1A KO tumors. Furthermore, addition of doxorubicin reduced proliferation and increased apoptosis in both HIF1A WT and HIF1A KO cells (FIGs. 6E and 14, top middle). Consistent with previous *in vitro* data (FIG. 4), pharmacological inhibition of EGLN1 caused a dramatic reduction in proliferating cells and increased apoptosis in the region of tumor exposed to the local microdevice delivery of FG-4592 (FIGs. 6E and 14, bottom middle) while knockout of HIF1A exhibited no change in proliferation or apoptosis upon local delivery of FG-4592. Similarly, addition of VH298 resulted in the reduction of proliferating cells and increased apoptosis, which was also dependent on intact HIF1A (FIG. 6E and 14, bottom). These data collectively demonstrated (1) that genetic and pharmacological perturbation of EGLN1 reduced tumor burden and cell proliferation, while increasing apoptosis *in vivo* and (2) that this effect required HIF1A.

In sum, as demonstrated herein, under normal conditions, EGLN1 functions to hydroxylate HIF1A and target it for ubiquitination and subsequent degradation by VHL (FIG. 7A). In a subset of clear cell ovarian cancer and melanomas, genetic knockout or pharmacological inhibition of EGLN1 was herein identified to stabilize HIF1A and inhibit proliferation (FIG. 7B). Similarly, genetic knockout or pharmacological inhibition of VHL prevented HIF1A ubiquitination and degradation, which resulted in HIF1A stabilization and, subsequently, a decrease in proliferation (FIG. 7C). Deletion of *HIF1A* rescued both EGLN1 dependency and VHL dependency (FIG. 7D). Pharmacological inhibition of EGLN1 recapitulated the effects of genetic suppression of EGLN1, both in vitro and in vivo, which has newly indicated that EGLN1 inhibition provides a potential therapeutic strategy for these tumors.

As described above, genetic deletion of *EGLN1* led to HIF 1A accumulation (FIG. 3A), which in turn led to a decrease in proliferation (FIG. 3B) and reduced fitness in a competition assay, as compared to EGLN1-functional cells (FIG. 3E). After pharmacological inhibition of EGLN1, similar accumulation of HIF1A and increased HIF1A activity were observed (FIGs. 4A and 4B).

The currently disclosed findings indicated that increased HIF1A decreased the fitness of EGLN1-dependent cells. Specifically, inactivation of EGLN1, using either genetic or pharmacologic means, resulted in a pronounced decrease in proliferation. It was observed that this decrease in proliferation coincided with stabilization of HIF1A. Thus, it seemed likely that HIF1A loss could rescue EGLN1-mediated death. Indeed, EGLN1-dependent cell lines were rendered insensitive to EGLN1 inhibition by loss of HIF1A (FIGs. 5C and 5D). Together, these observations demonstrated that EGLN1 dependency requires HIF1A expression.

A large number of cell lines dependent on VHL have been identified herein (FIG. 8A). It has been further identified herein that cell lines dependent on EGLN1 were also dependent on VHL (FIGs. 3D, 4H, 12H and 12I). While all the EGLN1-dependent cell lines were also dependent on VHL in the CRISPR dataset (FIGs. 1D and 1E), it was surprising to observe VHL scored as an essential gene (FIG. 8A) . As VHL is downstream of EGLN1 and directly responsible for the ubiquitination and degradation of HIF1A (16, 17, 37-39), one possible reason for this observation was that loss of VHL could drive stabilization and accumulation of HIF1A in cell lines. Dependency on VHL in EGLN1-dependent cell lines was found to be dependent on the presence of functional HIF1A. Similar to observations for EGLN1 dependency, knockout of HIF1A rescued VHL dependency (FIG. 5F). This finding therefore indicated that VHL also functions as a cancer dependency in EGLN1-dependent cells, *via* negative regulation of HIF1A.

Previous reports found that over-expression of HIF1A (40, 41, 63) was associated with multiple tumor types, and tumors in hypoxia have been described to require HIF1A to survive (14-17). However, other reports also implicated HIF1A as a negative regulator of proliferation (47-49). Without wishing to be bound by theory, there are several possibilities that could explain these prior art observations. First, the observed effects of activating HIF1A may be tumor-type specific. In the instant disclosure, significant enrichment in EGLN1 dependency in melanoma and clear cell ovarian cancers was observed. Indeed, the clear cell ovarian cancer cell lines of the instnat disclosure died when HIF1A expression was stabilized.

Alternatively, the observed difference in sensitivity was possibly dependent upon whether the cells experienced hypoxia. However, as described herein, it was observed that under conditions of 5% oxygen EGLN1 dependent cells were still susceptible to EGLN1 inhibition. Furthermore, *in vivo* growth of tumors was examined under presumably hypoxic conditions, and it was found that genetic knockout and small molecule inhibition of EGLN1 reduced tumor growth and suppressed proliferation. These findings indicated that cell line dependence on EGLN1 was independent of hypoxia.

The standard procedure used to treat clear cell ovarian cancer remains surgical cytoreduction and subsequent chemotherapy using platinum agents and taxane (53). Clear cell ovarian cancers, as compared to other histological ovarian subtypes, are resistant to chemotherapy (53). Thus, targeted therapy has been proposed, and the need for potential targets and therapy has been pressing. EGLN1 inhibitors are currently in clinical trials for anemia in patients with chronic kidney disease (CKD) (40-42). The observations of the instant disclosure have indicated that these inhibitors are also useful in EGLN1-dependent cancers. The data disclosed herein have also indicated that cancers with low *HIF1A* expression will not respond to EGLN1 therapy, while the subset of patients with high *HIF1A* expression will respond. The association of EGLN1 therapy response with high *HIF1A* expression was stronger in melanoma and extremely strong in clear cell ovarian cancer cells. In fact, previous studies demonstrated that clear cell ovarian cancer patients had higher expression of HIF1A (34, 35). Therefore, melanoma and clear cell ovarian cancer patients, as well as other cancer patients potentially expressing high HIF1A, are among the ideal patient populations in which to assess the efficacy of EGLN1-targeted cancer therapies, such as those disclosed herein.

### References

1. R. Barrangou et al., Advances in CRISPR-Cas9 genome engineering: lessons learned from RNA interference. Nucleic Acids Res. 43, 3407-19 (2015).
2. A. Tsherniak et al., Defining a Cancer Dependency Map. Cell 170, 564-576 (2017).
3. D. E. Root, W. C. Hacohen N Fau et al., Genome-scale loss-of-function screening with a lentiviral RNAi library. Nat Methods 17, 715-9 (2006).
4. O. Shalem, N. E. Sanjana, F. Zhang, High-throughput functional genomics using CRISPR-Cas9. Nature reviews. Genetics 16, 299-311 (2015).
5. E. R. McDonald, 3rd et al., Project DRIVE: A Compendium of Cancer Dependencies and Synthetic Lethal Relationships Uncovered by Large-Scale, Deep RNAi Screening. Cell 170, 577-592 (2017).
6. F. J. Sanchez-Rivera, T. Jacks, Applications of the CRISPR-Cas9 system in cancer biology. Nat Rev Cancer. 15, 387-95 (2015).
7. Meyers et al., Computational correction of copy number effect improves specificity of CRISPR-Cas9 essentiality screens in cancer cells. Nat Genet 49, 1779-1784 (2017).
8. J. Peng, Y. Zhou, S. Zhu, W. Wei, High-throughput screens in mammalian cells using the CRISPR-Cas9 system. FEBS J. 282, 2089-96 (2015).
9. D. U. Kim et al., Analysis of a genome-wide set of gene deletions in the fission yeast Schizosaccharomyces pombe. Nat Biotechnol 28, 617-623 (2010).
10. T. Zhan, M. Boutros, Towards a compendium of essential genes - From model organisms to synthetic lethality in cancer cells. Critical Reviews in Biochemistry and Molecular Biology 51, 74-85 (2016).
11. B. Luo et al., Highly parallel identification of essential genes in cancer cells. Proc Natl Acad Sci USA 105, 20380-5 (2008).
12. H. W. Cheung et al., Systematic investigation of genetic vulnerabilities across cancer cell lines reveals lineage-specific dependencies in ovarian cancer. Proc Natl Acad Sci USA 108, 12372-7 (2011).
13. B. Paolella et al., Copy-number and gene dependency analysis reveals partial copy loss of wild-type SF3B1 as a novel cancer vulnerability. LID - 10.7554/eLife.23268 [doi] LID - e23268 [pii].
14. E. Berra et al., HIF prolyl-hydroxylase 2 is the key oxygen sensor setting low steady-state levels of HIF-1α in normoxia. The EMBO Journal 22, 4082-4090 (2003).
15. D. A. Tennant et al., Reactivating HIF prolyl hydroxylases under hypoxia results in metabolic catastrophe and cell death. Oncogene 28, 4009-21 (2009).
16. W. G. Kaelin, The VHL Tumor Suppressor Gene: Insights into Oxygen Sensing and Cancer. Transactions of the American Clinical and Climatological Association 128, 298-307 (2017).
17. K. K. To, L. E. Huang, Suppression of hypoxia-inducible factor 1alpha (HIF-1alpha) transcriptional activity by the HIF prolyl hydroxylase EGLN1. J Biol Chem 280, 38102-7 (2005).
18. A. J. Aguirre et al., Genomic Copy Number Dictates a Gene-Independent Cell Response to CRISPR/Cas9 Targeting. Cancer Discov 6, 914-29 (2016).
19. Cancer Data Science. Broad Institute Cancer Dependency Map, CRISPR Avana Dataset 18Q2 (Avana_public 18Q2). figshare. doi.org/10.6084/m9.figshare.6205118.v1 (2018).
20. Cancer Data Science. DEMETER2 data. figshare. doi.org/10.6084/m9.figshare.6025238.v4 (2018).
21. J. W. Kim et al., Characterizing genomic alterations in cancer by complementary functional associations. Nat Biotechnol 34, 539-46 (2016)
22. T. P. Howard et al., Functional Genomic Characterization of Cancer Genomes. Cold Spring Harb Symp Quant Biol 81, 237-246 (2016).
23. T. Kaur, S. D. Slavcev Ra Fau - Wettig, S. D. Wettig, Addressing the challenge: current and future directions in ovarian cancer therapy. Curr Gene Ther 9, 434-58 (2009)
24. M. S. Anglesio, M. S. Carey, M. Köbel, H. MacKay, D. G. Huntsman, Clear cell carcinoma of the ovary: A report from the first Ovarian Clear Cell Symposium, June 24th, 2010. Gynecologic Oncology 121, 407-415.
25. J. K. Chan et al., Do clear cell ovarian carcinomas have poorer prognosis compared to other epithelial cell types? A study of 1411 clear cell ovarian cancers. Gynecologic Oncology 109, 370-376.
26. K. Yamaguchi et al., Epigenetic determinants of ovarian clear cell carcinoma biology. Int J Cancer 135, 585-97 (2014).
27. E. Lengyel et al., Epithelial ovarian cancer experimental models. Oncogene 33, 3619-33 (2014).
28. J. Haley et al., Functional characterization of a panel of high-grade serous ovarian cancer cell lines as representative experimental models of the disease. Oncotarget 7, 32810-20 (2016).
29. N. Ahmed, K. L. Stenvers, Getting to know ovarian cancer ascites: opportunities for targeted therapy-based translational research. Front Oncol 23, 256 (2013).
30. G. C. Jayson, E. C. Kohn, H. C. Kitchener, J. A. Ledermann, Ovarian cancer. Lancet 384, 1376-88 (2014).
31. C. M. Beaufort et al., Ovarian Cancer Cell Line Panel (OCCP): Clinical Importance of In Vitro Morphological Subtypes. PLOS ONE 9, e103988 (2014).
32. D. Lim, E. Oliva, Precursors and pathogenesis of ovarian carcinoma. Pathology 45, 229242 (2013).
33. M. S. Anglesio et al., Type-Specific Cell Line Models for Type-Specific Ovarian Cancer Research. PLOS ONE 8, e72162 (2013).
34. S. Lee, W. R. Garner Ei Fau - Welch, R. S. Welch Wr Fau - Berkowitz, S. C. Berkowitz Rs Fau - Mok, S. C. Mok, Over-expression of hypoxia-inducible factor 1 alpha in ovarian clear cell carcinoma. Gynecol Oncol 106, 311-7 (2007).
35. R. Osada et al., Expression of hypoxia-inducible factor 1alpha, hypoxia-inducible factor 2alpha, and von Hippel-Lindau protein in epithelial ovarian neoplasms and allelic loss of von Hippel-Lindau gene: nuclear expression of hypoxia-inducible factor 1alpha is an independent prognostic factor in ovarian carcinoma. Hum Pathol 38, 1310-20 (2007).
36. T. Jokilehto et al., Overexpression and nuclear translocation of hypoxia-inducible factor prolyl hydroxylase PHD2 in head and neck squamous cell carcinoma is associated with tumor aggressiveness. Clin Cancer Res 12, 1080-7 (2006).
37. O. Aprelikova et al., Regulation of HIF prolyl hydroxylases by hypoxia-inducible factors. J Cell Biochem 92, 491-501 (2004).
38. M. Ivan, W. G. Kaelin, Jr., The EGLN-HIF O2-Sensing System: Multiple Inputs and Feedbacks. Mol Cell 66, 772-779 (2017).
39. J. Frost et al., Potent and selective chemical probe of hypoxic signaling downstream of HIF-alpha hydroxylation via VHL inhibition. Nature Communications 7, 13312 (2016).
40. L. Del Vecchio, F. Locatelli, Roxadustat in the treatment of anaemia in chronic kidney disease. Expert Opin Investig Drugs 27, 125-133 (2018).
41. A. Besarab et al., Randomized placebo-controlled dose-ranging and pharmacodynamics study of roxadustat (FG-4592) to treat anemia in nondialysis-dependent chronic kidney disease (NDD-CKD) patients. Nephrol Dial Transplant 30, 1665-73 (2015).
42. R. Provenzano et al., Roxadustat (FG-4592) Versus Epoetin Alfa for Anemia in Patients Receiving Maintenance Hemodialysis: A Phase 2, Randomized, 6- to 19-Week, Open-Label, Active-Comparator, Dose-Ranging, Safety and Exploratory Efficacy Study. American Journal of Kidney Diseases 67, 912-924 (2016).
43. C. Buisson et al., Detection by LC-MS/MS of HIF stabilizer FG-4592 used as a new doping agent: Investigation on a positive case. Journal of Pharmaceutical and Biomedical Analysis 121, 181-187 (2016).
44. I. H. Jain et al., Hypoxia as a therapy for mitochondrial disease. Science 352, 54-61 (2016).
45. S. Gao, J. Zhou, Y. Zhao, P. Toselli, W. Li, Hypoxia-response element (HRE)-directed transcriptional regulation of the rat lysyl oxidase gene in response to cobalt and cadmium. Toxicological sciences : an official journal of the Society of Toxicology 132, 379-389 (2013).
46. O. Jonas et al., An implantable microdevice to perform high-throughput in vivo drug sensitivity testing in tumors. Science translational medicine 7, 284ra257-284ra257 (2015).
47. C. Shen et al., Genetic and functional studies implicate HIF1alpha as a 14q kidney cancer suppressor gene. Cancer Discov 1, 222-35 (2011).
48. B. Chiavarina et al., HIF1-alpha functions as a tumor promoter in cancer associated fibroblasts, and as a tumor suppressor in breast cancer cells: Autophagy drives compartmentspecific oncogenesis. Cell Cycle 9, 3534-51 (2010).
49. A. Klotzsche-von Ameln, et al., Inhibition of HIF prolyl hydroxylase-2 blocks tumor growth in mice through the antiproliferative activity of TGFβ. Cancer Res 71(9), 3306-16 (2011).
50. D.A. Chan et al., Tumor vasculature is regulated by PHD2-mediated angiogenesis and bone marrow-derived cell recruitment. Cancer Cell 15(6), 527-38 (2009).
51. M.R. Bordoli et al., Prolyl-4-hydroxylase PHDS2- and hypoxia-inducible factor 2-dependent regulation of amphiregulin contributes to breast tumorigenesis. Onocogene 30(3) 548-60 (2011).
52. M.M. Winslow et al., Suppression of lung adenocarcinoma progression by Nkx2-1. Nature 473 101-4 (2011).
53. L.P. Cobb and D.M. Gershenson Treatment of Rare Epithelial Ovarian Tumors Hematol Oncol Clin North Am 32 1011-1024 (2018).
54. J. M. McFarland et al., Improved estimation of cancer dependencies from large-scale RNAi screens using model-based normalization and data integration. Nature Communications 9, 4610 bioRxiv, (2018).
55. Cancer Cell Line Encyclopedia Consortium, and Genomics of Drug Sensitivity in Cancer Consortium. 2015. Pharmacogenomic Agreement between Two Cancer Cell Line Data Sets. Nature 528 (7580):84-87. doi.org/10.1038/nature15736.

## Claims

1. An EGLN1 inhibitor or a VHL inhibitor for use in treating a subject having or at risk of developing an EGLN1-dependent ovarian cancer, wherein the treatment comprises:
(a) obtaining a sample from a subject having or at risk of developing the EGLN1-dependent ovarian cancer;
(b) identifying the presence or absence in the sample of the EGLN1-dependent ovarian cancer; and
(c) selecting an EGLN1 inhibitor or a VHL inhibitor as a treatment for the subject if the EGLN1-dependent ovarian cancer is identified in the sample, thereby selecting a treatment for the subject having or at risk of developing the EGLN1-dependent ovarian cancer, optionally further comprising:
(d) administering the selected EGLN1 inhibitor or VHL inhibitor to the subject;
wherein the EGLN1 inhibitor is selected from the group consisting of:
FG-4592, FG-2216, Molidustat, IOX2, Vadadustat and Daprodustat, optionally wherein the EGLN1 inhibitor is FG-4592; and
an oligonucleotide inhibitor of EGLN1, optionally wherein the oligonucleotide inhibitor of EGLN1 is selected from the group consisting of an antisense oligonucleotide, a siRNA and a sgRNA; and/or, wherein the VHL inhibitor is selected from the group consisting of:
VH298; and
an oligonucleotide inhibitor of VHL, optionally wherein the oligonucleotide inhibitor of VHL is selected from the group consisting of an antisense oligonucleotide, a siRNA and a sgRNA

2. The EGLN1 inhibitor or a VHL inhibitor for use according to claim 1, wherein the EGLN1-dependent ovarian cancer is a clear cell ovarian cancer.

3. The EGLN1 inhibitor or a VHL inhibitor for use according to claim 1, wherein step (b) comprises identifying the presence or absence in the sample of elevated Hypoxia Inducible Factor 1 Alpha (HIF1A), as compared to an appropriate control.

4. The EGLN1 inhibitor or a VHL inhibitor for use according to claim 1, wherein identifying step (b) comprises use of a kit for identifying the EGLN1-dependent ovarian cancer in a sample and selecting an EGLN1 inhibitor or a VHL inhibitor therapy for a subject, and instructions for its use.

5. The EGLN1 inhibitor or a VHL inhibitor for use according to claim 1, wherein the subject is human.

6. An EGLN1 inhibitor or a VHL inhibitor for use in treating or preventing an ovarian cancer in a subject having or at risk of developing such a cancer, wherein the treatment comprises:
(a) obtaining a sample from a subject having or at risk of developing an ovarian cancer;
(b) identifying the presence or absence in the sample of an EGLN1-dependent ovarian cancer; and
(c) administering an EGLN1 inhibitor or a VHL inhibitor to the subject if the EGLN1-dependent ovarian cancer is identified in the sample, thereby treating or preventing an ovarian cancer in a subject having or at risk of developing such a cancer;
wherein the EGLN1 inhibitor is selected from the group consisting of:
FG-4592, FG-2216, Molidustat, IOX2, Vadadustat and Daprodustat, optionally wherein the EGLN1 inhibitor is FG-4592; and
the VHL inhibitor is an oligonucleotide inhibitor of VHL, optionally wherein the oligonucleotide inhibitor of VHL is selected from the group consisting of an antisense oligonucleotide, a siRNA and a sgRNA.

7. The EGLN1 inhibitor or a VHL inhibitor for use according to claim 6, wherein step (b) comprises performing a single multiplex PCR reaction that is analyzed by capillary electrophoresis.

8. The EGLN1 inhibitor or a VHL inhibitor for use according to claim 6, wherein step (b) comprises identifying the presence or absence in the sample of elevated Hypoxia Inducible Factor 1 Alpha (HIF1A), as compared to an appropriate control.

9. The EGLN1 inhibitor or a VHL inhibitor for use according to claim 6, wherein: when the ovarian cancer is a clear cell ovarian cancer;
identifying step (b) comprises use of a kit for identifying the EGLN1-dependent ovarian cancer in the sample and selecting an EGLN1 inhibitor or a VHL inhibitor therapy for a subject, and instructions for its use;
and/or the subject is human.

## Patentansprüche

1. EGLN1-Inhibitor oder VHL-Inhibitor zur Verwendung bei der Behandlung eines Subjekts, das einen EGLN1-abhängigen Eierstockkrebs aufweist oder bei dem das Risiko besteht, daran zu erkranken, wobei die Behandlung umfasst:
(a) Erhalten einer Probe von einem Subjekt, das den EGLN1-abhängigen Eierstockkrebs aufweist oder bei dem das Risiko besteht, daran zu erkranken;
(b) Identifizieren des Vorhandenseins oder Fehlens des EGLN1-abhängigen Eierstockkrebses in der Probe; und
(c) Auswählen eines EGLN1-Inhibitors oder eines VHL-Inhibitors als Behandlung für das Subjekt, wenn der EGLN1-abhängige Eierstockkrebs in der Probe identifiziert wird, wodurch eine Behandlung für das Subjekt ausgewählt wird, das den EGLN1-abhängigen Eierstockkrebs aufweist oder bei dem das Risiko besteht, daran zu erkranken, optional weiter umfassend:
(d) Verabreichen des ausgewählten EGLN1-Inhibitors oder VHL-Inhibitors an das Subjekt;
wobei der EGLN1-Inhibitor aus der Gruppe ausgewählt ist, bestehend aus:
FG-4592, FG-2216, Molidustat, IOX2, Vadadustat und Daprodustat, wobei der EGLN1-Inhibitor optional FG-4592 ist; und
einem Oligonukleotid-Inhibitor von EGLN1, wobei der Oligonukleotid-inhibitor von EGLN1 optional aus der Gruppe ausgewählt ist, die aus einem Antisense-Oligonukleotid, einer siRNA und einer sgRNA besteht; und/oder wobei der VHL-Inhibitor aus der Gruppe ausgewählt ist, bestehend aus:
VH298; und
einem Oligonukleotid-Inhibitor von VHL, wobei der Oligonukleotid-Inhibitor von VHL optional aus der Gruppe ausgewählt ist, die aus einem Antisense-Oligonukleotid, einer siRNA und einer sgRNA besteht

2. EGLN1-Inhibitor oder VHL-Inhibitor zur Verwendung nach Anspruch 1, wobei der EGLN1-abhängige Eierstockkrebs ein klarzelliger Eierstockkrebs ist.

3. EGLN1-Inhibitor oder VHL-Inhibitor zur Verwendung nach Anspruch 1, wobei Schritt (b) das Identifizieren des Vorhandenseins oder Fehlens eines erhöhten Hypoxieinduzierbaren Faktors 1 Alpha (HIF1A) in der Probe im Vergleich zu einer geeigneten Kontrolle umfasst.

4. EGLN1-Inhibitor oder VHL-Inhibitor zur Verwendung nach Anspruch 1, wobei Identifizierungsschritt (b) die Verwendung eines Kits zum Identifizieren des EGLN1-abhängigen Eierstockkrebses in einer Probe und Auswählen einer EGLN1-Inhibitor- oder einer VHL-Inhibitor-Therapie für ein Subjekt, und Anweisungen für dessen Verwendung umfasst.

5. EGLN1-Inhibitor oder VHL-Inhibitor zur Verwendung nach Anspruch 1, wobei das Subjekt ein Mensch ist.

6. EGLN1-Inhibitor oder VHL-Inhibitor zur Verwendung bei der Behandlung oder Vorbeugung von Eierstockkrebs bei einem Subjekt, das einen solchen Krebs aufweist oder bei dem das Risiko besteht, daran zu erkranken, wobei die Behandlung umfasst:
(a) Erhalten einer Probe von einem Subjekt, das einen Eierstockkrebs aufweist oder bei dem das Risiko besteht, daran zu erkranken;
(b) Identifizieren des Vorhandenseins oder Fehlens eines EGLN1-abhängigen Eierstockkrebses in der Probe; und
(c) Verabreichen eines EGLN1-Inhibitors oder eines VHL-Inhibitors an das Subjekt, wenn der EGLN1-abhängige Eierstockkrebs in der Probe identifiziert wird, dadurch Behandeln oder Vorbeugen eines Eierstockkrebses bei einem Subjekt, das einen solchen Krebs aufweist oder bei dem das Risiko besteht, daran zu erkranken;
wobei der EGLN1-Inhibitor aus der Gruppe ausgewählt ist, bestehend aus:
FG-4592, FG-2216, Molidustat, IOX2, Vadadustat und Daprodustat, wobei der EGLN1-Inhibitor optional FG-4592 ist; und
der VHL-Inhibitor ein Oligonukleotid-Inhibitor von VHL ist, wobei der Oligonukleotid-Inhibitor von VHL optional aus der Gruppe ausgewählt ist, die aus einem Antisense-Oligonukleotid, einer siRNA und einer sgRNA besteht.

7. EGLN1-Inhibitor oder VHL-Inhibitor zur Verwendung nach Anspruch 6, wobei Schritt (b) Durchführen einer einzigen Multiplex-PCR-Reaktion umfasst, die durch Kapillarelektrophorese analysiert wird.

8. EGLN1-Inhibitor oder VHL-Inhibitor zur Verwendung nach Anspruch 6, wobei Schritt (b) das Identifizieren des Vorhandenseins oder Fehlens eines erhöhten Hypoxieinduzierbaren Faktors 1 Alpha (HIF1A) in der Probe im Vergleich zu einer geeigneten Kontrolle umfasst.

9. EGLN1-Inhibitor oder VHL-Inhibitor zur Verwendung nach Anspruch 6, wobei: wenn der Eierstockkrebs ein klarzelliger Eierstockkrebs ist;
Identifizierungsschritt (b) die Verwendung eines Kits zum Identifizieren des EGLN1-abhängigen Eierstockkrebses in der Probe und Auswählen einer EGLN1-Inhibitor- oder einer VHL-Inhibitor-Therapie für ein Subjekt, und Anweisungen für dessen Verwendung umfasst;
und/oder das Subjekt ein Mensch ist.

## Revendications

1. inhibiteur de l'EGLN1 ou inhibiteur de VHL pour utilisation dans le traitement d'un sujet présentant ou risquant de développer un cancer de l'ovaire dépendant de l'EGLN1, dans lequel le traitement comprend :
(a) l'obtention d'un échantillon d'un sujet présentant ou risquant de développer le cancer de l'ovaire dépendant de l'EGLN1 ;
(b) l'identification de la présence ou de l'absence dans l'échantillon du cancer de l'ovaire dépendant de l'EGLN1 ; et
(c) la sélection d'un inhibiteur de l'EGLN1 ou d'un inhibiteur de VHL comme traitement pour le sujet si le cancer de l'ovaire dépendant de l'EGLN1 est identifié dans l'échantillon, sélectionnant ainsi un traitement pour le sujet présentant ou risquant de développer le cancer de l'ovaire dépendant de l'EGLN1, comprenant éventuellement en outre :
(d) l'administration de l'inhibiteur de l'EGLN1 ou de l'inhibiteur de VHL sélectionné au sujet ;
dans lequel l'inhibiteur de l'EGLN1 est sélectionné dans le groupe consistant en :
FG-4592, FG-2216, Molidustat, IOX2, Vadadustat et Daprodustat, éventuellement dans lequel l'inhibiteur de l'EGLN1 est FG-4592 ; et
un inhibiteur d'oligonucléotide de l'EGLN1, éventuellement dans lequel l'inhibiteur d'oligonucléotide de l'EGLN1 est sélectionné dans le groupe consistant en un oligonucléotide antisens, un siRNA et un sgRNA ; et/ou dans lequel l'inhibiteur de VHL est sélectionné dans le groupe consistant en :
VH298 ; et
un inhibiteur oligonucléotidique de VHL, éventuellement dans lequel l'inhibiteur oligonucléotidique de VHL est sélectionné dans le groupe consistant en un oligonucléotide antisens, un siRNA et un sgRNA.

2. inhibiteur de l'EGLN1 ou inhibiteur de VHL pour utilisation selon la revendication 1, dans lequel le cancer de l'ovaire dépendant de l'EGLN1 est un cancer de l'ovaire à cellules claires.

3. inhibiteur de l'EGLN1 ou inhibiteur de VHL pour utilisation selon la revendication 1, dans lequel l'étape (b) comprend l'identification de la présence ou de l'absence dans l'échantillon d'un facteur 1 alpha inductible par l'hypoxie (HIF1A) élevé, par rapport à une commande appropriée.

4. inhibiteur de l'EGLN1 ou inhibiteur de VHL pour utilisation selon la revendication 1, dans lequel l'étape d'identification (b) comprend l'utilisation d'un kit pour identifier le cancer de l'ovaire dépendant de l'EGLN1 dans un échantillon et sélectionner une thérapie par inhibiteur de l'EGLN1 ou par inhibiteur de VHL pour un sujet, et des instructions pour son utilisation.

5. inhibiteur de l'EGLN1 ou inhibiteur de VHL pour utilisation selon la revendication 1, dans lequel le sujet est humain.

6. inhibiteur de l'EGLN1 ou un inhibiteur de VHL pour utilisation dans le traitement ou la prévention d'un cancer de l'ovaire chez un sujet présentant ou risquant de développer un tel cancer, dans lequel le traitement comprend :
(a) l'obtention d'un échantillon d'un sujet présentant ou risquant de développer un cancer de l'ovaire ;
(b) l'identification de la présence ou de l'absence dans l'échantillon d'un cancer de l'ovaire dépendant de l'EGLN1 ; et
(c) l'administration d'un inhibiteur de l'EGLN1 ou d'un inhibiteur de VHL au sujet si le cancer de l'ovaire dépendant de l'EGLN1 est identifié dans l'échantillon, traitant ou prévenant ainsi un cancer de l'ovaire chez un sujet présentant ou risquant de développer un tel cancer ;
dans lequel l'inhibiteur de l'EGLN1 est sélectionné dans le groupe consistant en :
FG-4592, FG-2216, Molidustat, IOX2, Vadadustat et Daprodustat, éventuellement dans lequel l'inhibiteur de l'EGLN1 est FG-4592 ; et
l'inhibiteur de VHL est un inhibiteur oligonucléotidique de VHL, éventuellement dans lequel l'inhibiteur oligonucléotidique de VHL est sélectionné dans le groupe consistant en un oligonucléotide antisens, un siRNA et un sgRNA.

7. inhibiteur d'EGLN1 ou inhibiteur de VHL pour utilisation selon la revendication 6, dans lequel l'étape (b) comprend la réalisation d'une seule réaction PCR multiplex qui est analysée par électrophorèse capillaire.

8. inhibiteur de l'EGLN1 ou inhibiteur de VHL pour utilisation selon la revendication 6, dans lequel l'étape (b) comprend l'identification de la présence ou de l'absence dans l'échantillon d'un facteur 1 alpha inductible par l'hypoxie (HIF1A) élevé, par rapport à une commande appropriée.

9. inhibiteur de l'EGLN1 ou inhibiteur de VHL pour utilisation selon la revendication 6, dans lequel : lorsque le cancer de l'ovaire est un cancer de l'ovaire à cellules claires ;
l'étape d'identification (b) comprend l'utilisation d'un kit pour identifier le cancer de l'ovaire dépendant de l'EGLN1 dans l'échantillon et sélectionner une thérapie par inhibiteur de l'EGLN1 ou par inhibiteur de VHL pour un sujet, et des instructions pour son utilisation ;
et/ou le sujet est humain.
